# EUROPEAN PATENT SPECIFICATION

(11) **EP 2 870 239 B1**
(45) Date of publication and mention of the grant of the patent: **31.10.2018**
(21) Application number: 13734062.6
(22) Date of filing: 03.07.2013
(51) Int. Cl.: C12N 9/10, C12P 19/04

(54) **GENETICALLY MODIFIED MICROORGANISMS CAPABLE OF PRODUCING BETA-GLUCANS AND METHODS FOR PRODUCING BETA-GLUCANS**
VERFAHREN ZUR MIKROBIOLOGISCHEN HERSTELLUNG VON BETA GLUCAN; DAFÜR GEEIGNETE, GENETISCH MODIFIZIERTE MIKROORGANISMEN
PROCÉDÉ DE PRODUCTION DE BÊTA-GLUCANES PAR FERMENTATION DE MICRO-ORGANISMES GÉNÉTIQUEMENT MODIFIÉS

(30) Priority: 04.07.2012 EP 12174882
(43) Date of publication of application: 13.05.2015
(73) Proprietor: Wintershall Holding GmbH, 34119 Kassel (DE); BASF SE, 67056 Ludwigshafen am Rhein (DE)
(72) Inventor: BROCKMANN, Beata, Morristown, NJ 07960 (US); HEROLD, Andrea, 69469 Weinheim (DE); ZELDER, Oskar, 67346 Speyer (DE); HAEFNER, Stefan, 67346 Speyer (DE); FLECK, Christian, 69207 Sandhausen (DE); SCHRÖDER, Hartwig, 69226 Nußloch (DE); GRANSTRÖM, Mari, FIN-04230 Kerava (FI); SCHMIDT, Julia, Kristiane, 69115 Heidelberg (DE)
(74) Representative: BASF IP Association
(86) International application number: PCT/EP2013/064024
(87) International publication number: WO 2014/006088

(56) References cited:
- JENIEL E. NETT ET AL: "Genetic Basis of Candida Biofilm Resistance Due to Drug-Sequestering Matrix Glucan", THE JOURNAL OF INFECTIOUS DISEASES, vol. 202, no. 1, 1 July 2010 (2010-07-01), pages 171-175, XP055078679, ISSN: 0022-1899, DOI: 10.1086/651200
- J. E. NETT ET AL: "Interface of Candida albicans Biofilm Matrix-Associated Drug Resistance and Cell Wall Integrity Regulation", EUKARYOTIC CELL, vol. 10, no. 12, 1 December 2011 (2011-12-01), pages 1660-1669, XP055078691, ISSN: 1535-9778, DOI: 10.1128/EC.05126-11
- DATABASE UniProt [Online] 5 October 2010 (2010-10-05), "SubName: Full=Glycosyltransferase family 48 protein;", XP002712804, retrieved from EBI accession no. UNIPROT:D8PVE6 Database accession no. D8PVE6
- DATABASE UniProt [Online] 5 October 2010 (2010-10-05), "SubName: Full=Glycosyltransferase family 48 protein;", XP002712805, retrieved from EBI accession no. UNIPROT:D8Q7W6 Database accession no. D8Q7W6
- SCHUREN F H J ET AL: "HIGHLY-EFFICIENT TRANSFORMATION OF THE HOMOBASIDIOMYCETE SCHIZOPHYLLUM COMMUNE TO PHLEOMYCIN RESISTANCE", CURRENT GENETICS, NEW YORK, NY, US, vol. 26, no. 2, 1 January 1994 (1994-01-01), pages 179-183, XP008051258, ISSN: 0172-8083, DOI: 10.1007/BF00313808
- JOCHEN SCHMID ET AL: "Scleroglucan: biosynthesis, production and application of a versatile hydrocolloid", APPLIED MICROBIOLOGY AND BIOTECHNOLOGY, SPRINGER, BERLIN, DE, vol. 91, no. 4, 6 July 2011 (2011-07-06), pages 937-947, XP019931861, ISSN: 1432-0614, DOI: 10.1007/S00253-011-3438-5

## Description

The present invention relates to genetically modified microorganisms capable of producing beta-glucans (herein also referred to as β-glucans), characterized said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain. D-glucan synthase-activity compared to a corresponding non-modified control microorganism of the same strain, wherein said polymer is selected from the group consisting of schizophyllan and scleroglucan, and wherein said microorganism is *Schizophyllum commune.*

The present invention also relates to the use of such microorganisms for producing such β-glucans. Furthermore, the present invention relates to methods for producing such β-glucans comprising the introduction of a polynucleotide encoding a polypeptide having 1,3-β-D-glucan synthase-activity into a microorganism being able to synthesize β-glucans. In context of the present invention, the term "β-glucans" particularly comprise polymers consisting of a linear main chain of β-D-(1-3)-glucopyranosyl units having a single β-D-glucopyranosyl unit (1-6) linked to a β-D-glucopyranosyl unit of the linear main chain with an average branching degree of about 0.3.

β-glucans are known well-conserved components of cell walls in several microorganisms, particularly in fungi and yeast (Novak, Endocrine, Metabol & Immune Disorders - Drug Targets (2009), 9: 67-75). Biochemically, β-glucans comprise non-cellulosic polymers of β-glucose linked via glycosidic β(1-3) bonds exhibiting a certain branching pattern with β(1-6) bound glucose molecules (Novak, *loc cit).* A large number of closely related β-glucans exhibit a similar branching pattern such as schizophyllan, scleroglucan, pendulan, cinerian, laminarin, lentinan and pleuran, all of which exhibit a linear main chain of β-D-(1-3)-glucopyranosyl units with a single β-D-glucopyranosyl unit (1-6) linked to a β-D-glucopyranosyl unit of the linear main chain with an average branching degree of about 0.3 (Novak, *loc cit*; EP-B1 463540; Stahmann, Appl Environ Microbiol (1992), 58: 3347-3354; Kim, Biotechnol Letters (2006), 28: 439-446; Nikitina, Food Technol Biotechnol (2007), 45: 230-237). Although these β-glucans are structurally closely related, their respective microbial producers are not. Examples of microorganisms producing these structurally closely related β-glucans are *Schizophyllum commune* (for schizophyllan; Martin, Biomacromolecules (2000), 1: 49-60; Rau, Methods in Biotechnol (1999), 10: 43-55, DOI: 10.1007/978-1-59259-261-6_4); *Sclerotium rolfsii, Sclerotium glucanicum,* and *Sclerotium delphinii* (for scleroglucan; Survase, Food Technol Biotechnol (2007), 107-118); *Porodisculus pendulus* (for pendulan; EP-B1 463540); *Botrytis cinerea* (for cinerian; Stahmann, *loc cit*) *Laminaria* sp. (for laminarin; Kim, *loc cit*); and *Lentinula edoles* (for lentinan; Nikitina, *loc cit).* At least two of said β-glucans - schizophyllan and scleroglucan - even share an identical structure and differ only slightly in their molecular mass, *i.e.* in their chain length (Survase, *loc cit*)*.*

Such β-glucans are widely used as thickeners and find application in several applications such as food industry and particularly oil industry (enhanced oil recovery, EOR) (Survase, *loc cit*)*.* Also, such β-glucans are used in the pharmaceutical industry in tablet formulations and excipients as well as in immunotherapy as antiviral agents (Survase, *loc cit).*

Industrial production of β-glucans is mostly performed by fermentation processes using their natural microbial producers. Classical ways to improve β-glucan synthesis, e.g., of schizophyllan is based on manipulation of the development of S. *commune* (Rau, Habilitation, Braunschweig 1997). The most common approach is to convert dicaryotic cells via protoplast generation into monocarytic cells (Rau, Habilitation, Braunschweig 1997). Another approach is to cross different monocaryotic cells to form a new dicaryotic cell (Rau, Habilitation, Braunschweig 1997). Further possible approaches comprise, e.g., a classical random based mutagenesis using UV radiation, transposon mutagenesis or using suitable chemicals (e.g., nitrosoguanidin (NTG or N-methyl-N'-nitro-N-nitrosoguanidin), 2-aminofluorene (2-AF), 4-nitro-o-phenylenediamine (NPD), 2-methoxy-6-chloro-9-(3-(2-chloroethyl) aminopropylamino)acridine × 2HCl (ICR-191), 4-nitroquinolone-N-oxide (NQNO), benzo[α]pyrene (B[alpha]p), or sodium azide (SA)) (Czyz, J Appl Genet (2002), 43(3): 377-389). Due to the rearrangement of genetic material within the crossing event it is possible to select strains exhibiting higher β-glucan (schizophyllan) productivity.

Yet, all of these approaches are undirected and do not allow targeted modification of the β-glucan producing microorganisms. In fact, results and efficiency of such approaches are not predictable and identification and selection of improved strains is labored and costly.

This technical problem has been solved by the means and methods described herein below and as defined in the claims.

In particular, as has been surprisingly found in context with the present invention, overexpression of 1,3-β-D-glucan synthase in a β-glucan producing microorganism such as, e.g., S. *commune* or *S. rolfsii* leads to significant higher yields of the respective β-glucan. This finding was indeed unexpected given the fact that the biosynthetic pathway of β-glucan synthesis was only poorly understood and moreover, for most β-glucan producing microorganisms (such as *Schizophyllum commune*), there was no proposed β-glucan biosynthesis pathway available at all. Moreover, in context of those microorganisms whose β-glucan biosynthesis pathway was at least investigated (such as *Pediococcus parvulus*), enzymes such as α-phosphoglucomutase (α-PGM) and particularly UDP-glucose pyrophosphorylase (UGP) were assumed to represent a bottle-neck in β-glucan synthesis (Velasco, Int J Food Microbiol (2007), 115: 325-354). Accordingly, overexpression of these enzymes was assumed to increase the yields of β-glucan synthesis (Velasco, *loc cit).* Yet, as has been found in context with the present invention, overexpression of UGP in *S. commune* did not result in an increased yield of the β-glucan schizophyllan. In sharp contrast, as further described herein below and in the Examples, it has been found in context of the present invention that *S. commune* possesses two copies of 1,3-β-D-glucan synthase (genome sequence known from Ohm, Nature Biotech (2010), 28: 957-963) and, surprisingly, that overexpressing either of the two copies of 1,3-β-D-glucan synthase in *S. commune* leads to significant higher yields in the production of schizophyllan. Given that schizophyllan has a structure which is closely related to other β-glucans such as scleroglucan, pendulan, cinerian, laminarin, lentinan and pleuran (all of which are polymers consisting of a linear main chain of β-D-(1-3)-glucopyranosyl units having a single β-D-glucopyranosyl unit (1-6) linked to a β-D-glucopyranosyl unit of the linear main chain with an average branching degree of about 0.3), it appears to be likely that overexpression of polypeptides having 1,3-β-D-glucan synthase activity in corresponding microorganisms as also described herein may therefore result in higher yields of those β-glucans.

Accordingly, the present invention relates to a genetically modified microorganism capable of producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0.3, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polymer is selected from the group consisting of schizophyllan and scleroglucan, and wherein said microorganism is *Schizophyllum commune.*
Said polynucleotide may be endogenous or exogenous. For example, in context with the present invention, the overexpression of said polynucleotide may result from the introduction of a strong (e.g., constitutive or inducible) promoter upstream of said polynucleotide thereby increasing the expression level of said polynucleotide, or, preferably, from the introduction of at least one copy of a polynucleotide encoding a polypeptide having 1,3-β-D-glucan synthase-activity. In one embodiment, the present invention relates to a genetically modified microorganism capable of producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0.3, characterized in that said genetically modified microorganism contains at least one copy more of a polynucleotide encoding a polypeptide having 1,3-β-D-glucan synthase-activity compared to a corresponding non-modified control microorganism of the same strain, and wherein said polymer is selected from the group consisting of schizophyllan and scleroglucan, and wherein said microorganism is *Schizophyllum commune.* Said genetically modified microorganism is preferably capable of stably maintaining and expressing the additional polynucleotide encoding a polypeptide having 1,3-β-D-glucan synthase-activity. Said genetically modified microorganism may originate from a corresponding non-modified microorganism which preferably *per se, i.e.* naturally, contains a polynucleotide encoding a polypeptide having 1,3-β-D-glucan synthase-activity. Also, said genetically modified microorganism is preferably *per se, i.e.* before modification, able to produce a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0.3 as described herein. Into said genetically modified microorganism, a strong promoter or at least one polynucleotide encoding a polypeptide having 1,3-β-D-glucan synthase-activity may have been introduced. Non-limiting examples of means and methods for the introduction of a promoter sequence into a microorganism may comprise *inter alia* homologous recombination as known in the art (Ohm, World J Microbiol Biotechnol (2010), 26: 1919-1923). Also, in context with the present invention, the microorganism may have been modified such that more polypeptide having 1,3-β-D-glucan synthase-activity is expressed, e.g., by inserting a strong promoter as described herein, by adding introns into a polynucleotide encoding a polypeptide having 1,3-β-D-glucan synthase-activity, by adapting the codon usage, by improving the ribosomal binding site for better translational initiation, by introducing elements in the mRNA that stabilize it, or by inserting a polynucleotide with a higher transcription level having 1,3-β-D-glucan synthase-activity into the microorganism (cf. Ohm, *loc cit).*

In context with the present invention, the promoter may be introduced into said microorganism upstream of a polynucleotide encoding a polypeptide having 1,3-β-D-glucan synthase-activity and in a manner that said promoter increases or enhances the expression of said polynucleotide. Non-limiting examples of means and methods for the introduction of a polynucleotide into a microorganism may comprise transformation, transduction and transfection as commonly known in the art and as also exemplified herein (Sambrook and Russell (2001), Molecular Cloning: A Laboratory Manual, CSH Press, Cold Spring Harbor, NY, USA; Current Protocols in Molecular Biology, Update May 9, 2012, Print ISSN: 1934-3639, Online ISSN: 1934-3647; Methods in Yeast Genetics, A Laboratory Course Manual, Cold Spring Harbor Laboratory Press, 1990; van Peer, Applied Environ Microbiol (2009), 75: 1243-1247; Schmid, "Genetics of Scleroglucan Production by Sclerotium rolfsii", dissertation Technische Universität Berlin, D83 (2008)). Non-limiting examples of means and methods for the introduction of a promoter sequence into a microorganism may comprise *inter alia* homologous recombination as known in the art (Ohm, World J Microbiol Biotechnol (2010), 26: 1919-1923). Strong promoters to be introduced upstream of a polynucleotide encoding a polypeptide having 1,3-β-D-glucan synthase-activity in context with the present invention may comprise, *inter alia,* constitutive promoters such as, e.g., tef1 promoter (translation and elongation factor 1a, *S. commune, A. niger*), gpdA promoter (glyceraldehyde-3-phosphate dehydrogenase, *S. commune, A. niger*; Schuren, Cur Genet (1998), 33: 151-156), trpC promoter (tryptophan biosynthesis, *Aspergilus nidulans*) or inducible promoters such as, e.g., glaA promoter (glucoamylase, *A*. *niger*), alcA (alcohol dehydrogenase, *A. nidulans*) cbhl (cellobiohydrolase I, *Trichoderma reesei;* Knabe, Dissertation "Untersuchung von Signalkomponenten der sexuellen Entwicklung bei dem Basidiomyceten Schizophyllum commune" (2008)) thiA (thiamine biosynthesis, *Aspergillus oryzae*) (Moore, Biotechnology, Vol. III, Genetic Engeneering of Fungal Cells, Enceclopedia of Life Support Systems (2007)). In context with the present invention, preferred promoters comprise tef1 and gdpA.

Generally, in context with the present invention, the polynucleotide encoding a polypeptide having 1,3-β-D-glucan synthase-activity may be introduced into the microorganism in any suitable form, e.g., comprised in a vector, a plasmid, or as naked nucleic acid as further described and exemplified herein. The polynucleotide introduced into the microorganism may then be exogenous, on a vector/plasmid within the microorganism (*i.e.* outside of the microbial chromosome(s)), or it may be incorporated into the microbial chromosome(s) by, e.g., random (ectopic) or homologous recombination or any other suitable method as known in the art. In context with the present invention, the polynucleotide encoding a polypeptide having 1,3-β-D-glucan synthase-activity which has been introduced into the microorganism (*i.e.* the additional copy to the natural endogenous polynucleotide encoding a polypeptide having 1,3-β-D-glucan synthase-activity of a corresponding unmodified strain) does not necessarily have to have the same nucleotide sequence as the natural endogenous polynucleotide encoding a polypeptide having 1,3-β-D-glucan synthase-activity of a corresponding unmodified strain, as long as it has 1,3-β-D-glucan synthase-activity as described herein.

In one embodiment of the present invention, the genetically modified microorganism is able to produce at least 1.5 times, more preferably at least 1.8 times more, more preferably at least 2.0 times more, and most preferably at least 2.2 times more β-glucan polymer compared to the corresponding non-modified control microorganism. In this context, production of, e.g., 1.5 times "more" β-glucan polymer may mean that a genetically modified microorganism produces an amount of β-glucan polymer which is 1.5 times higher compared to the amount of β-glucan polymer produced in the same time under the same conditions by a corresponding non-modified control microorganism. Alternatively, production of, e.g., 1.5 times "more" β-glucan polymer may mean that a genetically modified microorganism produces the same amount of β -glucan polymer as a corresponding non-modified control organism under the same conditions, however, 1.5 times faster. The amount of produced β-glucan polymer may be measured by methods known in the art and as also described herein.

Furthermore, the present invention relates to the use of a genetically modified microorganism as described herein for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0.3.

Furthermore, the present invention relates to a method of producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, said method comprising the steps of:
(a) introducing into a microorganism being able to synthesize said polymer
   (i) a strong (e.g., constitutive or inducible) promoter upstream of a polynucleotide encoding a polypeptide having 1,3-β-D-glucan synthase-activity thereby increasing the expression of said polynucleotide, or, preferably,
   (ii) a polynucleotide encoding a polypeptide having 1,3-β-D-glucan synthase-activity;
(b) culturing said microorganism of (a) in a medium, thereby allowing said microorganism to produce said polymer; and
(c) optionally recovering said polymer from the medium, wherein said polymer is selected from the group consisting of schizophyllan and scleroglucan, and wherein said microorganism is *Schizophyllum commune.*
As regards step (c) of the method described and provided herein, it is noted that in some cases (e.g., when β-glucans such as schizophyllan is used for oil drilling purposes), the culture broth may also be used directly (e.g., pumped into the drill hole), without previous recovery of the pure β-glucan. As such, the recovery step (c) is optional. Strong promoters to be introduced upstream of a polynucleotide encoding a polypeptide having 1,3-β-D-glucan synthase-activity in context with the present invention may comprise, *inter alia,* constitutive promoters such as, e.g., tef1 promoter (translation and elongation factor 1a, *S*. *commune, A. niger*)*,* gpdA promoter (glyceraldehyde-3-phosphate, S. *commune, A. niger*), trpC promoter (tryptophan biosynthesis, *Aspergilus nidulans*) or inducible promoters such as, e.g., glaA promoter (glucoamylase, *A. niger*), alcA (alcohol dehydrogenase, *A. nidulans*) cbhl (cellobiohydrolase I, *Trichoderma reesei*) thiA (thiamine biosynthesis, *Aspergillus oryzae*), tef1 and gdpA being preferred promoters. In context with the present invention, the promoter is preferably introduced into said microorganism upstream of a polynucleotide encoding a polypeptide having 1,3-β-D-glucan synthase-activity and in a manner that said promoter increases or enhances the expression of said polynucleotide. Said promoter or said polynucleotide encoding a polypeptide having 1,3-β-D-glucan synthase-activity may be introduced in said microorganism by any means and methods known in the art, preferably in a manner that after introduction the promoter can increase the expression of said polynucleotide or that said polynucleotide can be stably maintained and expressed by the microorganism, respectively. Non-limiting examples of means and methods for the introduction of a promoter sequence into a microorganism may comprise, *inter alia,* recombinant homology as known in the art (Ohm, *loc cit*)*.* Non-limiting examples of such methods for the introduction of a polynucleotide into a microorganism may comprise transformation, transduction and transfection as commonly known in the art and as also exemplified herein (Sambrook and Russell (2001), Molecular Cloning: A Laboratory Manual, CSH Press, Cold Spring Harbor, NY, USA; Current Protocols in Molecular Biology, Update May 9, 2012, Print ISSN: 1934-3639, Online ISSN: 1934-3647; Methods in Yeast Genetics, A Laboratory Course Manual, Cold Spring Harbor Laboratory Press, 1990; van Peer, Applied Environ Microbiol (2009), 75: 1243-1247; Schmid, "Genetics of Scleroglucan Production by Sclerotium rolfsii", dissertation Technische Universität Berlin, D83 (2008)).

In context with the present invention, the strong promoter introduced into a microorganism upstream of a polynucleotide encoding a polypeptide having 1,3-β-D-glucan synthase-activity preferably increases the expression level of said polynucleotide at least 1.5-fold, more preferably at least 1.8-fold, more preferably at least 2.0-fold, and most preferably at least 2.2-fold. In this context, the expression level of a polynucleotide can be easily assessed by the skilled person by methods known in the art, e.g., by quantitative RT-PCR, Northern Blot (for assessing the amount of expressed mRNA levels), Dot Blot, Microarray or the like.

Generally, the term "overexpression" as used herein comprises both, overexpression of polynucleotides (e.g., on the transcriptional level) and overexpression of polypeptides (e.g., on the translation level). Accordingly, the present invention relates to a genetically modified microorganism capable of producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0.3, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polymer is selected from the group consisting of schizophyllan and scleroglucan, and wherein said microorganism is *Schizophyllum commune.* In context with the present invention, a genetically modified microorganism is to be considered as "overexpressing" a polynucleotide encoding a polypeptide having 1,3-β-D-glucan synthase-activity if it expresses at least 1.5-fold, more preferably at least 1.8-fold, more preferably at least 2.0-fold, and most preferably at least 2.2-fold of said polynucleotide compared to a non-modified control microorganism of the same strain. In this context, the expression level of a polynucleotide can be easily assessed by the skilled person by methods known in the art, e.g., by quantitative RT-PCR (qRT-PCR), Northern Blot (for assessing the amount of expressed mRNA levels), Dot Blot, Microarray or the like (see, e.g., Sambrook, loc cit; Current Protocols in Molecular Biology, Update May 9, 2012, Print ISSN: 1934-3639, Online ISSN: 1934-3647). Preferably, the amount of expressed polynucleotide is measured by qRT-PCR. Furthermore, in context with the present invention, a genetically modified microorganism is to be considered as "overexpressing" a polypeptide having 1,3-β-D-glucan synthase-activity if it expresses at least 1.5-fold, more preferably at least 1.8-fold, more preferably at least 2.0-fold, and most preferably at least 2.2-fold of said polypeptide compared to a non-modified control microorganism of the same strain. In this context, the expression level of a polypeptide can be easily assessed by the skilled person by methods known in the art, e.g., by Western Blot, ELISA, EIA, RIA, or the like (see, e.g., Sambrook, loc cit; Current Protocols in Molecular Biology, Update May 9, 2012, Print ISSN: 1934-3639, Online ISSN: 1934-3647). Preferably, the amount of expressed polypeptide is measured by Western Blot.

Generally, in context with the present invention, the polynucleotide encoding a polypeptide having 1,3-β-D-glucan synthase-activity may be introduced into the microorganism in any suitable form, e.g., comprised in a vector, a plasmid or as naked nucleic acid. The polynucleotide introduced into the microorganism may then be exogenous (e.g., on a vector or a plasmid) within the microorganism (*i.e.* outside of the microbial chromosome(s)), or it may be incorporated into the microbial chromosome(s) by, e.g., random (ectopic) or homologous recombination or any other suitable method as known in the art. In context with the present invention, the polynucleotide encoding a polypeptide having 1,3-β-D-glucan synthase-activity which has been introduced into the microorganism (*i.e.* the additional copy to the natural endogenous polynucleotide encoding a polypeptide having 1,3-β-D-glucan synthase-activity of a corresponding unmodified strain) does not necessarily have to have the same nucleotide sequence as the natural endogenous polynucleotide encoding a polypeptide having 1,3-β-D-glucan synthase-activity of a corresponding unmodified strain, as long as it has 1,3-β-D-glucan synthase-activity as described herein.

Methods for culturing microorganisms such as fermentation processes are known in the art and also described and exemplified herein (Kumari, Bioresource Technol (2008), 99: 1036-1043; Reyes, J Natural Studies (2009), 7(2), January-June). In context with the present invention, such methods allow the respective microorganism to grow and to produce the desired β-glucan as described and exemplified herein. Suitable media may comprise, e.g., coconut water as described in Reyes, *loc cit.* Furthermore, as known in the art, there are several media particularly suitable for particular microorganisms. For example, also in context with the present invention, suitable media for culturing *S. commune* comprise CYM medium (25 g agar (Difco), 20 g glucose (Sigma), 2 g trypticase peptone (Roth), 2 g yeast extract (Difco), 0.5 g MgSO₄ x 7 H₂O (Roth), 0.5 g KH₂PO₄ and 1 g K₂HPO₄ (both from Riedel-de Haën) per liter H₂O) (particularly useful for cultivation on solid support) or a medium comprising 30 g glucose (Sigma), 3 g yeast extract (Difco), 1 g KH₂PO₄ (Riedel-de Haën), 0.5 g MgSO₄ x 7 H₂O (Roth) per liter H₂O (particularly useful for liquid cultures) as also described and exemplified herein. Further suitable media for culturing *S. rolfsii* are known in the art (Survase, Bioresource Technol (2006), 97: 989-993). The β-glucan produced in accordance to the present invention can be recovered by various methods known in the art and described herein (see also "Recommended Practices for Evaluation of Polymers Used in Enhanced Oil Recovery Operations, API Recommended Practice 63 (RP 63), 1st Ed, American Petoleum Institute, Washington D.C., June 1, 1990; Kumari, Bioresource Technol (2008), 99: 1036-1043).

In context with the present invention, the term "average branching degree about 0,3" may mean that in average about 3 of 10 β-D-(1-3)-glucopyranosyl units are (1-6) linked to a single β-D-glucopyranosyl unit. In this context, the term "about" may mean that the average branching degree may be within the range from 0.1 to 0.5, preferably from 0.2 to 0.4, more preferably from 0.25 to 0.35, more preferably from 0.25 to 0.33, more preferably from 0.27 to 0.33, and most preferably from 0.3 to 0.33. It may also be 0.3 or 0.33. Schizophyllan, scleroglucan, pendulan, cinerian, laminarin, lentinan and pleuran all have an average branching degree between 0.25 and 0.33; for example, scleroglucan and schizophyllan have an average branching degree of 0.3 to 0.33 (Survase, *loc cit;* Novak, *loc cit).* The average branching degree of a β-glucan can be determined by methods known in the art, e.g., by periodic oxidation analysis, methylated sugar analysis and NMR (Brigand, Industrial Gums, Academic Press, New York/USA (1993), 461-472).

The polymer to be produced is selected from the group consisting of schizophyllan and scleroglucan.

The microorganism described herein (hereinafter also referred to as "microorganism in context of the present invention") may generally be a microorganism which is *per se* (*i.e.* naturally, in a non-modified state in context with the present invention) capable of synthesizing β-glucan polymers, particularly those polymers consisting of a linear main chain of β-D-(1-3)-glucopyranosyl units having a single β-D-glucopyranosyl unit (1-6) linked to a β-D-glucopyranosyl unit of the linear main chain with an average branching degree of about 0.3. That is, such microorganisms preferably possess *per se* (*i.e.* naturally, in a non-modified state in context with the present invention) a polynucleotide encoding a polypeptide having 1,3-β-D-glucan synthase-activity. Non-limiting examples of microorganisms in context of the present invention are *Schizophyllum commune, Sclerotium rolfsii, Sclerotium glucanicum, Sclerotium delphinii, Porodisculus pendulus, Botrytis cinerea, Laminaria* sp., *Lentinula edoles,* and *Monilinia fructigena.* The microorganism in context with the present invention is S. *commune.*

The polynucleotide encoding a polypeptide having 1,3-β-D-glucan synthase-activity as referred to and to be employed in context with the present invention (hereinafter also referred to as the "polynucleotide in context of the present invention") may be a 1,3-β-D-glucan synthase gene. For example, the polynucleotide in context of the present invention may comprise or may consist of a nucleic acid sequence which is at least 70%, preferably at least 75%, more preferably at least 80%, more preferably at least 85%, more preferably at least 90%, more preferably at least 95%, more preferably at least 96%, more preferably at least 97%, more preferably at least 98%, more preferably at least 99%, more preferably at least 99,5%, and most preferably 100% identical to SEQ ID NO: 1, 3, 5, 7, 9, 11, 13 or 15, provided that the polypeptide encoded by said polynucleotide has 1,3-β-D-glucan synthase-activity as further described and exemplified herein below. SEQ ID NO: 1 represents the nucleotide sequence of the gene of glucan synthase I of *S. commune* strain Lu15531 (obtained from Jena University (Germany) strain collection, Germany, Prof. E. Kothe; Jena University internal strain name: W22). SEQ ID NO: 3 represents the nucleotide sequence of the gene of glucan synthase II of *S. commune* strain Lu15531. SEQ ID NO: 5 represents the cDNA sequence of glucan synthase I of *S. commune* strain Lu15531. SEQ ID NO: 7 represents the cDNA sequence of glucan synthase II of S. *commune* strain Lu15531. SEQ ID NO: 9 represents the nucleotide sequence of the gene of glucan synthase I of *S. commune* strain Lu15634 (strain collection, BASF SE; monocaryotic strain originating from dicaryotic *S. commune* strain from strain collection at the Technical University of Braunschweig (Germany), Prof. Rau; generated by spore isolation). SEQ ID NO: 11 represents the nucleotide sequence of the gene of glucan synthase II of *S. commune* strain Lu15634. SEQ ID NO: 13 represents the cDNA sequence of glucan synthase I of *S. commune* strain Lu15634. SEQ ID NO: 15 represents the cDNA sequence of glucan synthase II of *S*. *commune* strain Lu15634.

The polypeptide as referred to and to be used in context with the present invention and the polypeptide encoded by the polynucleotide in context of the present invention (said polypeptides hereinafter also referred to as the "polypeptide in context of the present invention") has 1,3-β-D-glucan synthase-activity. In one embodiment, it is a 1,3-β-D-glucan synthase. For example, the polypeptide in context of the present invention may comprise or consist of an amino acid sequence which at least 70%, preferably at least 75%, more preferably at least 80%, more preferably at least 85%, more preferably at least 90%, more preferably at least 95%, more preferably at least 96%, more preferably at least 97%, more preferably at least 98%, more preferably at least 99%, more preferably at least 99,5%, and most preferably 100% identical to SEQ ID NO: 6, 8, 14 or 16, provided that the polypeptide has 1,3-β-D-glucan synthase-activity. SEQ ID NO: 6 represents the amino acid sequence of glucan synthase I of *S. commune* strain Lu15531. SEQ ID NO: 8 represents the amino acid sequence of glucan synthase II of *S*. *commune* strain Lu15531. SEQ ID NO: 14 represents the amino acid sequence of glucan synthase I of *S. commune* strain Lu15634. SEQ ID NO: 16 represents the amino acid sequence of glucan synthase II of *S. commune* strain Lu15634.

In context with the present invention, the term "1,3-β-D-glucan synthase-activity" means that the respective polypeptide is capable of catalyzing the elongation of the 1,3-β-D-glucan chain (chain can be linear or branched) using UDP-glucose as substrate (see Inoue, Eur J Biochem (1995), 231: 845-854). For example, in context with the present invention, a polynucleotide may be considered to encode a polypeptide having 1,3-β-D-glucan synthase-activity if an *S. commune* cell which is transformed with said polynucleotide and which expresses said polynucleotide constitutively is able to produce at least 50%, more preferably at least 75%, more preferably at least 100%, more preferably at least 120%, more preferably at least 150%, more preferably at least 200%, and most preferably at least 220% more schizophyllan compared to an *S. commune* cell not being transformed with said polynucleotide, wherein the following conditions may be applied. The respective S. *commune* cultures with transformed and non-transformed cells, respectively, may be cultivated as follows. For the liquid cultures, the following medium may be used (hereinafter referred to as "Standard Medium"): 30 g glucose (Sigma), 3 g yeast extract (Difco), 1 g KH₂PO₄ (Riedel-de Haën), 0.5 g MgSO₄ x 7 H₂O (Roth) per liter H₂O. For both, pre-cultures and for main culture, 250 ml shaking flasks filled with 30 ml Standard Medium may be used. The cultivation may be carried out at 27 °C and 225 rpm. Before each inoculation, the biomass may be homogenized for 1 minute at 13500 rpm using T 25 digital ULTRA-TURRAX® (IKA). The first pre-culture may be inoculated with 50 mg of wet biomass. The cultures may then be incubated for 72 hours. After 72 hours, the second pre-culture may be started. The concentration of the homogenized wet biomass from the first pre-culture used for inoculation may be 250 mg. Cultivation time may be 45 hours. After 45 hours, the main culture may be inoculated with 500 mg of homogenized wet biomass from the second pre-culture and cultivated for another 45 hours. Subsequently, the cultures may be treated as follows. 10 ml of the culture, 20 ml H₂O and 90 µl Acticide BW20 may be mixed. The sample may then be digested for 24 h at 40 °C with β-glucanase (0.3 ml) (Erbslöh).

After the incubation, the sample may be centrifuged (e.g., 30 minutes at 3400 g) and the supernatant may be analyzed for glucose content using HPLC cation exchanger (Aminex HPX-87-H, BIO-RAD) with 0.5 M H₂SO₄ (Roth) as eluent and 0.5 ml/min flow rate at 30 °C. The typical schizophyllan structure as described herein may be confirmed by further analytical approaches as described in the Example herein below (e.g., by NMR and XRD). The same evaluation may be performed *mutatis mutandis* for assessing whether a given polypeptide has 1,3-β-D-glucan synthase-activity in context of the present invention. In this case, a corresponding polynucleotide encoding said polypeptide to be assessed is evaluated *mutatis mutandis* as described above. If the expression of such a polynucleotide encoding said polypeptide to be assessed is considered to encode a polypeptide having 1,3-β-D-glucan synthase-activity as described above, the polypeptide itself is considered to have 1,3-β-D-glucan synthase-activity.

The level of identity between two or more sequences (e.g., nucleic acid sequences or amino acid sequences) can be easily determined by methods known in the art, e.g., by BLAST analysis. Generally, in context with the present invention, if two sequences (e.g., polynucleotide sequences or amino acid sequences) to be compared by, e.g., sequence comparisons differ in identity, then the term "identity" may refer to the shorter sequence and that part of the longer sequence that matches said shorter sequence. Therefore, when the sequences which are compared do not have the same length, the degree of identity may preferably either refer to the percentage of nucleotide residues in the shorter sequence which are identical to nucleotide residues in the longer sequence or to the percentage of nucleotides in the longer sequence which are identical to nucleotide sequence in the shorter sequence. In this context, the skilled person is readily in the position to determine that part of a longer sequence that matches the shorter sequence. Furthermore, as used herein, identity levels of nucleic acid sequences or amino acid sequences may refer to the entire length of the respective sequence and is preferably assessed pair-wise, wherein each gap is to be counted as one mismatch. These definitions for sequence comparisons (e.g., establishment of "identity" values) are to be applied for all sequences described and disclosed herein.

Moreover, the term "identity" as used herein means that there is a functional and/or structural equivalence between the corresponding sequences. Nucleic acid/amino acid sequences having the given identity levels to the herein-described particular nucleic acid/amino acid sequences may represent derivatives/variants of these sequences which, preferably, have the same biological function. They may be either naturally occurring variations, for instance sequences from other varieties, species, etc., or mutations, and said mutations may have formed naturally or may have been produced by deliberate mutagenesis. Furthermore, the variations may be synthetically produced sequences. The variants may be naturally occurring variants or synthetically produced variants or variants produced by recombinant DNA techniques. Deviations from the above-described nucleic acid sequences may have been produced, e.g., by deletion, substitution, addition, insertion and/or recombination. The term "addition" refers to adding at least one nucleic acid residue/amino acid to the end of the given sequence, whereas "insertion" refers to inserting at least one nucleic acid residue/amino acid within a given sequence. The term "deletion" refers to deleting or removal of at least one nucleic acid residue or amino acid residue in a given sequence. The term "substitution" refers to the replacement of at least one nucleic acid residue/amino acid residue in a given sequence. Again, these definitions as used here apply, *mutatis mutandis,* for all sequences provided and described herein.

Generally, as used herein, the terms "polynucleotide" and "nucleic acid" or "nucleic acid molecule" are to be construed synonymously. Generally, nucleic acid molecules may comprise *inter alia* DNA molecules, RNA molecules, oligonucleotide thiophosphates, substituted ribo-oligonucleotides or PNA molecules. Furthermore, the term "nucleic acid molecule" may refer to DNA or RNA or hybrids thereof or any modification thereof that is known in the art (see, e.g., US 5525711, US 471 1955, US 5792608 or EP 302175 for examples of modifications). The polynucleotide sequence may be single- or double- stranded, linear or circular, natural or synthetic, and without any size limitation. For instance, the polynucleotide sequence may be genomic DNA, cDNA, mitochondrial DNA, mRNA, antisense RNA, ribozymal RNA or a DNA encoding such RNAs or chimeroplasts (Gamper, Nucleic Acids Research, 2000, 28, 4332 - 4339). Said polynucleotide sequence may be in the form of a vector, plasmid or of viral DNA or RNA. Also described herein are nucleic acid molecules which are complementary to the nucleic acid molecules described above and nucleic acid molecules which are able to hybridize to nucleic acid molecules described herein. A nucleic acid molecule described herein may also be a fragment of the nucleic acid molecules in context of the present invention. Particularly, such a fragment is a functional fragment. Examples for such functional fragments are nucleic acid molecules which can serve as primers.

The term "hybridization" or "hybridizes" as used herein in context of nucleic acid molecules/DNA sequences may relate to hybridizations under stringent or non-stringent conditions. If not further specified, the conditions are preferably non-stringent. Said hybridization conditions may be established according to conventional protocols described, for example, in Sambrook, Russell "Molecular Cloning, A Laboratory Manual", Cold Spring Harbor Laboratory, N. Y. (2001); Current Protocols in Molecular Biology, Update May 9, 2012, Print ISSN: 1934-3639, Online ISSN: 1934-3647; Ausubel, "Current Protocols in Molecular Biology", Green Publishing Associates and Wiley Interscience, N. Y. (1989), or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as 0.1 x SSC, 0.1 % SDS at 65 °C. Non-stringent hybridization conditions for the detection of homologous or not exactly complementary sequences may be set at 6 x SSC, 1% SDS at 65 °C. As is well known, the length of the probe and the composition of the nucleic acid to be determined constitute further parameters of the hybridization conditions. Variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. In accordance to the invention described herein, low stringent hybridization conditions for the detection of homologous or not exactly complementary sequences may, for example, be set at 6 x SSC, 1% SDS at 65 °C. As is well known, the length of the probe and the composition of the nucleic acid to be determined constitute further parameters of the hybridization conditions.

Hybridizing nucleic acid molecules also comprise fragments of the above described molecules. Such fragments may represent nucleic acid molecules which code for a functional 1,3-β-D-glucan synthase as described herein or a functional fragment thereof which can serve as a primer. Furthermore, nucleic acid molecules which hybridize with any of the aforementioned nucleic acid molecules also include complementary fragments, derivatives and variants of these molecules. Additionally, a hybridization complex refers to a complex between two nucleic acid sequences by virtue of the formation of hydrogen bonds between complementary G and C bases and between complementary A and T bases; these hydrogen bonds may be further stabilized by base stacking interactions. The two complementary nucleic acid sequences hydrogen bond in an antiparallel configuration. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed). The terms complementary or complementarity refer to the natural binding of polynucleotides under permissive salt and temperature conditions by base-pairing. For example, the sequence "A-G-T" binds to the complementary sequence "T-C-A". Complementarity between two single-stranded molecules may be "partial", in which only some of the nucleic acids bind, or it may be complete when total complementarity exists between single-stranded molecules. The degree of complementarity between nucleic acid strands has significant effects on the efficiency and strength of hybridization between nucleic acid strands. This is of particular importance in amplification reactions, which depend upon binding between nucleic acids strands. The term "hybridizing sequences" preferably refers to sequences which display a sequence identity of at least 45%, more preferably at least 50%, more preferably at least 55%, more preferably at least 60%, more preferably at least 65%, more preferably at least 70%, more preferably at least 75%, more preferably at least 80%. more preferably at least 85%, more preferably at least 90%, more preferably at least 95%, more preferably at least 96%, more preferably at least 97%, more preferably at least 98% more preferably at least 99%, more preferably at least 99,5%, and most preferably 100% identity with a nucleic acid sequence as described herein encoding a 1,3-β-D-glucan synthase.

Also described herein are vectors containing a polynucleotide in context of the present invention. The present invention relates also to a vector comprising the polynucleotide in context of the present invention. The term "vector" as used herein particularly refers to plasmids, cosmids, viruses, bacteriophages and other vectors commonly used in genetic engineering. In a preferred embodiment, the vectors are suitable for the transformation, transduction and/or transfection of microorganisms as described herein, e.g., fungal cells, prokaryotic ells (e.g., bacteria), yeast, and the like. Specific examples of microorganisms in context with the present invention are *Schizophyllum commune, Sclerotium rolfsii, Sclerotium glucanicum, Sclerotium delphinii, Porodisculus pendulus, Botrytis cinerea, Laminaria* sp., *Lentinula edoles,* and *Monilinia fructigena.* In a particularly preferred embodiment, said vectors are suitable for stable transformation of the microorganism, for example to express the polypeptide having 1,3-β-D-glucan synthase activity as described herein.
Accordingly, in one aspect of the invention, the vector as provided is an expression vector. Generally, expression vectors have been widely described in the literature. As a rule, they may not only contain a selection marker gene and a replication-origin ensuring replication in the host selected, but also a promoter, and in most cases a termination signal for transcription. Between the promoter and the termination signal there is preferably at least one restriction site or a polylinker which enables the insertion of a nucleic acid sequence/molecule desired to be expressed.

It is to be understood that when the vector provided herein is generated by taking advantage of an expression vector known in the prior art that already comprises a promoter suitable to be employed in context of this invention, for example expression of a polypeptide having 1,3-β-D-glucan synthase activity as described herein. The nucleic acid construct is preferably inserted into that vector in a manner the resulting vector comprises only one promoter suitable to be employed in context of this invention. The skilled person knows how such insertion can be put into practice. For example, the promoter can be excised either from the nucleic acid construct or from the expression vector prior to ligation. A non-limiting example of the vector of the present invention is pBluescript II comprising the polynucleotide in context of the present invention. Further examples of vectors suitable to comprise the polynucleotide in context of the present invention to form the described herein are known in the art and comprise, for example pDrive, pTOPO, pUC19 and pUC21.

Generally, the present invention relates to all the embodiments described herein as well as to all permutations and combinations thereof. The following particular aspects of the present invention must not be construed as limiting the scope of the present invention on such aspects.

In one aspect, the present invention relates to a genetically modified microorganism capable of producing schizophyllan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said microorganism is *Schizophyllum commune.*
In one aspect, the present invention relates to a genetically modified microorganism capable of producing schizophyllan, characterized in that said genetically modified microorganism contains at least one copy more of a polynucleotide encoding a polypeptide having 1,3-β-D-glucan synthase-activity compared to a corresponding non-modified control microorganism of the same strain, wherein said microorganism is *Schizophyllum commune.*

In another aspect, the present invention relates to a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain wherein said polymer is selected from the group consisting of schizophyllan and scleroglucan.

In another aspect, the present invention relates to a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, wherein said polymer is selected from the group consisting of schizophyllan and scleroglucan.

In one aspect, the present invention relates to a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said microorganism is *Schizophyllum commune.*

In another aspect, the present invention relates to a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, wherein said microorganism is *Schizophyllum commune.*

In another aspect, the present invention relates to a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, wherein said polymer is selected from the group consisting of schizophyllan and scleroglucan, and wherein said microorganism is *Schizophyllum commune.*

In another aspect, the present invention relates to a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, wherein said polymer is selected from the group consisting of schizophyllan and scleroglucan, and wherein said microorganism is *Schizophyllum commune.*

In another aspect, the present invention relates to a genetically modified microorganism capable of producing schizophyllan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, wherein said microorganism is *Schizophyllum commune.*

In another aspect, the present invention relates to a genetically modified microorganism capable of producing schizophyllan, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, wherein said microorganism is *Schizophyllum commune.*

In another aspect, the present invention relates to a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, wherein said polymer is selected from the group consisting of schizophyllan and scleroglucan.

In another aspect, the present invention relates to a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, wherein said polymer is selected from the group consisting of schizophyllan and scleroglucan.

In another aspect, the present invention relates to a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, wherein said microorganism is *Schizophyllum commune.*

In another aspect, the present invention relates to a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, wherein said microorganism is *Schizophyllum commune.*

In another aspect, the present invention relates to a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, wherein said polymer is selected from the group consisting of schizophyllan and scleroglucan, and wherein said microorganism is *Schizophyllum commune.*

In another aspect, the present invention relates to a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, wherein said polymer is selected from the group consisting of schizophyllan and scleroglucan, and wherein said microorganism is *Schizophyllum commune.*

In another aspect, the present invention relates to a genetically modified microorganism capable of producing schizophyllan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, wherein said microorganism is *Schizophyllum commune.*

In another aspect, the present invention relates to a genetically modified microorganism capable of producing schizophyllan, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, wherein said microorganism is *Schizophyllum commune.*

In another aspect, the present invention relates to a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, wherein said polymer is selected from the group consisting of schizophyllan and scleroglucan.

In another aspect, the present invention relates to a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, wherein said polymer is selected from the group consisting of schizophyllan and scleroglucan.

In another aspect, the present invention relates to a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, wherein said microorganism is *Schizophyllum commune.*

In another aspect, the present invention relates to a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, wherein said microorganism is *Schizophyllum commune.*

In another aspect, the present invention relates to the use of a genetically modified microorganism capable of producing schizophyllan, wherein said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing schizophyllan, wherein said genetically modified microorganism contains at least one copy more of a polynucleotide encoding a polypeptide having 1,3-β-D-glucan synthase-activity compared to a corresponding non-modified control microorganism of the same strain, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing schizophyllan, wherein said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, for producing schizophyllan, wherein said microorganism is *Schizophyllum commune.*

In another aspect, the present invention relates to the use of a genetically modified microorganism capable of producing schizophyllan, wherein said genetically modified microorganism contains at least one copy more of a polynucleotide encoding a polypeptide having 1,3-β-D-glucan synthase-activity compared to a corresponding non-modified control microorganism of the same strain, for producing schizophyllan, wherein said microorganism is *Schizophyllum commune.*

In another aspect, the present invention relates to the use of a genetically modified microorganism capable of producing schizophyllan, wherein said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, for producing scleroglucan, wherein said microorganism is *Schizophyllum commune.*

In another aspect, the present invention relates to the use of a genetically modified microorganism capable of producing schizophyllan, wherein said genetically modified microorganism contains at least one copy more of a polynucleotide encoding a polypeptide having 1,3-β-D-glucan synthase-activity compared to a corresponding non-modified control microorganism of the same strain, for producing scleroglucan, wherein said microorganism is *Schizophyllum commune.*

In another aspect, the present invention relates to the use of a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan.

In another aspect, the present invention relates to the use of a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan.

In another aspect, the present invention relates to the use of a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, for producing schizophyllan.

In another aspect, the present invention relates to the use of a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, for producing schizophyllan.

In another aspect, the present invention relates to the use of a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, for producing scleroglucan.

In another aspect, the present invention relates to the use of a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, for producing scleroglucan.

In another aspect, the present invention relates to the use of a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said microorganism is *Schizophyllum commune.*

In another aspect, the present invention relates to the use of a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said microorganism is *Schizophyllum commune.*

In another aspect, the present invention relates to the use of a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, for producing schizophyllan, wherein said microorganism is *Schizophyllum commune.*

In another aspect, the present invention relates to the use of a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, for producing schizophyllan, wherein said microorganism is *Schizophyllum commune.*

In another aspect, the present invention relates to the use of a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, for producing scleroglucan, wherein said microorganism is *Schizophyllum commune.*

In another aspect, the present invention relates to the use of a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, for producing scleroglucan, wherein wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing schizophyllan, wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing schizophyllan, wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing scleroglucan, wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing scleroglucan, wherein said microorganism is *Schizophyllum commune.*

In another aspect, the present invention relates to the use of a genetically modified microorganism capable of producing schizophyllan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing schizophyllan, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing schizophyllan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing schizophyllan wherein said microorganism is *Schizophyllum commune.*

In another aspect, the present invention relates to the use of a genetically modified microorganism capable of producing schizophyllan, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing schizophyllan, wherein said microorganism is *Schizophyllum commune.*

In another aspect, the present invention relates to the use of a genetically modified microorganism capable of producing schizophyllan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing scleroglucan, wherein said microorganism is *Schizophyllum commune.*

In another aspect, the present invention relates to the use of a genetically modified microorganism capable of producing schizophyllan, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing scleroglucan, wherein said microorganism is *Schizophyllum commune.*

In another aspect, the present invention relates to the use of a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan.

In another aspect, the present invention relates to the use of a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan.

In another aspect, the present invention relates to the use of a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing schizophyllan.

In another aspect, the present invention relates to the use of a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing schizophyllan.

In another aspect, the present invention relates to the use of a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing scleroglucan.

In another aspect, the present invention relates to the use of a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing scleroglucan.

In another aspect, the present invention relates to the use of a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing schizophyllan, wherein said microorganism is *Schizophyllum commune.*

In another aspect, the present invention relates to a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing schizophyllan, wherein said microorganism is *Schizophyllum commune.*

In another aspect, the present invention relates to the use of a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing scleroglucan, wherein said microorganism is *Schizophyllum commune.*

In another aspect, the present invention relates to the use of a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15, for producing scleroglucan, wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing schizophyllan, wherein wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing schizophyllan, wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing scleroglucan, wherein said microorganism is *Schizophyllum commune.*

In another aspect, the present invention relates to the use of a genetically modified microorganism capable of producing a polymer consisting of a linear main chain of β-D-(1-3)-glucopyranosyl units having a single β-D-glucopyranosyl unit (1-6) linked to a β -D-glucopyranosyl unit of the linear main chain with an average branching degree of about 0,3, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing scleroglucan, wherein said microorganism is *Schizophyllum commune.*

In another aspect, the present invention relates to the use of a genetically modified microorganism capable of producing schizophyllan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing schizophyllan, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing schizophyllan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing schizophyllan, wherein said microorganism is *Schizophyllum commune.*

In another aspect, the present invention relates to the use of a genetically modified microorganism capable of producing schizophyllan, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing schizophyllan, wherein said microorganism is *Schizophyllum commune.*

In another aspect, the present invention relates to the use of a genetically modified microorganism capable of producing schizophyllan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing scleroglucan, wherein said microorganism is *Schizophyllum commune.*

In another aspect, the present invention relates to the use of a genetically modified microorganism capable of producing schizophyllan, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing scleroglucan, wherein said microorganism is *Schizophyllum commune.*

In another aspect, the present invention relates to the use of a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan.

In another aspect, the present invention relates to the use of a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan.

In another aspect, the present invention relates to the use of a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing schizophyllan.

In another aspect, the present invention relates to the use of a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing schizophyllan.

In another aspect, the present invention relates to the use of a genetically modified microorganism of the species *Schizoyphyllum commune,* characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing scleroglucan.

In another aspect, the present invention relates to the use of a genetically modified microorganism of the species *Schizoyphyllum commune,* characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing scleroglucan.

In another aspect, the present invention relates to the use of a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, wherein said polymer is selected from the group consisting of schizophyllan and scleroglucan, and wherein said microorganism is *Schizophyllum commune.*

In another aspect, the present invention relates to the use of a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing schizophyllan, wherein said microorganism is *Schizophyllum commune.*

In another aspect, the present invention relates to the use of a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing schizophyllan, wherein said microorganism is *Schizophyllum commune.*

In another aspect, the present invention relates to the use of a genetically modified microorganism capable of producing scleroglucan, characterized in that said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing scleroglucan, wherein said microorganism is *Schizophyllum commune.*

In another aspect, the present invention relates to the use of a genetically modified microorganism capable of producing scleroglucan, characterized in that said genetically modified microorganism contains at least one copy more of a polynucleotide encoding a polypeptide having 1,3-β-D-glucan synthase-activity compared to a corresponding non-modified control microorganism of the same strain, wherein said polypeptide is at least 70%, 75%, 80%, 85%, 90%, 95%, 96%, 97%, 98%, 99%, 99,5%, or 100% identical to SEQ ID NO: 6, 8, 14 or 16, for producing scleroglucan, wherein said microorganism is *Schizophyllum commune.*

In another aspect, the present invention relates to a method of producing schizophyllan, said method comprising the steps of:
(a) introducing a strong (e.g., constitutive or inducible) promoter upstream of a polynucleotide encoding a polypeptide having 1,3-β-D-glucan synthase-activity thereby increasing the expression of said polynucleotide, or a polynucleotide encoding a polypeptide having 1,3-β-D-glucan synthase-activity into a microorganism being able to synthesize schizophyllan;
(b) culturing said microorganism of (a) in a medium, thereby allowing said microorganism to produce schizophyllan; and
(c) optionally recovering schizophyllan from the medium,
wherein said microorganism is *Schizophyllum commune.*

In another aspect, the present invention relates to a method of producing scleroglucan, said method comprising the steps of:
(a) introducing a strong (e.g., constitutive or inducible) promoter upstream of a polynucleotide encoding a polypeptide having 1,3-β-D-glucan synthase-activity thereby increasing the expression of said polynucleotide, or a polynucleotide encoding a polypeptide having 1,3-β-D-glucan synthase-activity into a microorganism being able to synthesize scleroglucan;
(b) culturing said microorganism of (a) in a medium, thereby allowing said microorganism to produce scleroglucan; and
(c) optionally recovering scleroglucan from the medium,
wherein said microorganism is *Schizophyllum commune.*

In another aspect, the present invention relates to a method of producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, said method comprising the steps of:
(a) introducing a strong (e.g., constitutive or inducible) promoter upstream of a polynucleotide encoding a polypeptide having 1,3-β-D-glucan synthase-activity thereby increasing the expression of said polynucleotide, or a polynucleotide encoding a polypeptide having 1,3-β-D-glucan synthase-activity into a microorganism of the species *Schizophyllum commune* being able to synthesize said polymer;
(b) culturing said microorganism of (a) in a medium, thereby allowing said microorganism to produce said polymer; and
(c) optionally recovering said polymer from the medium,
wherein said polymer is selected from the group consisting of schizophyllan and scleroglucan.

In another aspect, the present invention relates to a method of producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, said method comprising the steps of:
(a) introducing a polynucleotide encoding a polypeptide having 1,3-β-D-glucan synthase-activity into a microorganism being able to synthesize said polymer, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15;
(b) culturing said microorganism of (a) in a medium, thereby allowing said microorganism to produce said polymer; and
(c) optionally recovering said polymer from the medium,
wherein said polymer is selected from the group consisting of schizophyllan and scleroglucan, and wherein said microorganism is *Schizophyllum commune.*

In another aspect, the present invention relates to a method of producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, said method comprising the steps of:
(a) introducing a polynucleotide encoding a polypeptide having 1,3-β-D-glucan synthase-activity into a microorganism being able to synthesize said polymer, wherein said polypeptide is at least 70%, 75%, 80%, 85%, 90%, 95%, 96%, 97%, 98%, 99%, 99,5%, or 100% identical to SEQ ID NO: 6, 8, 14 or 16;
(b) culturing said microorganism of (a) in a medium, thereby allowing said microorganism to produce said polymer; and
(c) optionally recovering said polymer from the medium,
wherein said polymer is selected from the group consisting of schizophyllan and scleroglucan, and wherein said microorganism is *Schizophyllum commune.*

In another aspect, the present invention relates to a method of producing schizophyllan, said method comprising the steps of:
(a) introducing a polynucleotide encoding a polypeptide having 1,3-β-D-glucan synthase-activity into a microorganism being able to synthesize schizophyllan, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15;
(b) culturing said microorganism of (a) in a medium, thereby allowing said microorganism to produce schizophyllan; and
(c) optionally recovering schizophyllan from the medium,
wherein said microorganism is *Schizophyllum commune.*

In another aspect, the present invention relates to a method of producing scleroglucan, said method comprising the steps of:
(a) introducing a polynucleotide encoding a polypeptide having 1,3-β-D-glucan synthase-activity into a microorganism being able to synthesize scleroglucan, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15;
(b) culturing said microorganism of (a) in a medium, thereby allowing said microorganism to produce scleroglucan; and
(c) optionally recovering scleroglucan from the medium,
wherein said microorganism is *Schizophyllum commune.*

In another aspect, the present invention relates to a method of producing schizophyllan, said method comprising the steps of:
(a) introducing a polynucleotide encoding a polypeptide having 1,3-β-D-glucan synthase-activity into a microorganism being able to synthesize schizophyllan, wherein said polypeptide is at least 70%, 75%, 80%, 85%, 90%, 95%, 96%, 97%, 98%, 99%, 99,5%, or 100% identical to SEQ ID NO: 6, 8, 14 or 16;
(b) culturing said microorganism of (a) in a medium, thereby allowing said microorganism to produce schizophyllan; and
(c) optionally recovering schizophyllan from the medium,
wherein said microorganism is *Schizophyllum commune.*

In another aspect, the present invention relates to a method of producing scleroglucan, said method comprising the steps of:
(a) introducing a polynucleotide encoding a polypeptide having 1,3-β-D-glucan synthase-activity into a microorganism being able to synthesize scleroglucan, wherein said polypeptide is at least 70%, 75%, 80%, 85%, 90%, 95%, 96%, 97%, 98%, 99%, 99,5%, or 100% identical to SEQ ID NO: 6, 8, 14 or 16;
(b) culturing said microorganism of (a) in a medium, thereby allowing said microorganism to produce scleroglucan; and
(c) optionally recovering scleroglucan from the medium,
wherein microorganism is *Schizophyllum commune.*

In another aspect, the present invention relates to a method of producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, said method comprising the steps of:
(a) introducing a polynucleotide encoding a polypeptide having 1,3-β-D-glucan synthase-activity into a microorganism of the species *Schizophyllum commune* being able to synthesize said polymer, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15;
(b) culturing said microorganism of (a) in a medium, thereby allowing said microorganism to produce said polymer; and
(c) optionally recovering said polymer from the medium,
wherein said polymer is selected from the group consisting of schizophyllan and scleroglucan.

In another aspect, the present invention relates to a method of producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0,3, said method comprising the steps of:
(a) introducing a polynucleotide encoding a polypeptide having 1,3-β-D-glucan synthase-activity into a microorganism of the species *Schizophyllum commune* being able to synthesize said polymer, wherein said polypeptide is at least 70%, 75%, 80%, 85%, 90%, 95%, 96%, 97%, 98%, 99%, 99,5%, or 100% identical to SEQ ID NO: 6, 8, 14 or 16;
(b) culturing said microorganism of (a) in a medium, thereby allowing said microorganism to produce said polymer; and
(c) optionally recovering said polymer from the medium,
wherein said polymer is selected from the group consisting of schizophyllan and scleroglucan.

In another aspect, the present invention relates to a method of producing schizophyllan, said method comprising the steps of:
(a) introducing a polynucleotide encoding a polypeptide having 1,3-β-D-glucan synthase-activity into a microorganism of the species *Schizophyllum commune* being able to synthesize said polymer, wherein said polynucleotide is at least 70%, 75%, 80%, 85%, 90%, 95%, 96%, 97%, 98%, 99%, 99,5%, or 100% identical to SEQ ID NO: 1, 3, 5, 7, 9, 11, 13 or 15;
(b) culturing said microorganism of (a) in a medium, thereby allowing said microorganism to produce said polymer; and
(c) optionally recovering said polymer from the medium.

In another aspect, the present invention relates to a method of producing schizophyllan, said method comprising the steps of:
(a) introducing a polynucleotide encoding a polypeptide having 1,3-β-D-glucan synthase-activity into a microorganism of the species *Schizophyllum commune* being able to synthesize said polymer, wherein said polypeptide is at least 70%, 75%, 80%, 85%, 90%, 95%, 96%, 97%, 98%, 99%, 99,5%, or 100% identical to SEQ ID NO: 6, 8, 14 or 16;
(b) culturing said microorganism of (a) in a medium, thereby allowing said microorganism to produce said polymer; and
(c) optionally recovering said polymer from the medium.

The Figures show:

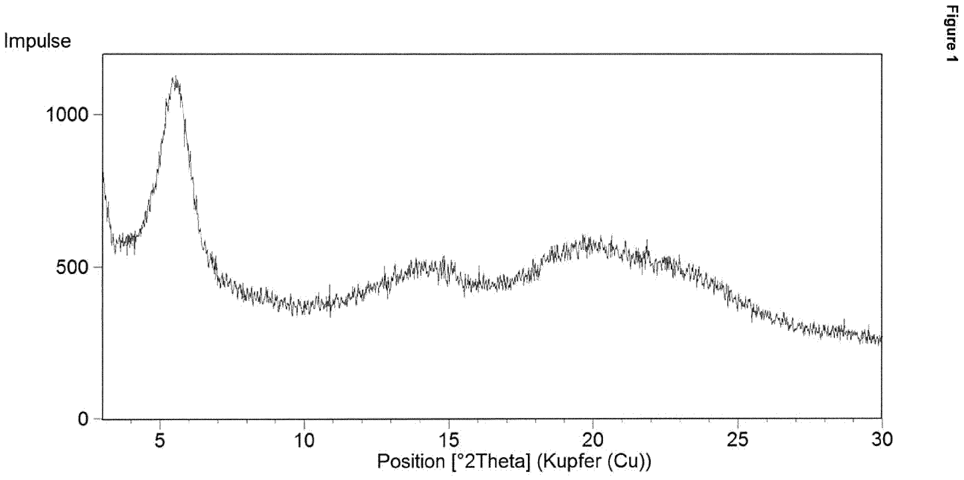

- **Figure 1**: **XRD Spectrum of Schizophyllan sample.** The triple helix could be seen as an intensive diffraction at 5 ° 2θ and the amorphous region of the material gives broad diffraction in the range of 20-25 ° 2θ
- **Figure 2**: **¹H-NMR of schizophyllan (50 mg of gel) in [D₆]-DMSO measured at 50 °C (16 scans, 600 MHz).** The substitution pattern of schizophyllan can be assigned from the integrations of the CH₂OH at 3.7 ppm and CH₂O (ether) at 4.1 ppm signals, the ratio was determined to be 3.3:1 indicating the correct repeating unit.
- **Figure 3**: **13C-NMR of schizophyllan (50 mg of gel) in [D6]-DMSO measured at 50 °C (10.000 scans, 600 MHz).** Assignment of the signals, δ (ppm): 60 and 61 (C-6), 68 (C6-C β(1-6)), 68 (C4-OH side glucose), 70 (C-2 backbone), 72 (C-2), 76 (C-5), 76.7 (C-3 side glucose), 86 (c-§ backbone), 103 (C-1).

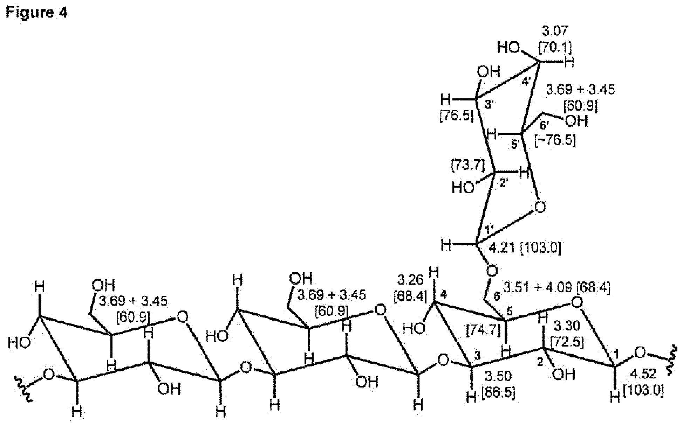

- **Figure 4**: **Schematic picture of the repeating unit of schizophyllan.**

The following Examples illustrate the present invention. Yet, the present invention must not be construed as being limited by the following Examples.

### Examples

### Example 1

### Cloning of the β-1,3-glucan synthase expression plasmid (pGS 1) and transformation into S. commune

In the genome of *Schizophyllum commune,* two genes encoding for β-1,3-glucan synthase were identified by using BLAST analysis (query genes: 1,3-β-glucan synthase sequence from *Mycosphaerella graminicola, Saccharomyces cerevisiae, Cryptococcus neoformans, Schizosaccharomyces pombe*); cf. Ullman, Biochem J (1997), 326: 929-942. In context of the present invention, it was proven that the overexpression of either of these β-1,3-glucan synthases in *S. commune* results in increased yields of schizophyllan production.

Two expression plasmids (pGS_1)] and (pGS_2) (having pBluescript II as backbone) were generated carrying selection marker cassette (*amp^{R}, ura1*), strong constitutive promoter (Tef1 promoter), the synthase gene sequence (genomic seqence) and terminator sequence (Tef1 terminator).

All polynucleotide sequences described herein originate from *Schizopyllum commune.* The polynucleotides represented by SEQ ID NOs 1 and 3 (genes β-1,3-glucan synthases I and II of Lu15531, respectively) were synthesized by Eurofins MWG GmbH/Germany (http://www.eurofinsdna.com/de) according to the original sequence data sourced from JGI data base (http://www.jgi.doe.gov/Scommune; gene position: scaffold 2, 1194740-1200474 and gene position: scaffold 6, 1391067-1396555). The sequences were delivered on pMK plasmids (pMK_GS_1) and (pMK_GS_2) (Eurofins plasmids containing *kan^{R},* ColE1 origin and genomic sequence of respective β-1,3-glucan synthases). The polynucleotides were further used for the cloning of the complete expression plasmid. Plasmid (pMK_GS_1) contained a polynucleotide represented by SEQ ID NO: 1 flanked by 5' *Spe*I and 3' *Sal*I restriction sites. Plasmid (pMK_GS_2) contained a polynucleotide represented by SEQ ID NO: 3 flanked by 5' *Spe*I und 3' *Eco*RV restriction sites, respectively.

The individual elements (SEQ ID NOs. 17, 18 and 33 (Tef1 promoter, Tef1 terminator and *ura1*) were isolated from the genomic DNA of *Schizophyllum commune* using PCR technology prepared by established microbiologic protocols (Sambrook, loc cit; Current Protocols in Molecular Biology, Update May 9, 2012, Print ISSN: 1934-3639, Online ISSN: 1934-3647).

All plasmid isolations were conducted according to manufacturer's instructions using HiSpeed Maxi Kit (Quiagen/Germany). For this purpose, *Escherichia coli XL10* cells (Stratagene) containing the final expression plasmid or one of the interim plasmids were cultivated in Luria-Bertoni (LB) medium (Sigma-Aldrich) containing 50 mg/ml Ampicillin (Sigma-Aldrich).

For isolation of *tef1* promoter sequence (SEQ ID NO: 17), 50 µl PCR reaction contained 1.25 U *PfuUltra* Hotstart Mastermix (Stratagene) and 1.25 U Taq PCR Mastermix (Quiagen), 22 µl H₂O, 22.1 pmol of forward primer TefP_forw (Xbal) (SEQ ID NO: 21) and 100 pmol of reverse primer TefP_rev (Spel) (SEQ ID NO: 22), and 100 ng of template (genomic DNA of *Schizophyllum commune*). The reaction was carried out in Gene Amp® PCR System 9700 Thermal Cycler from PE Applied Biosystems. The following program was used for the amplification: initial heating step up to 95 °C for 4 minutes was followed by 30 cycles of 30 seconds denaturing at 95 °C, 30 seconds of annealing step at 55 °C, 1 minute elongation step at 72 °C, followed by one cycle at 72 °C for 10 minutes.

For amplification of the synthetic β-1,3-glucan synthase gene (SEQ ID NO: 1), 50 µl PCR reaction contained 1.25 U *PfuUltra* Hotstart Mastermix (Stratagene) and 1.25 U Taq PCR Mastermix (Quiagen), 22 µl H₂O, 100 pmol of forward primer GS1_forw (Spel) (SEQ ID NO: 27) and 22 pmol of reverse primer GS1_rev (Sall)(SEQ ID NO: 28), 100 ng template (pMK_GS_1). The reaction was carried out in Gene Amp® PCR System 9700 Thermal Cycler from PE Applied Biosystems. The following program was used for the amplification: an initial heating step up to 95 °C for 4 minutes was followed by 30 cycles of 30 seconds denaturing at 95 °C, 30 seconds of annealing step at 55 °C, 8 minutes elongation step at 72 °C, followed by one cycle at 72 °C for 10 minutes.

In the next PCR reaction step, fusion of the first two PCR products (*tef1* promoter (SEQ ID NO: 17) with β-1,3-glucan synthase gene (SEQ ID NO: 1) was carried out. 50 µl PCR reaction contained 1.25 U of Pwo Hotstart Mastermix (Roche) and 1.25 U Taq PCR Mastermix (Quiagen), 22 µl of H₂O, 22,1 pmol of each primer: Fusion TefP_GS1_forw (Xbal) (SEQ ID NO: 29) and Fusion TefP_GS1_rev (Sail) (SEQ ID NO: 30) and 100 ng of both templates. The reaction was carried out in Gene Amp® PCR System 9700 Thermal Cycler from PE Applied Biosystems. The following program was used for the fusion of both sequences: an initial heating step up to 95 °C for 4 minutes was followed by 30 cycles of 30 seconds denaturing at 95 °C, 30 seconds of annealing step at 55 °C, 8 minutes elongation step at 72 °C, followed by one cycle at 72 °C for 10 minutes.

The product of the fusion PCR was treated with *Sal*I and *Xba*I restriction enzymes (Roche) according to manufacturer's instructions and the vector (pBluescript 2KSP, Stratagene Cloning Systems) was linearized using the same restriction enzymes and subsequently treated with alkaline phosphatase (Roche) according to manufacturer's instructions. Both, the digested PCR product and the linearized pBluescript 2KSP vector, were ligated using T4 DNA Ligase (New England Biolabs, Inc., Beverly, MA/USA) and transformed into *Escherichia coli* XL10 cells (Stratagene) according to manufacturer's instructions.

For isolation of *tef1* terminator sequence (SEQ ID NO: 18) following PCR reaction was carried out: 50 µl PCR reaction contained 1.25 U of Pwo Hotstart Mastermix (Roche) and 1.25 U Taq PCR Mastermix (Quiagen), 22 µl of H₂O, 24 pmol of forward primer TefT_forw (Sail) (SEQ ID NO: 23) and 21 pmol of reverse primer TefT_rev (Sail) (SEQ ID NO: 24), and 100 ng of template (genomic DNA of *Schizophyllum commune*). The reaction was carried out in Gene Amp® PCR System 9700 Thermal Cycler from PE Applied Biosystems. The following program was used: an initial heating step up to 95 °C for 4 minutes was followed by 30 cycles of 30 seconds denaturing at 95 °C, 30 seconds of annealing step at 60 °C, 1 minute elongation step at 72 °C, followed by one cycle at 72 °C for 10 minutes. The PCR product was treated with *Sal*I restriction enzyme (Roche) and ligated with the plasmid containing *tef1* promoter and β-1,3-glucan synthase, which was before linearized with *Sal*I restriction enzyme (Roche) and treated with alkaline phosphatase (Roche) according to manufacturer's instructions. After ligation, the DNA construct was transformed into *Escherichia coli* XL10 cells (Stratagene) according to manufacturer's instructions.

To enable screening of *Schizophyllum commune* strains after transformation with the β-1,3-glucan synthase expression, a plasmid selection marker (*ura1;* SEQ ID NO: 33) was introduced into the plasmid. For that purpose, *ura1* gene was isolated from the genomic DNA of *Schizophyllum commune.* The PCR reaction contained 2.5 U of Pwo Hotstart Mastermix (Roche), 22 µl of H₂O, 21 pmol of forward primer Ura_forw (Notl) (SEQ ID NO: 19),38 pmol of reverse primer Ura_rev (XbaI) (SEQ ID NO: 20) and 100 ng of the template (genomic DNA of *Schizophyllum commune*). The reaction was carried out in Gene Amp® PCR System 9700 Thermal Cycler from PE Applied Biosystems. The following program was used: an initial heating step up to 95 °C for 4 minutes was followed by 30 cycles of 30 seconds denaturing at 95 °C, 30 seconds of annealing step at 60 °C, 2 minutes elongation step at 72 °C, followed by one cycle at 72 °C for 10 minutes. The PCR Product was digested with *Xba*I and *Not*I restriction enzymes (Roche) and ligated into the *Xba*I/*Not*I site of the β-1,3-glucan synthase expression plasmid (pGS_1) using T4 DNA Ligase (New England Biolabs, Inc., Beverly,MA/USA). The resulting plasmid encoding β-1,3-glucan synthase with *tef1* promoter and terminator, and containing *ura1* selection marker was transformed into *Escherichia coli* XL10 cells (Stratagene) according to manufacturer's instructions.

For the transformation of *Schizophyllum commune* with the β-1,3-glucan synthase expression plasmid (pGS_1), plasmid preparation was carried out as follows. *Escherichia coli* XL10 cells containing the β-1,3-glucan synthase expression plasmid were cultivated in Luria-Bertoni (LB) medium (Sigma-Aldrich) containing 50 mg/ml Ampicillin (Sigma-Aldrich) and the plasmid isolation was conducted according to manufacturer's instructions using HiSpeed Maxi Kit (Quiagen).

*Schizophyllum commune* (Lu15527; obtained from strain collection of University of Jena (Germany), Prof. E. Kothe, Jena University internal strain name: 12-43) was transformed based on the method described by van Peer *et al.* (van Peer, *loc cit*) as basis. The method was modified according to the description below.

For preparation of *S. commune* protoplasts, fresh culture was inoculated on a plate containing complex medium (CYM). For incubation at 26 °C for 2-3 days, plates were sealed with parafilm.

For inoculation of liquid preculture (50 ml working volume), the biomass from the plate was macerated for 1 minute at 13500 rpm using T 25 digital ULTRA-TURRAX® (IKA), inoculated in shaking flask containing liquid CYM medium and incubated at 30 °C, 220 rpm for further 3 days. Main culture was inoculated with 15 ml of the preculture in 200 ml CYM medium and incubated further 3 days at 30 °C at 220 rpm. After finishing the culture growth, the main culture was divided in four 50 ml samples and centrifuged (4000 rpm, 15 min). Obtained pellet was washed twice with 1 M MgSO₄ (50ml) (Roth). After washing, four samples were united and dissolved 50 ml 1M MgSO₄.

To enable cell wall lysis, 100 mg Caylase (Cayla, Toulouse, France) were dissolved in 1 mL 1 M MgSO₄ and added to the pellet suspension. The sample was incubated over night at 30 °C under slight shaking (70 rpm). Subsequently distilled water was added to the sample (in 1:1 ratio), which was then incubated under slight shaking (70 rpm) for further 5 min. After this step, cells were incubated without shaking for 10 min and subsequently centrifuged (1100 rpm, 20 min, 4 °C). After the supernatant was filtrated using Miracloth-Membrane, one volume of cold 1 M sorbitol was added and the sample was allowed to equilibrate for 10 min. Subsequently, the sample was centrifuged (2000 rpm, 20 min, 2 °C). Pellet was washed by re-suspending carefully in 1 M sorbitol and centrifugation step was repeated. Finally the protoplasts were resuspended in 1 M sorbitol and 50 mM CaCl₂ at a concentration of 10⁸ protoplasts per ml.

DNA used for transformation was a circular plasmid (pGS_1) and the integration in the genome of *S. commune* was ectopic. To transform the protoplasts with the DNA, 100 µl protoplasts and 10µl DNA (5-10 µg) were gently mixed and incubated for 60 min on ice. Subsequently, one volume of PEG 4000 (40 %) was added and the sample was incubated for 5 to 10 min on ice. After adding 2.5 ml regeneration medium (complete medium containing 0,1 µg/ml Phleomycin and 0.5 M MgSO₄), the sample was incubated at 30 °C, 70 rpm overnight.

After PEG mediated transformation, regenerated protoplasts were spread on petri dishes containing 40 ml solidified minimal medium: 2 g aspartic acid (Roth), 20 g glucose (Sigma), 0.5 g MgSO₄ (Roth), 0.5 g KH₂PO₄, 1 g K₂HPO₄ (both from Riedel-de Haën), 120 µg thiaminhydrochlorid (Roth) per liter, pH 6,3 containing 1 % low melting agarose (Sigma). Selection plates were incubated 5 days at 30 °C.

### Example 2

### Cloning of the β-1,3-glucan synthase expression plasmid [pGS 21 and transformation into S. commune

The expression plasmid for the second β-1,3-glucan synthase (SEQ ID NO: 3) (pGS_2) was prepared analogously to the preparation of (pGS_1) as described above in Example 1.

As a source of the promoter sequence *tef1* (SEQ ID NO: 17); the same PCR product as in Example 1 was used.

Polynucleotide represented by SEQ ID NO: 3 was amplified from the (pMK_GS_2) plasmid following PCR reaction: 50 µl PCR reaction contained 1.25 U *PfuUltra* Hotstart Mastermix (Stratagene) and 1.25 U Taq PCR Mastermix (Quiagen), 22 µl H₂O, 23 pmol of each primer: GS2_forw (Spel) /SEQ ID NO: 31) and GS2_rev (EcoRV)(SEQ ID NO: 32), 100 ng of template (pMK_GS_2). The reaction was carried out in Gene Amp® PCR System 9700 Thermal Cycler from PE Applied Biosystems. The following program was used for the amplification: an initial heating step up to 95 °C for 4 minutes was followed by 30 cycles of 30 seconds denaturing at 95 °C, 30 seconds of annealing step at 53 °C, 8 minutes elongation step at 72 °C, followed by one cycle at 72 °C for 10 minutes.

For isolation of *tef1* terminator sequence (SEQ ID NO: 18) and introduction of the EcoRV (5') and *Apa*I (3') sites, the following PCR reaction was carried out: 50 µl PCR reaction contained 1.25 U of Pwo Hotstart Mastermix (Roche) and 1.25 U Taq PCR Mastermix (Quiagen), 22 µl of H₂O, 37 pmol of forward primer TefT_forw (EcoRV) (SEQ ID NO: 25) and 25 pmol of reverse primer TefT_rev (Apal)(SEQ ID NO: 26), and 100 ng of template (genomic DNA of *Schizophyllum commune*). The reaction was carried out in Gene Amp® PCR System 9700 Thermal Cycler from PE Applied Biosystems. The following program was used: an initial heating step up to 95 °C for 4 minutes was followed by 30 cycles of 30 seconds denaturing at 95 °C, 30 seconds of annealing step at 58 °C, 1 minute elongation step at 72 °C, followed by one cycle at 72 °C for 10 minutes. The PCR product was treated with *Eco*RV and *Apa*I restriction enzyme (Roche) and ligated with the vector (pBluescript 2KSP, Stratagene Cloning Systems), which was before digested the same restriction enzymes. After ligation, the DNA construct was transformed into *Escherichia coli* XL10 cells (Stratagene), according to manufacturer's instructions.

Subsequently, *tef1* promoter was cloned into the plasmid. For this purpose, the PCR product was digested with *Xba*I and *Spe*I (Roche) and ligated with the plasmid described above according to *manufacturer's* instructions, containing *tef1* terminator which was linearized using *Xba*I and *Spe*I*.* The ligation was carried out as described in Example 1 herein. After ligation, the DNA construct was transformed into *Escherichia coli* XL10 cells (Stratagene) according to manufacturer's instructions.

Subsequently, *ura1* was cloned into the plasmid. The same PCR product as in Example 1 was used. After digestion of the PCR product with *Not*I and *Xba*I*,* the fragment was cloned into the plasmid carrying the polynucleotide represented by SEQ ID NO: 7, *tef1* promoter and terminator sequences. Before ligation, the plasmid was linearized by *Not*I and *Xba*I*.* Transformation was carried out as described above in Example 1.

Finally, β-1,3-glucan synthase (SEQ ID NO: 3) was ligated into the plasmid. For this purpose, the PCR product was treated with *Spe*I and EcoRV and ligated into the target expression plasmid as described above. Transformation was carried out as described above in Example 1.

Transformation of *Schizophyllum commune* with (pGS_2) followed as described in Example 1.

### Example 3

### Verification of the functionality of the engineered S. commune strains

Genetically modified *S. commune* strains generated as described above were tested in shaking flasks for increased schizophyllan production. To assure the reproducibility of the results, a three-step cultivationwas applied, consisting of two pre-cultures and one main culture as further described herein below.

For the cultivation of the genetically modified *Schizophyllum commune* strains, two different media were used. For cultivation on solid media, CYM medium (25 g agar (Difco), 20 g glucose (Sigma), 2 g trypticase peptone (Roth), 2 g yeast extract (Difco), 0.5 g MgSO₄ x 7 H₂O (Roth), 0.5 g KH₂PO₄ and 1 g K₂HPO₄ (both from Riedel-de Haën) per liter H₂O) was used. Strains were inoculated on agar plates containing CYM medium covered with cellophane (to avoid mycelium growth into the agar) and incubated for three to four days at 26 °C.

For the liquid cultures, the following medium was used (hereinafter referred to as "Standard Medium"): 30 g glucose (Sigma), 3 g yeast extract (Difco), 1 g KH₂PO₄ (Riedel-de Haën), 0.5 g MgSO₄ x 7H₂O (Roth) per liter H₂O.

For both pre-cultures and for main culture, 250 ml shaking flasks filled with 30 ml Standard Medium were used. The cultivation was carried out at 27 °C and 225 rpm. Before each inoculation, the biomass was homogenized for 1 minute at 13500 rpm using T 25 digital ULTRA-TURRAX® (IKA).

The first pre-culture was inoculated with 50 mg of wet biomass. The cultures were incubated for 72 hours. After 72 hours, the second pre-culture was started. The concentration of the homogenized wet biomass from the first pre-culture used for inoculation was 250 mg. Cultivation time was 45 hours. After 45 hours, the main culture was inoculated with 500 mg of homogenized wet biomass from the second pre-culture and cultivated for another 45 hours.

After the cultivation was finished, standard analytical methods as described herein below were applied to define the biomass concentration, schizophyllan concentration, ethanol concentration and residual glucose in medium. 50 ml aliquots of the cultures were stabilized with 3 g/l Acticide BW20 (Thor).

Ethanol and glucose concentration was estimated using HPLC method. For this purpose 14 ml of the culture were centrifuged (30 min, 8500 rpm). The supernatant was sterile-filtrated and 1 ml of the filtrate was injected for the HPLC analysis (HPLC cation exchanger: Aminex HPX-87-H, BIO-RAD with 0.5 M H₂SO₄, Roth, as eluent and 0.5 ml/min flow rate at 30 °C).

Due to the fact that schizophyllan consists only of glucose molecules, the quantification of this polymer can be done using standard analytical methods for glucose. 10 ml of the culture, 20 ml H₂O and 90 µl Acticide BW20 were mixed. The sample was digested for 24 h at 40 °C with β-glucanase (0.3 ml) (Erbslöh). After the incubation, the sample was centrifuged (30 minutes at 3400 g) and the supernatant was analyzed for glucose and ethanol content using HPLC cation exchanger (Aminex HPX-87-H, BIO-RAD) with 0.5 M H₂SO₄ (Roth) as eluent and 0.5 ml/min flow rate at 30°C.

For the biomass determination, the remaining biomass in form of pellet (after β-glucanase digestion sample was centrifuged) was washed twice with 50 ml H₂O, filtrated using Whatman-Filter (with determination of filter's weight before filtration), washed twice with H₂O and dried in HB43S drying scale from Mettler Toledo. Drying of the filter was carried out for 5 to 10 minutes at 180 °C. Subsequently, weight of the dry filter was determined.

The evaluation of the results obtained in shaking flasks showed clear effect of the overexpression of both β-1,3-glucan synthases on the schizophyllan production. Because of the fact that in the expression plasmid was ectopically integrated into genome and the integration locus has an explicit effect on the expression of the target gene, 40 clones carrying the plasmid (pGS_1) and 40 clones carrying the plasmid (pGS_2) were tested in shaking flask experiments. The increase of schizophyllan production in the genetically modified strains is shown in Table 1 in comparison to the non-modified *Schizophyllum commune* control strain. The results shown in the Table 1 refer to the best strain of each 40 strains tested. For classification of the strains, the amount of schizophyllan in the sample was decisive. 10 ml of the culture, 20 ml H₂O and 90 µl Acticide BW20 were mixed. The sample was digested for 24 h at 40 °C with 0.3 ml β-glucanase (Erbslöh). After the incubation, the sample was centrifuged (30 minutes at 3400 g) and the supernatant was analyzed for glucose and ethanol content using HPLC cation exchanger (Aminex HPX-87-H, BIO-RAD) with 0.5 M H₂SO₄ (Roth) as eluent and 0.5 ml/min flow rate at 30 °C.

In addition to increased yields of schizophyllan production in the genetically modified *S. commune* strains, a clear decrease in the synthesis of the by-product ethanol was observed. This can be an indication that the excess rate of glucose by up-regulated β-1,3-glucan synthase activity is metabolized more directly in the schizophyllan pathway instead of partly being used for ethanol synthesis.

**Table 1: Comparison of Schizophyllum commune control strain with two genetically modified S. commune strains carrying glucan synthase expression plasmid (pGS_1) or (pGS_2).**

| **Strain** | **Schizophyllan [%]** | **EtOH [% [%]** |
|---|---|---|
| *S. commune* control strain | 100 | 100 |
| *S. commune* (pGS_1) | 220 | 9 |
| *S. commune* (pGS_2) | 215 | 3.6 |

### Structure and conformation analysis of the product

To assure that the polymer synthesized through genetically modified *S. commune* strains is schizophyllan, XRD and NMR methods were applied to confirm the structure of the molecule as follows.

Powder X-ray diffraction (XRD) allows rapid, non-destructive analysis of materials consisting of multiple components. Moreover, the sample preparation is straightforward. The data from the measurement is presented as a diffractogram in which the diffracted intensity (I) is shown as a function of scattering angle 2θ. The crystallinity of the given material can be determined by this measurement. In general, crystalline materials have reflection patterns of a series of sharp peaks whereas amorphous materials give a broad signals. Many polymers exhibit semicrystalline behaviour which can also be detected by XRD (Hammond, The basics of chrystallography and diffraction, 3rd Ed., Oxford University Press 2009).

### Sample preparation from aqueous solution

Aqueous solution containing schizophyllan was poured in ethanol to precipitate schizophyllan. The precipitation was filtered and dried either in a vacuum oven. The dried sample was measured by XRD.

### Sample measurement and results by XRD

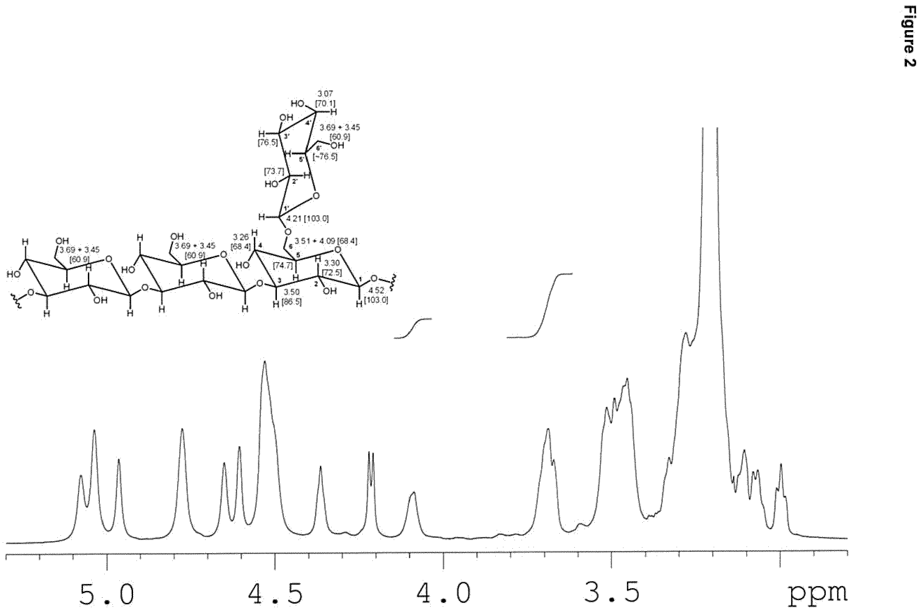

Schizophyllan exhibits a triple helical structure. This was evident from the diffractogram of the precipitated and dried schizophyllan sample (Figure 2). The triple helix could be seen as an intensive diffraction at 5 ° 2θ and the amorphous region of the material gives broad diffraction in the range of 20-25 ° 2θ (Hisamatsu, Carbohydr Res (1997), 298: 117).

### Sample measurement and results by NMR

The NMR spectra were recorded on a Varian VNMRS 600 MHz system equipped with a ¹³C-enhanced cryo probe (inverse configuration) at ambient temperatures or at 50 °C using standard pulse sequences for ¹H and ¹³C.

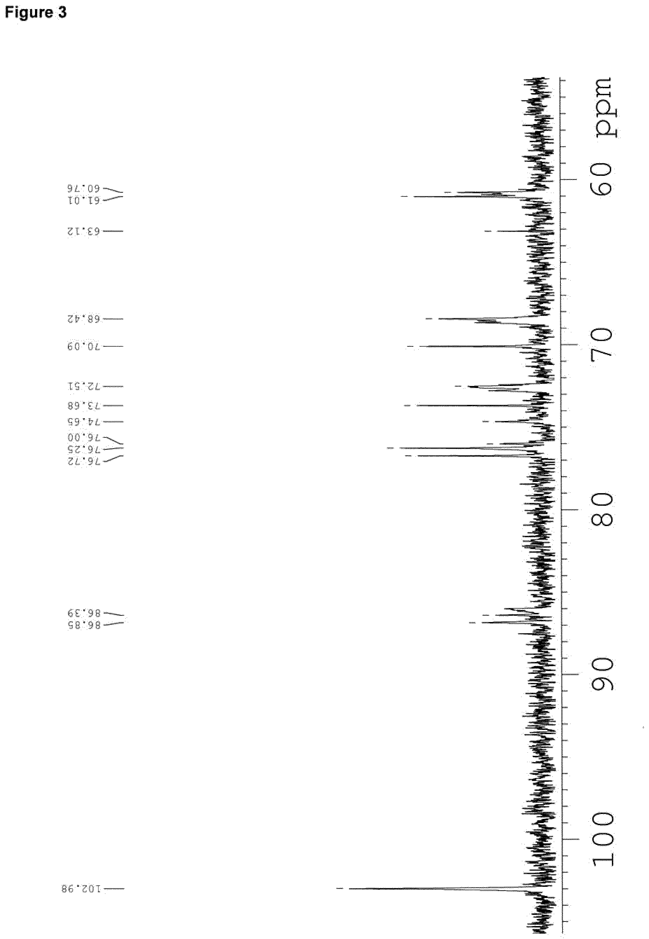

It is known that schizophyllan has a triple helical structure formed by three β(1-3)-D-glucan chains held together by hydrogen bonds in water. This structure is shielded in the magnetic field due to the rigid, ordered conformation. This means that in NMR spectrum, chemical shifts for schizophyllan are not obtained (Rinaudo, Carbohydr Polym (1982), 2: 135; Vlachou, Carbohydr Polym (2001), 46: 349) (2D NMR). In order to investigate the molecular structure of schizophyllan and not the macromolecular structure consisting of triple helices and further to record the successful NMR spectra with a good signal-to-noise ratio, the conformation of the triple helix has to be changed. It is also known that the triple helix of schizophyllan can be altered to form a random coil structure by addition of DMSO. When the DMSO concentration exceeds a certain threshold values (*i.e.* 87%), the conformation change takes place; therefore deuterated [D₆]-DMSO was used as a solvent for the measurements. This conformation matter is important to take into consideration when conducting NMR experiments for schizophyllan. Hence, the sample was measured in [D₆]-DMSO, the well-resolved spectra can be obtained (Figure 2 and 3).

### Summary

The chemical structures of the materials from *S. commune* (GS_1) and *S. commune* (GS_2) strain was identified to be the correct for that of schizophyllan. In addition, the materials exhibit the triple helix conformations. suspended in 1 M sorbitol and 50 mM CaCl₂ at a concentration of 10⁸ protoplasts per ml.

DNA used for transformation was a circular plasmid (pGS_1) and the integration in the genome of *S. commune* was ectopic. To transform the protoplasts with the DNA, 100 µl protoplasts and 10µl DNA (5-10 µg) were gently mixed and incubated for 60 min on ice. Subsequently, one volume of PEG 4000 (40 %) was added and the sample was incubated for 5 to 10 min on ice. After adding 2.5 ml regeneration medium (complete medium containing 0,1 µg/ml Phleomycin and 0.5 M MgSO₄), the sample was incubated at 30 °C, 70 rpm overnight.

After PEG mediated transformation, regenerated protoplasts were spread on petri dishes containing 40 ml solidified minimal medium: 2 g aspartic acid (Roth), 20 g glucose (Sigma), 0.5 g MgSO₄ (Roth), 0.5 g KH₂PO₄, 1 g K₂HPO₄ (both from Riedel-de Haën), 120 µg thiaminhydrochlorid (Roth) per liter, pH 6,3 containing 1 % low melting agarose (Sigma). Selection plates were incubated 5 days at 30 °C.

### Example 2

### Cloning of the β-1,3-glucan synthase expression plasmid [pGS 2] and transformation into S. commune

The expression plasmid for the second β-1,3-glucan synthase (SEQ ID NO: 3) (pGS_2) was prepared analogously to the preparation of (pGS_1) as described above in Example 1.

As a source of the promoter sequence *tef1* (SEQ ID NO: 17); the same PCR product as in Example 1 was used.

Polynucleotide represented by SEQ ID NO: 3 was amplified from the (pMK_GS_2) plasmid following PCR reaction: 50 µl PCR reaction contained 1.25 U *PfuUltra* Hotstart Mastermix (Stratagene) and 1.25 U Taq PCR Mastermix (Quiagen), 22 µl H₂O, 23 pmol of each primer: GS2_forw (Spel) /SEQ ID NO: 31) and GS2_rev (EcoRV)(SEQ ID NO: 32), 100 ng of template (pMK_GS_2). The reaction was carried out in Gene Amp® PCR System 9700 Thermal Cycler from PE Applied Biosystems. The following program was used for the amplification: an initial heating step up to 95 °C for 4 minutes was followed by 30 cycles of 30 seconds denaturing at 95 °C, 30 seconds of annealing step at 53 °C, 8 minutes elongation step at 72 °C, followed by one cycle at 72 °C for 10 minutes.

For isolation of *tef1* terminator sequence (SEQ ID NO: 18) and introduction of the EcoRV (5') and *Apa*I (3') sites, the following PCR reaction was carried out: 50 µl PCR reaction contained 1.25 U of Pwo Hotstart Mastermix (Roche) and 1.25 U Taq PCR Mastermix (Quiagen), 22 µl of H₂O, 37 pmol of forward primer TefT_forw (EcoRV) (SEQ ID NO: 25) and 25 pmol of reverse primer TefT_rev (Apal)(SEQ ID NO: 26), and 100 ng of template (genomic DNA of *Schizophyllum commune*). The reaction was carried out in Gene Amp® PCR System 9700 Thermal Cycler from PE Applied Biosystems. The following program was used: an initial heating step up to 95 °C for 4 minutes was followed by 30 cycles of 30 seconds denaturing at 95 °C, 30 seconds of annealing step at 58 °C, 1 minute elongation step at 72 °C, followed by one cycle at 72 °C for 10 minutes. The PCR product was treated with EcoRV and *Apa*I restriction enzyme (Roche) and ligated with the vector (pBluescript 2KSP, Stratagene Cloning Systems), which was before digested the same restriction enzymes. After ligation, the DNA construct was transformed into *Escherichia coli* XL10 cells (Stratagene), according to manufacturer's instructions.

Subsequently, *tef1* promoter was cloned into the plasmid. For this purpose, the PCR product was digested with *Xba*I and *Spe*I (Roche) and ligated with the plasmid described above according to *manufacturer's* instructions, containing *tef1* terminator which was linearized using *Xba*I and *Spe*I. The ligation was carried out as described in Example 1 herein. After ligation, the DNA construct was transformed into *Escherichia coli* XL10 cells (Stratagene) according to manufacturer's instructions.

Subsequently, *ura1* was cloned into the plasmid. The same PCR product as in Example 1 was used. After digestion of the PCR product with *Not*I and *Xba*I, the fragment was cloned into the plasmid carrying the polynucleotide represented by SEQ ID NO: 7, *tef1* promoter and terminator sequences. Before ligation, the plasmid was linearized by *Not*I and *Xba*I. Transformation was carried out as described above in Example 1.

Finally, β-1,3-glucan synthase (SEQ ID NO: 3) was ligated into the plasmid. For this purpose, the PCR product was treated with *Spe*I and *Eco*RV and ligated into the target expression plasmid as described above. Transformation was carried out as described above in Example 1.

Transformation of *Schizophyllum commune* with (pGS_2) followed as described in Example 1.

### Example 3

### Verification of the functionality of the engineered S. commune strains

Genetically modified *S. commune* strains generated as described above were tested in shaking flasks for increased schizophyllan production. To assure the reproducibility of the results, a three-step cultivationwas applied, consisting of two pre-cultures and one main culture as further described herein below.

For the cultivation of the genetically modified *Schizophyllum commune* strains, two different media were used. For cultivation on solid media, CYM medium (25 g agar (Difco), 20 g glucose (Sigma), 2 g trypticase peptone (Roth), 2 g yeast extract (Difco), 0.5 g MgSO₄ x 7 H₂O (Roth), 0.5 g KH₂PO₄ and 1 g K₂HPO₄ (both from Riedel-de Haën) per liter H₂O) was used. Strains were inoculated on agar plates containing CYM medium covered with cellophane (to avoid mycelium growth into the agar) and incubated for three to four days at 26 °C.

For the liquid cultures, the following medium was used (hereinafter referred to as "Standard Medium"): 30 g glucose (Sigma), 3 g yeast extract (Difco), 1 g KH₂PO₄ (Riedel-de Haën), 0.5 g MgSO₄ x 7 H₂O (Roth) per liter H₂O.

For both pre-cultures and for main culture, 250 ml shaking flasks filled with 30 ml Standard Medium were used. The cultivation was carried out at 27 °C and 225 rpm.

Before each inoculation, the biomass was homogenized for 1 minute at 13500 rpm using T 25 digital ULTRA-TURRAX® (IKA).

The first pre-culture was inoculated with 50 mg of wet biomass. The cultures were incubated for 72 hours. After 72 hours, the second pre-culture was started, The concentration of the homogenized wet biomass from the first pre-culture used for inoculation was 250 mg. Cultivation time was 45 hours. After 45 hours, the main culture was inoculated with 500 mg of homogenized wet biomass from the second pre-culture and cultivated for another 45 hours.

After the cultivation was finished, standard analytical methods as described herein below were applied to define the biomass concentration, schizophyllan concentration, ethanol concentration and residual glucose in medium. 50 ml aliquots of the cultures were stabilized with 3 g/l Acticide BW20 (Thor).

Ethanol and glucose concentration was estimated using HPLC method. For this purpose 14 ml of the culture were centrifuged (30 min, 8500 rpm). The supernatant was sterile-filtrated and 1 ml of the filtrate was injected for the HPLC analysis (HPLC cation exchanger: Aminex HPX-87-H, BIO-RAD with 0.5 M H₂SO₄, Roth, as eluent and 0.5 ml/min flow rate at 30 °C).

Due to the fact that schizophyllan consists only of glucose molecules, the quantification of this polymer can be done using standard analytical methods for glucose. 10 ml of the culture, 20 ml H₂O and 90 µl Acticide BW20 were mixed. The sample was digested for 24 h at 40 °C with β-glucanase (0.3 ml) (Erbslöh). After the incubation, the sample was centrifuged (30 minutes at 3400 g) and the supernatant was analyzed for glucose and ethanol content using HPLC cation exchanger (Aminex HPX-87-H, BIO-RAD) with 0.5 M H₂SO₄ (Roth) as eluent and 0.5 ml/min flow rate at 30°C.

For the biomass determination, the remaining biomass in form of pellet (after β-glucanase digestion sample was centrifuged) was washed twice with 50 ml H₂O, filtrated using Whatman-Filter (with determination of filter's weight before filtration), washed twice with H₂O and dried in HB43S drying scale from Mettler Toledo. Drying of the filter was carried out for 5 to 10 minutes at 180 °C. Subsequently, weight of the dry filter was determined.

The evaluation of the results obtained in shaking flasks showed clear effect of the overexpression of both β-1,3-glucan synthases on the schizophyllan production. Because of the fact that in the expression plasmid was ectopically integrated into genome and the integration locus has an explicit effect on the expression of the target gene, 40 clones carrying the plasmid (pGS_1) and 40 clones carrying the plasmid (pGS_2) were tested in shaking flask experiments. The increase of schizophyllan production in the genetically modified strains is shown in Table 1 in comparison to the non-modified *Schizophyllum commune* control strain. The results shown in the Table 1 refer to the best strain of each 40 strains tested. For classification of the strains, the amount of schizophyllan in the sample was decisive. 10 ml of the culture, 20 ml H₂O and 90 µl Acticide BW20 were mixed. The sample was digested for 24 h at 40 °C with 0.3 ml p-glucanase (Erbslöh). After the incubation, the sample was centrifuged (30 minutes at 3400 g) and the supernatant was analyzed for glucose and ethanol content using HPLC cation exchanger (Aminex HPX-87-H, BIO-RAD) with 0.5 M H₂SO₄(Roth) as eluent and 0.5 ml/min flow rate at 30 °C.

In addition to increased yields of schizophyllan production in the genetically modified *S. commune* strains, a clear decrease in the synthesis of the by-product ethanol was observed. This can be an indication that the excess rate of glucose by up-regulated β-1,3-glucan synthase activity is metabolized more directly in the schizophyllan pathway instead of partly being used for ethanol synthesis.

**Table 1: Comparison of Schizophyllum commune control strain with two genetically modified S. commune strains carrying glucan synthase expression plasmid (pGS_1) or (pGS_2).**

| **Strain** | **Schizophyllan [%]** | **EtOH [% [%]** |
|---|---|---|
| *S. commune* control strain | 100 | 100 |
| *S. commune* (pGS_1) | 220 | 9 |
| *S. commune* (pGS_2) | 215 | 3.6 |

### Structure and conformation analysis of the product

To assure that the polymer synthesized through genetically modified *S. commune* strains is schizophyllan, XRD and NMR methods were applied to confirm the structure of the molecule as follows.

Powder X-ray diffraction (XRD) allows rapid, non-destructive analysis of materials consisting of multiple components. Moreover, the sample preparation is straightforward. The data from the measurement is presented as a diffractogram in which the diffracted intensity (I) is shown as a function of scattering angle 2θ. The crystallinity of the given material can be determined by this measurement. In general, crystalline materials have reflection patterns of a series of sharp peaks whereas amorphous materials give a broad signals. Many polymers exhibit semicrystalline behaviour which can also be detected by XRD (Hammond, The basics of chrystallography and diffraction, 3rd Ed., Oxford University Press 2009).

### Sample preparation from aqueous solution

Aqueous solution containing schizophyllan was poured in ethanol to precipitate schizophyllan. The precipitation was filtered and dried either in a vacuum oven. The dried sample was measured by XRD.

### Sample measurement and results by XRD

Schizophyllan exhibits a triple helical structure. This was evident from the diffractogram of the precipitated and dried schizophyllan sample (Figure 2). The triple helix could be seen as an intensive diffraction at 5 ° 2θ and the amorphous region of the material gives broad diffraction in the range of 20-25 ° 2θ (Hisamatsu, Carbohydr Res (1997), 298: 117).

### Sample measurement and results by NMR

The NMR spectra were recorded on a Varian VNMRS 600 MHz system equipped with a ¹³C-enhanced cryo probe (inverse configuration) at ambient temperatures or at 50 °C using standard pulse sequences for ¹H and ¹³C.

It is known that schizophyllan has a triple helical structure formed by three β(1-3)-D-glucan chains held together by hydrogen bonds in water. This structure is shielded in the magnetic field due to the rigid, ordered conformation. This means that in NMR spectrum, chemical shifts for schizophyllan are not obtained (Rinaudo, Carbohydr Polym (1982), 2: 135; Vlachou, Carbohydr Polym (2001), 46: 349) (2D NMR). In order to investigate the molecular structure of schizophyllan and not the macromolecular structure consisting of triple helices and further to record the successful NMR spectra with a good signal-to-noise ratio, the conformation of the triple helix has to be changed. It is also known that the triple helix of schizophyllan can be altered to form a random coil structure by addition of DMSO. When the DMSO concentration exceeds a certain threshold values (*i.e.* 87%), the conformation change takes place; therefore deuterated [D₆]-DMSO was used as a solvent for the measurements. This conformation matter is important to take into consideration when conducting NMR experiments for schizophyllan. Hence, the sample was measured in [D₆]-DMSO, the well-resolved spectra can be obtained (Figure 2 and 3).

### Summary

The chemical structures of the materials from *S. commune* (GS_1) and *S. commune* (GS_2) strain was identified to be the correct for that of schizophyllan. In addition, the materials exhibit the triple helix conformations.

### Sequences referred to in the present application

**Table 2: Assignment of SEQ ID NOs.**

| **SEQ ID NO:** | **type of sequence** | **description** |
|---|---|---|
| 1 | nucleotide sequence | Gene sequence* 1,3-β-D-glucan synthase I of *S. commune* strain Lu15531 |
| 2 | amino acid sequence | translation of SEQ ID NO:5 |
| 3 | nucleotide sequence | Gene sequence* 1,3-β-D-glucan synthase I of *S. commune* strain Lu15531 |
| 4 | amino acid sequence | translation of SEQ ID NO:7 |
| 5 | nucleotide sequence | cDNA 1,3-β-D-glucan synthase I of *S. commune* strain Lu15531 |
| 6 | amino acid sequence | polypeptide sequence 1,3-β-D-glucan synthase I of *S. commune* strain Lu15531 |
| 7 | nucleotide sequence | cDNA 1,3-β-D-glucan synthase II of *S. commune* strain Lu15531 |
| 8 | amino acid sequence | polypeptide sequence 1,3-β-D-glucan synthase II of *S. commune* strain Lu15531 |
| 9 | nucleotide sequence | Gene sequence* 1,3-β-D-glucan synthase I of *S. commune* strain Lu15534 |
| 10 | amino acid sequence | translation of SEQ ID NO:13 |
| 11 | nucleotide sequence | Gene sequence* 1,3-β-D-glucan synthase II of *S. commune* strain Lu15634 |
| 12 | amino acid sequence | translation of SEQ ID NO:15 |
| 13 | nucleotide sequence | cDNA 1,3-β-D-glucan synthase I of *S. commune* strain Lu15634 |
| 14 | amino acid sequence | polypeptide sequence 1,3-β-D-glucan synthase I of *S. commune* strain Lu15634 |
| 15 | nucleotide sequence | cDNA 1,3-β-D-glucan synthase II of *S. commune* strain Lu15634 |
| 16 | amino acid sequence | polypeptide sequence 1,3-β-D-glucan synthase II of *S. commune* strain Lu15634 |
| 17 | nucleotide sequence | *tef1* promoter from *S. commune* |
| 18 | nucleotide sequence | *tef1* terminator from *S. commune* |
| 19 | nucleotide sequence | Ura_forw (Notl) primer |
| 20 | nucleotide sequence | Ura_rev (Xbal) primer |
| 21 | nucleotide sequence | Tefp_forw (Xbal) primer |
| 22 | nucleotide sequence | TefP_rev (Spel) primer |
| 23 | nucleotide sequence | Teft_forw (Sall) primer |
| 24 | nucleotide sequence | Teft_rev (Sall) primer |
| 25 | nucleotide sequence | Teft_forw (Sall) primer |
| 26 | nucleotide sequence | Teft_rev (Apal) primer |
| 27 | nucleotide sequence | GS1_forw (Spel) primer |
| 28 | nucleotide sequence | GS1_rev (Sall) primer |
| 29 | nucleotide sequence | Fusion TefP_GS1_forw (Xbal) primer |
| 30 | nucleotide sequence | Fusion TefP_GS1_rev (Sall) primer |
| 31 | nucleotide sequence | GS2_forw (Spel) primer |
| 32 | nucleotide sequence | GS2_rev (EcoRV) primer |
| 33 | nucleotide sequence | *ura* gene (S. commune) |
| 34 | amino acid sequence | Ura protein |

| | | |
|---|---|---|
| *Gene sequence includes introns and flanking regions. In the gene sequences below (for SEQ ID NOs. 1,3,9 and 11), predicted exons are shown in capital letters, introns are shown in lower case letters. | | |

**SEQ ID NO : 1**
   Gene sequence 1,3-β-D-glucan synthase I of *S. commune* strain Lu15531
   DNA
   *S. commune* 
CCCGTCCCTCAAGGCCGTTCTTTCGCTGGCGACCGACCCGGTGTTCGCGAGAA
CCTGTTGTTTCTGACGATCATCAGCCCTTTCTTCTCGTCGCTCTTTAGCTCTCCC
TAGACCGTCTTTTACTCTACTCTTCGACGCACGCCATGTCCGGCCCAGGATATG
GCAGGAATCCATTCGACAATCCCCCGCCCAACAGAGGTCCCTATGGCCAGCAG
CCAGGTTTCCCGGGGCCCGGCCCTCGGCCTTACGACTCGGACGCGGACATGA
GCCAGACCTATGGCAGCACAACCAGGCTCGCCGGCAGTGCCGGTTACAGCGA
CAGAAACGgtgcgcacgtcgctaccgtacttcctcgatcgtcgattcacataccatgcagGCAGCTTCGAC
GGCGACCGCTCCTACGCGCCCTCAATTGACTCGCGCGCCAGCGTGCCCAGCAT
ATCGCCCTTCGCAGACCCGGGTATCGGCTCTAATGAGCCGTATCCCGCTTGGT
CGGTCGAACGCCAGATTCCCATGTCCACGGAGGAGATTGAGGACATCTTCCTC
GACCTCACCCAAAAGTTTGGCTTCCAGCGCGACTCCATGCGGAATACGgtgcgtga
ataagcagcccactcgaccgcggaacagcacaattgacctgtcacccagTTCGACTTCATGATGCAC
CTCCTCGATTCCCGTGCCTCGCGCATGACGCCCAACCAAGCTCTGCTCACGCTT
CACGCCGACTACATTGGTGGCCAGCATGCCAATTACCGGAAGTGGTATTTCGCC
GCACAGCTCAACCTCGATGACGCGGTCGGCAAACCAATAACCCCGGTATCCA
GCGCTTGAAGACCATCAAGGGCGCTACGAAGACCAAGTCGCTCGACAGCGCAC
TCAACCGCTGGCGCAACGCGATGAACAACATGAGCCAGTACGATCGCCTCCGG
CAAATTGCGCTCTACCTCCTCTGCTGGGGTGAAGCAGGCAACATCCGTCTGGC
GCCCGAGTGCTTGTGCTTCATCTTCAAGTGCGCGGACGACTACTACAGAAGTCC
CGAGTGTCAGAACCGGATGGACCCCGTGCCGGAAGGGCTGTACCTGCAGACG
GTCATCAAGCCGCTCTATCGCTTCCTACGTGATCAGGCGTACGAAGTCGTTGAT
GGGAAGCAAGTGAAGCGCGAGAAGGACCACGACCAGATTATCGGTTATGACGA
CGTCAACCAGTTATTCTGGTATCCGGAAGGTTTGGCTAAGATCGTCATGTCGGA
CAACgtgcgtatgatcttatcggttaaaattcgtccgctcacatctttccagACACGACTTGTAGATGTAC
CTCCGGCGCAGCGGTTCATGAAGTTCGCCAAGATCGAGTGGAACCGCGTCTTC
TTCAAGACGTACTTTGAGAAGCGCTCTACTGCCCATCTCCTGGTCAACTTCAAC
CGTATATGGATCCTCCACGTCTCGATGTACTTCTTCTACACGGCATTCAACTCTC
CACGAGTCTACGCGCCGCACGGCAAACTCGACCCCTCCCCTGAGATGACCTGG
TCCGCGACTGCCCTTGGAGGCGCTGTGTCCACCATGATCATGATCCTTGCCACT
ATCGCGGAGTACACCTACATCCCCACGACATGGAACAATGCGTCGCACCTCAC
 
CACGCGGCTCATTTTCCTCCTGGTCATCCTCGCGCTCACTGCTGGCCCAACATT
CTATATCGCCATGATAGACGGACGCACGGACATCGGCCAAGTACCACTCATCGT
GGCCATAGTGCAGTTCTTCATCTCCGTCGTCGCCACCCTCGCTTTCGCTACCAT
CCCTTCTGGTCGCATGTTCGGCGACCGTGTGGCTGGCAAGTCAAGAAAGCACA
TGGCATCGCAGACGTTCACAGCGTCGTACCCGTCCATGAAGCGGTCATCTCGC
GTAGCGAGTATCATGCTGTGGCTTTTGGTCTTTGGCTGCAAATACGTCGAGTCT
TACTTCTTCTTGACGTCCTCCTTCTCCAGCCCGATCGCGGTCATGGCGCGTACG
AAGGTACAGGGCTGCAACGACCGTATCTTCGGCAGCCAGCTGTGCACGAATCA
GGTCCCGTTCGCGCTGGCAATCATGTACGTGATGGACCTGGTACTGTTCTTCCT
GGACACGTACCTGTGGTACATCATCTGGCTGGTGATCTTCTCGATGGTGCGCG
CGTTCAAGCTTGGTATCTCGATCTGGACGCCCTGGAGCGAGATCTTCACCCGCA
TGCCGAAGCGTATTTACGCAAAGCTGCTGGCGACGGCCGAGATGGAGGTCAAG
TATAAGCCCAAGgtatgctgaattcaatctggtcaggtgaattcaccctcatattgtggtacagGTGCTCGT
CTCACAAATCTGGAACGCGGTCATCATCTCCATGTACCGGAGCATCTCTTGTC
CATCGAGCACGTCCAGCGCTTGCTTTACCACCAGGTTGATGGTCCCGATGGCC
GCCGCACCCTCAGGGCACCGCCGTTCTTCACCAGCCAGCGAACTGCGAAGCCA
GGCCTGTTCTTCCCTCCTGGTGGCGAGGCTGAGCGCCGCATCTCGTTCTTTGC
CTCATCGCTGACGACCGCGCTCCCGGAGCCTCTGCCGATCGACGCCATGCCCA
CCTTCACCGTGCTCGTTCCCCATTACTCCGAGAAGATTCTGCTCAGTCTGCGCG
AGATTATCCGCGAGGAGGACCAGAACACCCGCGTTACCTTACTGGAGTACCTCA
AGCAGCTCCACCCTGTCGAATGGGACAATTTCGTCAAGGACACCAAGATCTTGG
CGGAAGAGTCGGGAGACGTCCAGGACGAGAAGCGCGCGCACGGACGACTT
GCCGTTCTATTGCATCGGGTTCAAGACCTCGTCACCAGAGTACACCCTGCGTAC
GCGTATCTGGGCCTCACTGCGCGCACAGACGCTGTACCGCACGGTCTCCGGTA
TGATGAACTACTCCAAGGCGATTAAGCTCCTCTATCGCGTCGAGAACCCGGATG
TCGTTCATGCCTTCGGTGGGAACACGGAACGTCTTGAACGCGAGCTTGAGCGC
ATGTCTCGCCGCAAGTTCAAGTTCGTCATCTCGATGCAGCGGTACTCCAAGTTC
AACAAGGAGGAGCAGGAGAACGCCGAGTTCCTTCTGCGCGCGTACCCGGATTT
GCAGATCGCGTACCTCGATGAAGAGCCCGGTCCCAGCAAGAGCGACGAGGTTC
GGTTGTTTTCGACACTCATCGACGGACACTCCGAGGTGGACGAGAAGACGGGC
CGCCGCAAGCCCAAGTTCCGCATCGAGCTGCCCGGTAACCCCATCCTCGGTGA
CGGGAAGTCGGATAACCAGAACCACGCCATCGTCTTCTACCGCGGCGAGTACA
TTCAGGTCATTGACGCTAACCAGGACAATTACCTGGAAGAGTGTCTCAAGATCC
GTAATGTCCTGGGCGAGTTTGAGGAATACTCCGTGTCGAGCCAGAGCCCGTAC
GCGCAGTGGGGCCACAAGGAGTTCAACAAGTGCCCCGTCGCTATCCTGGGTTC
CCGCGAGTACATCTTCTCGGAGAACATCGGTATCCTCGGTGACATCGCTGCCG
GCAAGGAACAGACGTTCGGTACCATTACGGCGCGTGCGCTTGCGTGGATCGGC
GGCAAGCTGCATTACGGTCACCCGGATTTCCTCAATGCGACGTTCATGACGACG
CGTGGTGGCGTGTCAAAAGCGCAGAAGGGCTTGCATCTTAACGAGGATATCTTC
GCTGGTATGACCGCCGTGTCCCGCGGAGGGCGCATCAAGCACATGGAGTACTA
CCAGTGCGGCAAAGGTCGTGATCTCGGATTCGGCACGATCTTGAACTTCCAGA
CCAAGATCGGTACTGGTATGGGCGAGCAGCTGCTCTCGCGCGAGTACTACTAT
CTGGGCACGCAATTGCCTATCGACCGGTTCTTGACGTTCTACTACGCGCACGCT
GGTTTCCATGTCAACAACATCCTGGTCATCTACTCCATCCAGGTCTTCATGGTCA
CCCgtaagtgcaggccctcatgaccgccgagcaagcagtctaacggatgtgcagTGCTGTACCTGGGC
ACATTGAACAAGCAGCTGTTCATCTGCAAGGTCAACTCCAATGGCCAGGTTCTT
AGTGGACAAGCTGGGTGCTACAACCTCATCCCGGTCTTCGAGTGGATTCGCCG
GAGTATCATCTCCATCTTCTTGGTGTTCTTCATCGCCTTCTTGCCGTTGTTCTTG
CAAGgtatgttcacttctcatgtgccatttgtcaatcgctcactcgtacgacagAGCTTTGCGAACGCGGA
ACAGGAAAGGCGTTGCTGCGTCTCGGGAAGCACTTCCTGTCACTGTCGCCCAT
 
CTTCGAAGTGTTCTCCACCCAAATCTACTCGCAGGCGCTCTTGAACAACATGAG
TTTCGGTGGTGCGCGCTACATCGCTACAGGACGCGGTTTCGCGACGAGTCGGA
TACCCTTCAACATCCTCTACTCGCGTTTCGCGCCGCCGAGCATCTACATGGGCA
TGCGTAATCTGCTGCTCTTGCTGTACGCGACGATGGCCATTTGGATCCCACACC
TGATCTACTTCTGGTTCTCCGTCCTCTCCCTCTGCATCGCGCCATTCATGTTCAA
TCCGCATCAATTCTCGTACGCTGACTTCATCATCGACTACCGGGAGTTCTTGCG
CTGGATGTCGCGCGGTAACTCGCGGACGAAGGCGAGTAGCTGGTACGGATATT
GCCGTCTGTCGCGTACCGCGATTACTGGGTACAAGAAGAAACTGGGACAC
CCGTCGGAGAAGCTGTCGGGCGATGTGCCGCGTGCGCCGTGGAGGAACGTCA
TCTTCTCGGAGATCCTTTGGCCCATCGGCGCGTGCATCATCTTCATCGTCGCGT
ACATGTTCGTCAAATCGTTCCCTGACGAGCAGGGCAACGCGCCGCCGAGCCCG
CTGGTCCGCATTCTGCTCATCGCGGTTGGCCCTACTGTGTGGAACGCGGCGGT
GCTCATCACGCTGTTCTTCCTGTCGCTCTTCCTGGGCCCGATGATGGATGGCTG
GGTCAAGTTCGGCTCAGTCATGGCGGCACTTGCGCATGGTCTAGCGCTCATAG
GCATGCTCACGTTCTTCGAGTTCTTCgtacgtccttcgcgttgttggtcgagtgctttgctaacaccg
ccttcagTGGTTCCTCGAGCTCTGGGATGCCTCGCACGCCGTGCTCGGCGTCATC
GCCATTATTGCCGTTCAGCGCGGATCCAGAAGATCCTCATTGCCGTCTTCCTG
ACGCGTGAGTACAAGCACGACGAGACGAACCGCGCGTGGTGGACAGGTAAATG
GTATGGACGCGGGCTGGGTACCTCGGCCATGTCCCAGCCGGCGCGCGAGTTC
ATCGTGAAGATCGTGGAGATGTCGCTGTGGACGTCGGACTTCCTGCTTGCGCA
CCTGTTGCTCATCATCTTGACGGTGCCGCTACTGCTGCCGTTCTTCAACTCGAT
CCATTCGACGATGCTTTgtgagtgatttgtagtcgttggtcacggatgattgctgactcgcgtgcagTCTG
GTTGCGCCCTTCGAAGCAGATTAGGCAACCTCTGTTCTCCACTAAGCAGAAGCG
GCAACGGCGATGGATTgtaagttcctttgattgctctggctaccgaccttcgctcacctgtctcagGTCATG
AAGTATACCGTGGTATATCTCGTGGTGGTGGCTTTCCTCGTTGCGCTCATCGCT
CTGCgtacgttttctgtcgcgctcaccctctattttcactaacgtttcctccagCCGCGCTCTTCCGCGAGA
GCATCCACTTCAACTGCGAGATCTGCCAGAGTATATAGTCATATAACGACGTCTA
TCGTATCGCCGGACGAGAGCCCCGTCGCCTACACACTGACATGGAATTGCTGT
GTATACAATCGATCTTCTGACCGCGTCGGGGGCGTTGCCGTCTTTCTACTATCA
ACTTGCTTGTGTATCAACATTTCTTCTCTCCAAGCCTACATTGACATAGAGTAATA
GCCCATGTTCATACAACAATCGCATAGCATTGCATATACCAT
**SEQ ID NO: 2**
   Translation of SEQ ID NO: 5
   amino acid
   *S. commune* 
MSGPGYGRNPFDNPPPNRGPYGQQPGFPGPGPRPYDSDADMSQTYGSTTRLAG
SAGYSDRNGSFDGDRSYAPSIDSRASVPSISPFADPGIGSNEPYPAWSVERQIPMS
TEEIEDIFLDLTQKFGFQRDSMRNTFDFMMHLLDSRASRMTPNQALLTLHADYIGGQ
HANYRKWYFAAQLNLDDAVGQTNNPGIQRLKTIKGATKTKSLDSALNRWRNAMNN
MSQYDRLRQIALYLLCWGEAGNIRLAPECLCFIFKCADDYYRSPECQNRMDPVPEG
LYLQTVIKPLYRFLRDQAYEVVDGKQVKREKDHDQIIGYDDVNQLFWYPEGLAKIVM
SDNTRLVDVPPAQRFMKFAKIEWNRVFFKTYFEKRSTAHLLVNFNRIWILHVSMYFF
YTAFNSPRVYAPHGKLDPSPEMTWSATALGGAVSTMIMILATIAEYTYIPTTWNNAS
HLTTRLIFLLVILALTAGPTFYIAMIDGRTDIGQVPLIVAIVQFFISVVATLAFATIPSGRM
FGDRVAGKSRKHMASQTFTASYPSMKRSSRVASIMLWLLVFGCKYVESYFFLTSSF
SSPIAVMARTKVQGCNDRIFGSQLCTNQVPFALAIMYVMDLVLFFLDTYLWYIIWLVI
FSMVRAFKLGISIWTPWSEIFTRMPKRIYAKLLATAEMEVKYKPKVLVSQIWNAVIISM
YREHLLSIEHVQRLLYHQVDGPDGRRTLRAPPFFTSQRTAKPGLFFPPGGEAERRIS
 
FFASSLTTALPEPLPIDAMPTFTVLVPHYSEKILLSLREIIREEDQNTRVTLLEYLKQLH
PVEWDNFVKDTKILAEESGDVQDEKRARTDDLPFYCIGFKTSSPEYTLRTRIWASLR
AQTLYRTVSGMMNYSKAIKLLYRVENPDVVHAFGGNTERLERELERMSRRKFKFVI
SMQRYSKFNKEEQENAEFLLRAYPDLQIAYLDEEPGPSKSDEVRLFSTLIDGHSEVD
EKTGRRKPKFRIELPGNPILGDGKSDNQNHAIVFYRGEYIQVIDANQDNYLEECLKIR
NVLGEFEEYSVSSQSPYAQWGHKEFNKCPVAILGSREYIFSENIGILGDIAAGKEQTF
GTITARALAWIGGKLHYGHPDFLNATFMTTRGGVSKAQKGLHLNEDIFAGMTAVSR
GGRIKHMEYYQCGKGRDLGFGTILNFQTKIGTGMGEQLLSREYYYLGTQLPIDRFLT
FYYAHAGFHVNNILVIYSIQVFMVTLLYLGTLNKQLFICKVNSNGQVLSGQAGCYNLI
PVFEWIRRSIISIFLVFFIAFLPLFLQELCERGTGKALLRLGKHFLSLSPIFEVFSTQIYS
QALLNNMSFGGARYIATGRGFATSRIPFNILYSRFAPPSIYMGMRNLLLLLYATMAIW
IPHLIYFWFSVLSLCIAPFMFNPHQFSYADFIIDYREFLRWMSRGNSRTKASSWYGY
CRLSRTAITGYKKKKLGHPSEKLSGDVPRAPWRNVIFSEILWPIGACIIFIVAYMFVKS
FPDEQGNAPPSPLVRILLIAVGPTVWNAAVLITLFFLSLFLGPMMDGWVKFGSVMAA
LAHGLALIGMLTFFEFFWFLELWDASHAVLGVIAIIAVQRGIQKILIAVFLTREYKHDET
NRAWWTGKWYGRGLGTSAMSQPAREFIVKIVEMSLWTSDFLLAHLLLIILTVPLLLP
FFNSIHSTMLFWLRPSKQIRQPLFSTKQKRQRRWIVMKYTVVYLVVVAFLVALIALPA
LFRESIHFNCEICQSI
**SEQ ID NO: 3**
   Gene sequence 1,3-β-D-glucan synthase II of *S. commune* strain Lu15531
   DNA
   *S. commune* 
CTGTCCAAAGAAGAGATCGAGGACATCTTCCTCGATCTGACGCAGAAGTTTGGC
TTTCAGCGGGATTCCATGCGGAACATGgtacgtggcgtatgcccatgtgcggcgttctgaggcctaa
acgttttccgccagTTCGACTTCACCATGCAGCTGCTTGACAGCCGAGCGTCTCGTATG
ACCCCCAACCAGGCGCTCCTCACCCTCCACGCCGACTACATTGGTGGCCAGCA
TGCGAACTACCGGAAGTGGTACTTCGCGGCGCAGCTCGACCTTGACGACGCCG
TGGGACAAACTCAGAATCCGGGTCTCAACCGCCTCAAGTCCACTCGCGGATCG
GGCAAGCGACCACGCCATGAAAGTCGCTGAACACGGCATTGGAGCGCTGGC
GGCAAGCCATGAACAACATGTCGCAGTATGACCGCTTACGCCAGATCGCGCTC
TACCTGCTCTGCTGGGGCGAAGCGGCGCAAGTGCGATTCATGCCCGAGTGCTT
GTGCTTCATCTTCAAGTGCGCCGACGACTATTATCGTTCGCCGGAGTGCCAGAA
CAGGATGGAGCCGGTACCGGAGGGTCTCTACCTGAGGACGGTCGTAAAGCCG
CTCTACAGATTTGTCCGGGATCAAGGCTATGAGGTGGTGGAGGGAAAATTCGTA
CGGCGGGAACGGGATCACGACCAAATCATTGGTTACGATGACGTGAATCAGCT
GTTCTGGTACCCGGAGGGCATTGCCCGTATCGTCCTGTCGGACAAGgtaagcacctc
tgtgcatcttctgtgacatacagggctaattgtcgagcagAGTCGTCTGGTCGACCTCCCTCCAGCA
CAGCGCTTCATGAAGTTCGACCGTATCGAGTGGAATCGCGTCTTCTTCAAGACG
TTCTACGAGACTCGATCCTTTACGCATCTTTTGGTCGACTTCAACCGTATCTGGG
TCGTGCACATCGCTCTCTACTTCTTCTACACCGCATACAACTCCCCCACGATCTA
CGCCATCAACGGCAACACTCCGACGTCTCTGGCTTGGAGCGCGACTGCGCTCG
GCGGTGCGGTAGCGACAGGTATCATGATCCTCGCCACGATCGCCGAGTTCTCG
CACATCCCCACGACATGGAACAACACCTCGCATCTGACTCGCCGCCTCGCCTTC
CTCCTCGTCACGCTCGGCCTCACATGTGGTCCGACGTTCTACGTCGCGATTGCA
GAGAGCAACGGGAGCGGCGGCTCTTTGGCCTTGATTCTCGGCATCGTCCAGTT
CTTCATCTCCGTCGTAGCGACTGCGCTCTTCACTATCATGCCTTCTGGTCGTAT
GTTCGGCGACCGCGTCGCAGGCAAGAGTCGCAAGTATCTCGCCAGCCAGACGT
TCACGGCCAGCTACCCGTCGTTGCCCAAGCACCAGCGGTTCGCATCACTCCTG
 
ATGTGGTTCCTCATCTTCGGGTGCAAGTTGACGGAGAGTTACTTCTTCCTGACG
TTGTCCTTCCGCGACCCTATTCGCGTCATGGTCGGCATGAAGATCCAGAACTGC
GAGGACAAGATTTTCGGCAGCGGCCTTTGCAGGAATCACGCAGCATTCACCCT
CACGATCATGTACATCATGGACCTCGTCTTGTTCTTCCTCGACACCTTCCTTTGG
TATGTCATCTGGAACTCGGTTTTCAGTATCGCACGCTCTTTCGTACTCGGCCTTT
CGATCTGGACACCATGGAGGGACATCTTCCAGCGTCTGCCGAAGCGTATCTAC
GCGAAGCTTCTAGCGACCGGCGACATGGAGGTCAAGTACAAGCCCAAGgtgtgtga
atagctcgctgtaaggttcttgattctgactcattcgcagGTCTTGGTTTCGCAAATCTGGAACGCCA
TCATCATCTCCATGTACCGCGAGCACTTGCTCTCTATCGAGCACGTTCAAAAGC
TCCTGTACCATCAAGTGGACACTGGCGAAGCCGGCAAGCGGAGTCTTCGCGCG
CCTCCGTTCTTCGTCGCGCAGGGCAGCAGCGGTGGCTCGGGCGAGTTCTTCCC
GCCTGGTAGCGAGGCTGAGCGTCGTATCTCTTTCTTCGCGCAGTCTCTATCTAC
GGAGATTCCTCAGCCCATCCCGGTTGACGCCATGCCGACGTTCACAGTGCTTA
CGCCTCACTACAGCGAGAAGgtgcgctttttcctgggcgcattcaacattagctgactgtcgtgcacagA
TCCTTCTTTCGCTCCGTGAGATTATCCGCGAGGAGGACCAGAACACCCGCGTG
ACATTGCTTGAGTATCTCAAGCAGCTTCACCCGGTCGAGTGGGAGAACTTCGTC
AAGGACACCAAGATTTTGGCCGAGGAGTCCGCTATGTTCAACGGTCCAAGTCCT
TTCGGCAACGATGAGAAGGGTCAGTCCAAGATGGACGATCTTCCTTTCTACTGC
ATCGGTTTCAAGAGCGCCGCGCCCGAGTACACCCTCCGCACCCGTATCTGGGC
GTCCTTGCGCGCGCAGACCCTCTACCGCACGGTCTCCGGCATGATGAACTATG
CGAAGGCGATTAAGCTGCTCTACCGCGTCGAGAACCCCGAGGTCGTGCAGCAG
TTCGGCGGTAACACGGACAAGCTCGAGCGCGAGTTGGAGCGGATGGCCCGGC
GGAAGTTCAAGTTCCTGGTGTCCATGCAGCGCTACTCGAAGTTCAACAAGGAGG
AGCACGAGAACGCCGAGTTCTTGCTCCGCGCGTACCCGGACCTGCAGATCGCG
TACCTGGAGGAAGAGCCTCCTCGCAAGGAGGGTGGCGATCCACGCATCTTCTC
TGCCCTCGTCGACGGCCACAGCGACATCATCCCGGAGACCGGCAAGCGGCGC
CCCAAGTTCCGCATCGAGCTGCCCGGCAACCCCATTCTCGGTGACGGCAAGTC
GGACAACCAGAACCACGCCATCGTCTTCTACCGCGGCGAGTACCTCCAGCTTAT
CGACGCCAACCAGGACAACTACCTCGAGGAGTGCTTGAAGATCCGTAACGTAC
TCGCCGAGTTCGAGGAGTACGACGTCTCTAGCCAGAGTCCGTACGCGCAGTGG
AGTGTCAAGGAGTTCAAGCGCTCCCCGGTCGCCATCGTCGGTGCACGCGAGTA
TATCTTCTCGGAGCACATCGGTATTCTCGGTGATTTGGCGGCTGGCAAGGAACA
GACGTTCGGTACGCTCACGGCACGCAACAACGCCTTCCTTGGCGGCAAGCTGC
ACTACGGTCACCCGGATTTCCTCAACGCCCTCTACATGAACACGCGCGGTGGT
GTCTCCAAGGCGCAGAAGGGTCTCCATCTCAACGAGGATATTTACGCCGGTATG
AACGCGGTCGGTCGCGGTGGACGCATCAAGCATAGCGAATACTACCAGTGCGG
CAAGGGTCGTGACCTCGGTTTTGGCACCATCTTGAACTTCCAGACCAAGATCGG
TACGGGTATGGGCGAGCAGATCCTCTCGCGCGAGTACTACTACCTCGGAACCC
AATTGCCCATCGATCGCTTCCTCACGTTCTACTACGCGCACCCAGGTTTCCAGA
TCAACAACATGCTGGTTATCCTATCCGTGCAGGTCTTCATCGTTACCAgtacgttgatt
gcatatcgttagcctgacagcgtctgacgaattcccagTGGTCTTCCTCGGTACCTTGAAGTCTTC
GGTCACGATCTGCAAGTACACGTCCAGCGGTCAGTACATCGGTGGTCAATCCG
GTTGCTACAACCTCGTCCCGGTCTTCCAGTGGATCGAGCGCTGCATCATCAGCA
TCTTCTTGGTGTTCATGATCGCTTTCATGCCGCTCTTCCTGCAAGgtaagagctcgtca
acctgctcaagggccttgcgctgatcatcatctcagAACTCGTCGAGCGCGGTACCTGGAGTGCC
ATCTGGCGTCTGCTCAAGCAGTTTATGTCGCTGTCGCCTGTCTTCGAGGTGTTC
TCCACCCAGATTCAGACACACTCCGTGTTGAGCAACTTGACGTTCGGTGGTGCG
CGTTACATCGCTACCGGTCGTGGGTTCGCCACCAGTCGTATCAGCTTCAGCATC
TTGTTCTCGCGTTTCGCAGGCCCGAGTATCTACCTCGGCATGCGCACGCTCATT
ATGCTGCTCTACGTGACGTTGACGATCTGGACGCCATGGGTCATTTACTTCTGG
 
GTTTCCATTCTCTCGCTCTGCATCGCGCCGTTCTTGTTCAATCCGCATCAATTCG
TCTTCTCGGATTTCCTCATCGACTACAGgtacgtcggacgagcgctgttccgcgacgtaagctgac
cggttatacagGGAATACCTCCGGTGGATGTCGCGTGGTAACTCGCGCTCGCACAAC
AACTCCTGGATTGGGTACTGCCGGTTGTCCCGCACGATGATCACTGGGTACAA
GAAGAAGAAGCTGGGCCACCCGTCGGAGAAGCTTTCCGGCGACGTTCCTCGTG
CAGGCTGGCGCGCCGTCTTATTCTCGGAGATCATCTTCCCGGCATGCATGGCC
ATCCTCTTCATCATCGCGTACATGTTCGTCAAGTCGTTCCCTCTCGACGGCAAG
CAGCCTCCCTCCGGCCTCGTTCGCATCGCCGTCGTGTCTATCGGCCCCATCGT
GTGGAACGCCGCCATCCTGTTGACGCTCTTCCTTGTGTCGTTGTTCCTCGGCCC
CATGCTCGACCCGGTCTTCCCCCTCTTCGGTTCCGTTATGGCCTTCATCGCGCA
TTTCCTCGGCACAATCGGAATGATTGGGTTCTTCGAGTTCCTGgtatgtgcccatacctttt
cattcgtcttcaactatctaacagattcatagTGGTTCCTCGAGTCCTGGGAGGCGTCGCATGCC
GTGCTGGGTCTCATCGCCGTCATCTCCATCCAGCGCGCCATTCACAAAATTCTT
ATCGCCGTTTTCCTCAGTCGCGAGTTCAAGCACGACGAGACGAACAGGGCTTG
GTGGACTGGTCGCTGGTATGGCCGTGGCCTCGGCACGCACGCCATGTCGCAG
CCGGCGCGTGAGTTCGTCGTCAAGATCATCGAGTTGTCGCTCTGGAGCTCGGA
TCTCATACTCGGCCACATCCTGCTGTTCATGCTTACTCCGGCTGTCCTCATCCC
GTACTTCGACCGTCTGCACGCCATGATGCTCTgtacgtcgtgtctcattgtttgtgttggtcatactct
taccctctcttagTCTGGCTGCGCCCCTCAAAGCAAATCCGCGCGCCTCTGTACTCAAT
CAAGCAGAAGAGGCAAAGACGCTGGATTgtcagtgttcagtgccttattctatcagctcttactgacgt
cttcatagATCATGAAGTACGGTACTGTATACGTTACCGTCATCGCGATCTTCGTCG
CGCTCATCGCGCTTCgtgagtacccttgctatctttcgtacctgagcgtcgctgacccctttcccagCCCTC
GTCTTCCGACACACTCTAAAGGTCGAGTGCTCCCTTTGCGACAGCTTGTAATAT
CGGACTCGTATATATCTAGACTTCTCCGCACCATGTGTAGCTGACGCTTGGGTA
TACTTCGCGGTGCCGAGCTAATTGTCGACGGACATTCTCCATCGTTGAGTGCAG
CGACATCGGGTGGTTTACGACACGGACACTTTTCATTGTACCCTCTACGAATGC
AAGAACTCTCTTACGACCAGTACCTATGTGCTAAGCCGTCGCCTGTTCAGGATC
ATACATACATACGTTTCTAGATACCTTACAGTTAGGCCTATTCAGGGAGAGTCTG
CATAAAA
**SEQ ID NO: 4**
   Translation of SEQ ID NO: 7
   amino acid
   *S*. *commune* 
MRNMFDFTMQLLDSRASRMTPNQALLTLHADYIGGQHANYRKWYFAAQLDLDDAV
GQTQNPGLNRLKSTRGSGKRPRHEKSLNTALERWRQAMNNMSQYDRLRQIALYLL
CWGEAAQVRFMPECLCFIFKCADDYYRSPECQNRMEPVPEGLYLRTVVKPLYRFV
RDQGYEVVEGKFVRRERDHDQIIGYDDVNQLFWYPEGIARIVLSDKSRLVDLPPAQ
RFMKFDRIEWNRVFFKTFYETRSFTHLLVDFNRIWVVHIALYFFYTAYNSPTIYAING
NTPTSLAWSATALGGAVATGIMILATIAEFSHIPTTWNNTSHLTRRLAFLLVTLGLTCG
PTFYVAIAESNGSGGSLALILGIVQFFISVVATALFTIMPSGRMFGDRVAGKSRKYLA
SQTFTASYPSLPKHQRFASLLMWFLIFGCKLTESYFFLTLSFRDPIRVMVGMKIQNC
EDKIFGSGLCRNHAAFTLTIMYIMDLVLFFLDTFLWYVIWNSVFSIARSFVLGLSIWTP
WRDIFQRLPKRIYAKLLATGDMEVKYKPKVLVSQIWNAIIISMYREHLLSIEHVQKLLY
HQVDTGEAGKRSLRAPPFFVAQGSSGGSGEFFPPGSEAERRISFFAQSLSTEIPQPI
PVDAMPTFTVLTPHYSEKILLSLREIIREEDQNTRVTLLEYLKQLHPVEWENFVKDTKI
LAEESAMFNGPSPFGNDEKGQSKMDDLPFYCIGFKSAAPEYTLRTRIWASLRAQTL
YRTVSGMMNYAKAIKLLYRVENPEVVQQFGGNTDKLERELERMARRKFKFLVSMQ
RYSKFNKEEHENAEFLLRAYPDLQIAYLEEEPPRKEGGDPRIFSALVDGHSDIIPETG
 
KRRPKFRIELPGNPILGDGKSDNQNHAIVFYRGEYLQLIDANQDNYLEECLKIRNVLA
EFEEYDVSSQSPYAQWSVKEFKRSPVAIVGAREYIFSEHIGILGDLAAGKEQTFGTL
TARNNAFLGGKLHYGHPDFLNALYMNTRGGVSKAQKGLHLNEDIYAGMNAVGRGG
RIKHSEYYQCGKGRDLGFGTILNFQTKIGTGMGEQILSREYYYLGTQLPIDRFLTFYY
AHPGFQINNMLVILSVQVFIVTMVFLGTLKSSVTICKYTSSGQYIGGQSGCYNLVPVF
QWIERCIISIFLVFMIAFMPLFLQELVERGTWSAIWRLLKQFMSLSPVFEVFSTQIQTH
SVLSNLTFGGARYIATGRGFATSRISFSILFSRFAGPSIYLGMRTLIMLLYVTLTIWTP
WVIYFWVSILSLCIAPFLFNPHQFVFSDFLIDYREYLRWMSRGNSRSHNNSWIGYCR
LSRTMITGYKKKKLGHPSEKLSGDVPRAGWRAVLFSEIIFPACMAILFIIAYMFVKSFP
LDGKQPPSGLVRIAVVSIGPIVWNAAILLTLFLVSLFLGPMLDPVFPLFGSVMAFIAHF
LGTIGMIGFFEFLWFLESWEASHAVLGLIAVISIQRAIHKILIAVFLSREFKHDETNRAW
WTGRWYGRGLGTHAMSQPAREFVVKIIELSLWSSDLILGHILLFMLTPAVLIPYFDRL
HAMMLFWLRPSKQIRAPLYSIKQKRQRRWIIMKYGTVYVTVIAIFVALIALPLVFRHTL
KVECSLCDSL
**SEQ ID NO: 5**
   cDNA 1,3-β-D-glucan synthase I of *S. commune* strain Lu15531
   DNA
   *S*. *commune* 
ATGTCCGGCCCAGGATATGGCAGGAATCCATTCGACAATCCCCCGCCCAACAG
AGGTCCCTATGGCCAGCAGCCAGGTTTCCCGGGGCCCGGCCCTCGGCCTTAC
GACTCGGACGCGGACATGAGCCAGACCTATGGCAGCACAACCAGGCTCGCCG
GCAGTGCCGGTTACAGCGACAGAAACGGCAGCTTCGACGGCGACCGCTCCTAC
GCGCCCTCAATTGACTCGCGCGCCAGCGTGCCCAGCATATCGCCCTTCGCAGA
CCCGGGTATCGGCTCTAATGAGCCGTATCCCGCTTGGTCGGTCGAACGCCAGA
TTCCCATGTCCACGGAGGAGATTGAGGACATCTTCCTCGACCTCACCCAAAAGT
TTGGCTTCCAGCGCGACTCCATGCGGAATACGTTCGACTTCATGATGCACCTCC
TCGATTCCCGTGCCTCGCGCATGACGCCCAACCAAGCTCTGCTCACGCTTCAC
GCCGACTACATTGGTGGCCAGCATGCCAATTACCGGAAGTGGTATTTCGCCGC
ACAGCTCAACCTCGATGACGCGGTCGGGCAAACCAATAACCCCGGTATCCAGC
GCTTGAAGACCATCAAGGGCGCTACGAAGACCAAGTCGCTCGACAGCGCACTC
AACCGCTGGCGCAACGCGATGAACAACATGAGCCAGTACGATCGCCTCCGGCA
AATTGCGCTCTACCTCCTCTGCTGGGGTGAAGCAGGCAACATCCGTCTGGCGC
CCGAGTGCTTGTGCTTCATCTTCAAGTGCGCGGACGACTACTACAGAAGTCCCG
AGTGTCAGAACCGGATGGACCCCGTGCCGGAAGGGCTGTACCTGCAGACGGT
CATCAAGCCGCTCTATCGCTTCCTACGTGATCAGGCGTACGAAGTCGTTGATGG
GAAGCAAGTGAAGCGCGAGAAGGACCACGACCAGATTATCGGTTATGACGACG
TCAACCAGTTATTCTGGTATCCGGAAGGTTTGGCTAAGATCGTCATGTCGGACA
ACACACGACTTGTAGATGTACCTCCGGCGCAGCGGTTCATGAAGTTCGCCAAGA
TCGAGTGGAACCGCGTCTTCTTCAAGACGTACTTTGAGAAGCGCTCTACTGCCC
ATCTCCTGGTCAACTTCAACCGTATATGGATCCTCCACGTCTCGATGTACTTCTT
CTACACGGCATTCAACTCTCCACGAGTCTACGCGCCGCACGGCAAACTCGACC
CCTCCCCTGAGATGACCTGGTCCGCGACTGCCCTTGGAGGCGCTGTGTCCACC
ATGATCATGATCCTTGCCACTATCGCGGAGTACACCTACATCCCCACGACATGG
AACAATGCGTCGCACCTCACCACGCGGCTCATTTTCCTCCTGGTCATCCTCGCG
CTCACTGCTGGCCCAACATTCTATATCGCCATGATAGACGGACGCACGGACATC
GGCCAAGTACCACTCATCGTGGCCATAGTGCAGTTCTTCATCTCCGTCGTCGCC
ACCCTCGCTTTCGCTACCATCCCTTCTGGTCGCATGTTCGGCGACCGTGTGGCT
GGCAAGTCAAGAAAGCACATGGCATCGCAGACGTTCACAGCGTCGTACCCGTC
 
CATGAAGCGGTCATCTCGCGTAGCGAGTATCATGCTGTGGCTTTTGGTCTTTGG
CTGCAAATACGTCGAGTCTTACTTCTTCTTGACGTCCTCCTTCTCCAGCCCGATC
GCGGTCATGGCGCGTACGAAGGTACAGGGCTGCAACGACCGTATCTTCGGCAG
CCAGCTGTGCACGAATCAGGTCCCGTTCGCGCTGGCAATCATGTACGTGATGG
ACCTGGTACTGTTCTTCCTGGACACGTACCTGTGGTACATCATCTGGCTGGTGA
TCTTCTCGATGGTGCGCGCGTTCAAGCTTGGTATCTCGATCTGGACGCCCTGGA
GCGAGATCTTCACCCGCATGCCGAAGCGTATTTACGCAAAGCTGCTGGCGACG
GCCGAGATGGAGGTCAAGTATAAGCCCAAGGTGCTCGTCTCACAAATCTGGAA
CGCGGTCATCATCTCCATGTACCGGGAGCATCTCTTGTCCATCGAGCACGTCCA
GCGCTTGCTTTACCACCAGGTTGATGGTCCCGATGGCCGCCGCACCCTCAGGG
CACCGCCGTTCTTCACCAGCCAGCGAACTGCGAAGCCAGGCCTGTTCTTCCCT
CCTGGTGGCGAGGCTGAGCGCCGCATCTCGTTCTTTGCCTCATCGCTGACGAC
CGCGCTCCCGGAGCCTCTGCCGATCGACGCCATGCCCACCTTCACCGTGCTCG
TTCCCCATTACTCCGAGAAGATTCTGCTCAGTCTGCGCGAGATTATCCGCGAGG
AGGACCAGAACACCCGCGTTACCTTACTGGAGTACCTCAAGCAGCTCCACCCT
GTCGAATGGGACAATTTCGTCAAGGACACCAAGATCTTGGCGGAAGAGTCGGG
AGACGTCCAGGACGAGAAGCGCGCGCACGGACGACTTGCCGTTCTATTGCA
TCGGGTTCAAGACCTCGTCACCAGAGTACACCCTGCGTACGCGTATCTGGGCC
TCACTGCGCGCACAGACGCTGTACCGCACGGTCTCCGGTATGATGAACTACTC
CAAGGCGATTAAGCTCCTCTATCGCGTCGAGAACCCGGATGTCGTTCATGCCTT
CGGTGGGAACACGGAACGTCTTGAACGCGAGCTTGAGCGCATGTCTCGCCGCA
AGTTCAAGTTCGTCATCTCGATGCAGCGGTACTCCAAGTTCAACAAGGAGGAGC
AGGAGAACGCCGAGTTCCTTCTGCGCGCGTACCCGGATTTGCAGATCGCGTAC
CTCGATGAAGAGCCCGGTCCCAGCAAGAGCGACGAGGTTCGGTTGTTTTCGAC
ACTCATCGACGGACACTCCGAGGTGGACGAGAAGACGGGCCGCCGCAAGCCC
AAGTTCCGCATCGAGCTGCCCGGTAACCCCATCCTCGGTGACGGAAGTCGGA
TAACCAGAACCACGCCATCGTCTTCTACCGCGGCGAGTACATTCAGGTCATTGA
CGCTAACCAGGACAATTACCTGGAAGAGTGTCTCAAGATCCGTAATGTCCTGGG
CGAGTTTGAGGAATACTCCGTGTCGAGCCAGAGCCCGTACGCGCAGTGGGGCC
ACAAGGAGTTCAACAAGTGCCCCGTCGCTATCCTGGGTTCCCGCGAGTACATCT
TCTCGGAGAACATCGGTATCCTCGGTGACATCGCTGCCGGCAAGGAACAGACG
TTCGGTACCATTACGGCGCGTGCGCTTGCGTGGATCGGCGGCAAGCTGCATTA
CGGTCACCCGGATTTCCTCAATGCGACGTTCATGACGACGCGTGGTGGCGTGT
CAAAAGCGCAGAAGGGCTTGCATCTTAACGAGGATATCTTCGCTGGTATGACCG
CCGTGTCCCGCGGAGGGCGCATCAAGCACATGGAGTACTACCAGTGCGGCAAA
GGTCGTGATCTCGGATTCGGCACGATCTTGAACTTCCAGACCAAGATCGGTACT
GGTATGGGCGAGCAGCTGCTCTCGCGCGAGTACTACTATCTGGGCACGCAATT
GCCTATCGACCGGTTCTTGACGTTCTACTACGCGCACGCTGGTTTCCATGTCAA
CAACATCCTGGTCATCTACTCCATCCAGGTCTTCATGGTCACCCTGCTGTACCT
GGGCACATTGAACAAGCAGCTGTTCATCTGCAAGGTCAACTCCAATGGCCAGGT
TCTTAGTGGACAAGCTGGGTGCTACAACCTCATCCCGGTCTTCGAGTGGATTCG
CCGGAGTATCATCTCCATCTTCTTGGTGTTCTTCATCGCCTTCTTGCCGTTGTTC
TTGCAAGAGCTTTGCGAACGCGGAACAGGAAAGGCGTTGCTGCGTCTCGGGAA
GCACTTCCTGTCACTGTCGCCCATCTTCGAAGTGTTCTCCACCCAAATCTACTC
GCAGGCGCTCTTGAACAACATGAGTTTCGGTGGTGCGCGCTACATCGCTACAG
GACGCGGTTTCGCGACGAGTCGGATACCCTTCAACATCCTCTACTCGCGTTTCG
CGCCGCCGAGCATCTACATGGGCATGCGTAATCTGCTGCTCTTGCTGTACGCG
ACGATGGCCATTTGGATCCCACACCTGATCTACTTCTGGTTCTCCGTCCTCTCC
CTCTGCATCGCGCCATTCATGTTCAATCCGCATCAATTCTCGTACGCTGACTTCA
TCATCGACTACCGGGAGTTCTTGCGCTGGATGTCGCGCGGTAACTCGCGGACG
 
AAGGCGAGTAGCTGGTACGGATATTGCCGTCTGTCGCGTACCGCGATTACTGG
GTACAAGAAGAAGAAACTGGGACACCCGTCGGAGAAGCTGTCGGGCGATGTGC
CGCGTGCGCCGTGGAGGAACGTCATCTTCTCGGAGATCCTTTGGCCCATCGGC
GCGTGCATCATCTTCATCGTCGCGTACATGTTCGTCAAATCGTTCCCTGACGAG
CAGGGCAACGCGCCGCCGAGCCCGCTGGTCCGCATTCTGCTCATCGCGGTTG
GCCCTACTGTGTGGAACGCGGCGGTGCTCATCACGCTGTTCTTCCTGTCGCTCT
TCCTGGGCCCGATGATGGATGGCTGGGTCAAGTTCGGCTCAGTCATGGCGGCA
CTTGCGCATGGTCTAGCGCTCATAGGCATGCTCACGTTCTTCGAGTTCTTCTGG
TTCCTCGAGCTCTGGGATGCCTCGCACGCCGTGCTCGGCGTCATCGCCATTATT
GCCGTTCAGCGCGGATCCAGAAGATCCTCATTGCCGTCTTCCTGACGCGTGA
GTACAAGCACGACGAGACGAACCGCGCGTGGTGGACAGGTAAATGGTATGGAC
GCGGGCTGGGTACCTCGGCCATGTCCCAGCCGGCGCGCGAGTTCATCGTGAA
GATCGTGGAGATGTCGCTGTGGACGTCGGACTTCCTGCTTGCGCACCTGTTGC
TCATCATCTTGACGGTGCCGCTACTGCTGCCGTTCTTCAACTCGATCCATTCGA
CGATGCTTTTCTGGTTGCGCCCTTCGAAGCAGATTAGGCAACCTCTGTTCTCCA
CTAAGCAGAAGCGGCAACGGCGATGGATTGTCATGAAGTATACCGTGGTATATC
TCGTGGTGGTGGCTTTCCTCGTTGCGCTCATCGCTCTGCCCGCGCTCTTCCGC
GAGAGCATCCACTTCAACTGCGAGATCTGCCAGAGTATATAG
**SEQ ID NO: 6**
   polypeptide sequence 1,3-β-D-glucan synthase I of *S. commune* strain Lu15531
   amino acid
   *S*. *commune* 
MSGPGYGRNPFDNPPPNRGPYGQQPGFPGPGPRPYDSDADMSQTYGSTTRLAG
SAGYSDRNGSFDGDRSYAPSIDSRASVPSISPFADPGIGSNEPYPAWSVERQIPMS
TEEIEDIFLDLTQKFGFQRDSMRNTFDFMMHLLDSRASRMTPNQALLTLHADYIGGQ
HANYRKWYFAAQLNLDDAVGQTNNPGIQRLKTIKGATKTKSLDSALNRWRNAMNN
MSQYDRLRQIALYLLCWGEAGNIRLAPECLCFIFKCADDYYRSPECQNRMDPVPEG
LYLQTVIKPLYRFLRDQAYEVVDGKQVKREKDHDQIIGYDDVNQLFWYPEGLAKIVM
SDNTRLVDVPPAQRFMKFAKIEWNRVFFKTYFEKRSTAHLLVNFNRIWILHVSMYFF
YTAFNSPRVYAPHGKLDPSPEMTWSATALGGAVSTMIMILATIAEYTYIPTTWNNAS
HLTTRLIFLLVILALTAGPTFYIAMIDGRTDIGQVPLIVAIVQFFISVVATLAFATIPSGRM
FGDRVAGKSRKHMASQTFTASYPSMKRSSRVASIMLWLLVFGCKYVESYFFLTSSF
SSPIAVMARTKVQGCNDRIFGSQLCTNQVPFALAIMYVMDLVLFFLDTYLWYIIWLVI
FSMVRAFKLGISIWTPWSEIFTRMPKRIYAKLLATAEMEVKYKPKVLVSQIWNAVIISM
YREHLLSIEHVQRLLYHQVDGPDGRRTLRAPPFFTSQRTAKPGLFFPPGGEAERRIS
FFASSLTTALPEPLPIDAMPTFTVLVPHYSEKILLSLREIIREEDQNTRVTLLEYLKQLH
PVEWDNFVKDTKILAEESGDVQDEKRARTDDLPFYCIGFKTSSPEYTLRTRIWASLR
AQTLYRTVSGMMNYSKAIKLLYRVENPDVVHAFGGNTERLERELERMSRRKFKFVI
SMQRYSKFNKEEQENAEFLLRAYPDLQIAYLDEEPGPSKSDEVRLFSTLIDGHSEVD
EKTGRRKPKFRIELPGNPILGDGKSDNQNHAIVFYRGEYIQVIDANQDNYLEECLKIR
NVLGEFEEYSVSSQSPYAQWGHKEFNKCPVAILGSREYIFSENIGILGDIAAGKEQTF
GTITARALAWIGGKLHYGHPDFLNATFMTTRGGVSKAQKGLHLNEDIFAGMTAVSR
GGRIKHMEYYQCGKGRDLGFGTILNFQTKIGTGMGEQLLSREYYYLGTQLPIDRFLT
FYYAHAGFHVNNILVIYSIQVFMVTLLYLGTLNKQLFICKVNSNGQVLSGQAGCYNLI
PVFEWIRRSIISIFLVFFIAFLPLFLQELCERGTGKALLRLGKHFLSLSPIFEVFSTQIYS
QALLNNMSFGGARYIATGRGFATSRIPFNILYSRFAPPSIYMGMRNLLLLLYATMAIW
IPHLIYFWFSVLSLCIAPFMFNPHQFSYADFIIDYREFLRWMSRGNSRTKASSWYGY
CRLSRTAITGYKKKKLGHPSEKLSGDVPRAPWRNVIFSEILWPIGACIIFIVAYMFVKS
 
FPDEQGNAPPSPLVRILLIAVGPTVWNAAVLITLFFLSLFLGPMMDGWVKFGSVMAA
LAHGLALIGMLTFFEFFWFLELWDASHAVLGVIAIIAVQRGIQKILIAVFLTREYKHDET
NRAWWTGKWYGRGLGTSAMSQPAREFIVKIVEMSLWTSDFLLAHLLLIILTVPLLLP
FFNSIHSTMLFWLRPSKQIRQPLFSTKQKRQRRWIVMKYTVVYLVVVAFLVALIALPA
LFRESIHFNCEICQSI
**SEQ ID NO: 7**
   cDNA 1,3-β-D-glucan synthase II of *S. commune* strain Lu15531
   DNA
   *S. commune* 
ATGCGGAACATGTTCGACTTCACCATGCAGCTGCTTGACAGCCGAGCGTCTCGT
ATGACCCCCAACCAGGCGCTCCTCACCCTCCACGCCGACTACATTGGTGGCCA
GCATGCGAACTACCGGAAGTGGTACTTCGCGGCGCAGCTCGACCTTGACGACG
CCGTGGGACAAACTCAGAATCCGGGTCTCAACCGCCTCAAGTCCACTCGCGGA
TCGGGCAAGCGACCACGCCATGAAAAGTCGCTGAACACGGCATTGGAGCGCTG
GCGGCAAGCCATGAACAACATGTCGCAGTATGACCGCTTACGCCAGATCGCGC
TCTACCTGCTCTGCTGGGGCGAAGCGGCGCAAGTGCGATTCATGCCCGAGTGC
TTGTGCTTCATCTTCAAGTGCGCCGACGACTATTATCGTTCGCCGGAGTGCCAG
AACAGGATGGAGCCGGTACCGGAGGGTCTCTACCTGAGGACGGTCGTAAAGCC
GCTCTACAGATTTGTCCGGGATCAAGGCTATGAGGTGGTGGAGGGAAAATTCGT
ACGGCGGGAACGGGATCACGACCAAATCATTGGTTACGATGACGTGAATCAGC
TGTTCTGGTACCCGGAGGGCATTGCCCGTATCGTCCTGTCGGACAAGAGTCGT
CTGGTCGACCTCCCTCCAGCACAGCGCTTCATGAAGTTCGACCGTATCGAGTG
GAATCGCGTCTTCTTCAAGACGTTCTACGAGACTCGATCCTTTACGCATCTTTTG
GTCGACTTCAACCGTATCTGGGTCGTGCACATCGCTCTCTACTTCTTCTACACC
GCATACAACTCCCCCACGATCTACGCCATCAACGGCAACACTCCGACGTCTCTG
GCTTGGAGCGCGACTGCGCTCGGCGGTGCGGTAGCGACAGGTATCATGATCCT
CGCCACGATCGCCGAGTTCTCGCACATCCCCACGACATGGAACAACACCTCGC
ATCTGACTCGCCGCCTCGCCTTCCTCCTCGTCACGCTCGGCCTCACATGTGGTC
CGACGTTCTACGTCGCGATTGCAGAGAGCAACGGAGCGGCGGCTCTTTGGCC
TTGATTCTCGGCATCGTCCAGTTCTTCATCTCCGTCGTAGCGACTGCGCTCTTC
ACTATCATGCCTTCTGGTCGTATGTTCGGCGACCGCGTCGCAGGCAAGAGTCG
CAAGTATCTCGCCAGCCAGACGTTCACGGCCAGCTACCCGTCGTTGCCCAAGC
ACCAGCGGTTCGCATCACTCCTGATGTGGTTCCTCATCTTCGGGTGCAAGTTGA
CGGAGAGTTACTTCTTCCTGACGTTGTCCTTCCGCGACCCTATTCGCGTCATGG
TCGGCATGAAGATCCAGAACTGCGAGGACAAGATTTTCGGCAGCGGCCTTTGC
AGGAATCACGCAGCATTCACCCTCACGATCATGTACATCATGGACCTCGTCTTG
TTCTTCCTCGACACCTTCCTTTGGTATGTCATCTGGAACTCGGTTTTCAGTATCG
CACGCTCTTTCGTACTCGGCCTTTCGATCTGGACACCATGGAGGGACATCTTCC
AGCGTCTGCCGAAGCGTATCTACGCGAAGCTTCTAGCGACCGGCGACATGGAG
GTCAAGTACAAGCCCAAGGTCTTGGTTTCGCAAATCTGGAACGCCATCATCATC
TCCATGTACCGCGAGCACTTGCTCTCTATCGAGCACGTTCAAAAGCTCCTGTAC
CATCAAGTGGACACTGGCGAAGCCGGCAAGCGGAGTCTTCGCGCGCCTCCGTT
CTTCGTCGCGCAGGGCAGCAGCGGTGGCTCGGGCGAGTTCTTCCCGCCTGGT
AGCGAGGCTGAGCGTCGTATCTCTTTCTTCGCGCAGTCTCTATCTACGGAGATT
CCTCAGCCCATCCCGGTTGACGCCATGCCGACGTTCACAGTGCTTACGCCTCA
 
CTACAGCGAGAAGATCCTTCTTTCGCTCCGTGAGATTATCCGCGAGGAGGACCA
GAACACCCGCGTGACATTGCTTGAGTATCTCAAGCAGCTTCACCCGGTCGAGTG
GGAGAACTTCGTCAAGGACACCAAGATTTTGGCCGAGGAGTCCGCTATGTTCAA
CGGTCCAAGTCCTTTCGGCAACGATGAGAAGGGTCAGTCCAAGATGGACGATC
TTCCTTTCTACTGCATCGGTTTCAAGAGCGCCGCGCCCGAGTACACCCTCCGCA
CCCGTATCTGGGCGTCCTTGCGCGCGCAGACCCTCTACCGCACGGTCTCCGGC
ATGATGAACTATGCGAAGGCGATTAAGCTGCTCTACCGCGTCGAGAACCCCGA
GGTCGTGCAGCAGTTCGGCGGTAACACGGACAAGCTCGAGCGCGAGTTGGAG
CGGATGGCCCGGCGGAAGTTCAAGTTCCTGGTGTCCATGCAGCGCTACTCGAA
GTTCAACAAGGAGGAGCACGAGAACGCCGAGTTCTTGCTCCGCGCGTACCCGG
ACCTGCAGATCGCGTACCTGGAGGAAGAGCCTCCTCGCAAGGAGGGTGGCGAT
CCACGCATCTTCTCTGCCCTCGTCGACGGCCACAGCGACATCATCCCGGAGAC
CGGCAAGCGGCGCCCCAAGTTCCGCATCGAGCTGCCCGGCAACCCCATTCTCG
GTGACGGCAAGTCGGACAACCAGAACCACGCCATCGTCTTCTACCGCGGCGAG
TACCTCCAGCTTATCGACGCCAACCAGGACAACTACCTCGAGGAGTGCTTGAAG
ATCCGTAACGTACTCGCCGAGTTCGAGGAGTACGACGTCTCTAGCCAGAGTCC
GTACGCGCAGTGGAGTGTCAAGGAGTTCAAGCGCTCCCCGGTCGCCATCGTCG
GTGCACGCGAGTATATCTTCTCGGAGCACATCGGTATTCTCGGTGATTTGGCGG
CTGGCAAGGAACAGACGTTCGGTACGCTCACGGCACGCAACAACGCCTTCCTT
GGCGGCAAGCTGCACTACGGTCACCCGGATTTCCTCAACGCCCTCTACATGAA
CACGCGCGGTGGTGTCTCCAAGGCGCAGAAGGGTCTCCATCTCAACGAGGATA
TTTACGCCGGTATGAACGCGGTCGGTCGCGGTGGACGCATCAAGCATAGCGAA
TACTACCAGTGCGGCAAGGGTCGTGACCTCGGTTTTGGCACCATCTTGAACTTC
CAGACCAAGATCGGTACGGGTATGGGCGAGCAGATCCTCTCGCGCGAGTACTA
CTACCTCGGAACCCAATTGCCCATCGATCGCTTCCTCACGTTCTACTACGCGCA
CCCAGGTTTCCAGATCAACAACATGCTGGTTATCCTATCCGTGCAGGTCTTCAT
CGTTACCATGGTCTTCCTCGGTACCTTGAAGTCTTCGGTCACGATCTGCAAGTA
CACGTCCAGCGGTCAGTACATCGGTGGTCAATCCGGTTGCTACAACCTCGTCC
CGGTCTTCCAGTGGATCGAGCGCTGCATCATCAGCATCTTCTTGGTGTTCATGA
TCGCTTTCATGCCGCTCTTCCTGCAAGAACTCGTCGAGCGCGGTACCTGGAGT
GCCATCTGGCGTCTGCTCAAGCAGTTTATGTCGCTGTCGCCTGTCTTCGAGGTG
TTCTCCACCCAGATTCAGACACACTCCGTGTTGAGCAACTTGACGTTCGGTGGT
GCGCGTTACATCGCTACCGGTCGTGGGTTCGCCACCAGTCGTATCAGCTTCAG
CATCTTGTTCTCGCGTTTCGCAGGCCCGAGTATCTACCTCGGCATGCGCACGCT
CATTATGCTGCTCTACGTGACGTTGACGATCTGGACGCCATGGGTCATTTACTT
CTGGGTTTCCATTCTCTCGCTCTGCATCGCGCCGTTCTTGTTCAATCCGCATCAA
TTCGTCTTCTCGGATTTCCTCATCGACTACAGGGAATACCTCCGGTGGATGTCG
CGTGGTAACTCGCGCTCGCACAACAACTCCTGGATTGGGTACTGCCGGTTGTC
CCGCACGATGATCACTGGGTACAAGAAGAAGAAGCTGGGCCACCCGTCGGAGA
AGCTTTCCGGCGACGTTCCTCGTGCAGGCTGGCGCGCCGTCTTATTCTCGGAG
ATCATCTTCCCGGCATGCATGGCCATCCTCTTCATCATCGCGTACATGTTCGTCA
AGTCGTTCCCTCTCGACGGCAAGCAGCCTCCCTCCGGCCTCGTTCGCATCGCC
GTCGTGTCTATCGGCCCCATCGTGTGGAACGCCGCCATCCTGTTGACGCTCTTC
CTTGTGTCGTTGTTCCTCGGCCCCATGCTCGACCCGGTCTTCCCCCTCTTCGGT
TCCGTTATGGCCTTCATCGCGCATTTCCTCGGCACAATCGGAATGATTGGGTTC
TTCGAGTTCCTGTGGTTCCTCGAGTCCTGGGAGGCGTCGCATGCCGTGCTGGG
TCTCATCGCCGTCATCTCCATCCAGCGCGCCATTCACAAAATTCTTATCGCCGTT
TTCCTCAGTCGCGAGTTCAAGCACGACGAGACGAACAGGGCTTGGTGGACTGG
TCGCTGGTATGGCCGTGGCCTCGGCACGCACGCCATGTCGCAGCCGGCGCGT
GAGTTCGTCGTCAAGATCATCGAGTTGTCGCTCTGGAGCTCGGATCTCATACTC
 
GGCCACATCCTGCTGTTCATGCTTACTCCGGCTGTCCTCATCCCGTACTTCGAC
CGTCTGCACGCCATGATGCTCTTCTGGCTGCGCCCCTCAAAGCAAATCCGCGC
GCCTCTGTACTCAATCAAGCAGAAGAGGCAAAGACGCTGGATTATCATGAAGTA
CGGTACTGTATACGTTACCGTCATCGCGATCTTCGTCGCGCTCATCGCGCTTCC
CCTCGTCTTCCGACACACTCTAAAGGTCGAGTGCTCCCTTTGCGACAGCTTGTA
A
**SEQ ID NO: 8**
   polypeptide sequence 1,3-β-D-glucan synthase II of *S. commune* strain Lu15531
   amino acid
   *S. commune* 
MRNMFDFTMQLLDSRASRMTPNQALLTLHADYIGGQHANYRKWYFAAQLDLDDAV
GQTQNPGLNRLKSTRGSGKRPRHEKSLNTALERWRQAMNNMSQYDRLRQIALYLL
CWGEAAQVRFMPECLCFIFKCADDYYRSPECQNRMEPVPEGLYLRTVVKPLYRFV
RDQGYEVVEGKFVRRERDHDQIIGYDDVNQLFWYPEGIARIVLSDKSRLVDLPPAQ
RFMKFDRIEWNRVFFKTFYETRSFTHLLVDFNRIWVVHIALYFFYTAYNSPTIYAING
NTPTSLAWSATALGGAVATGIMILATIAEFSHIPTTWNNTSHLTRRLAFLLVTLGLTCG
PTFYVAIAESNGSGGSLALILGIVQFFISVVATALFTIMPSGRMFGDRVAGKSRKYLA
SQTFTASYPSLPKHQRFASLLMWFLIFGCKLTESYFFLTLSFRDPIRVMVGMKIQNC
EDKIFGSGLCRNHAAFTLTIMYIMDLVLFFLDTFLWYVIWNSVFSIARSFVLGLSIWTP
WRDIFQRLPKRIYAKLLATGDMEVKYKPKVLVSQIWNAIIISMYREHLLSIEHVQKLLY
HQVDTGEAGKRSLRAPPFFVAQGSSGGSGEFFPPGSEAERRISFFAQSLSTEIPQPI
PVDAMPTFTVLTPHYSEKILLSLREIIREEDQNTRVTLLEYLKQLHPVEWENFVKDTKI
LAEESAMFNGPSPFGNDEKGQSKMDDLPFYCIGFKSAAPEYTLRTRIWASLRAQTL
YRTVSGMMNYAKAIKLLYRVENPEVVQQFGGNTDKLERELERMARRKFKFLVSMQ
RYSKFNKEEHENAEFLLRAYPDLQIAYLEEEPPRKEGGDPRIFSALVDGHSDIIPETG
KRRPKFRIELPGNPILGDGKSDNQNHAIVFYRGEYLQLIDANQDNYLEECLKIRNVLA
EFEEYDVSSQSPYAQWSVKEFKRSPVAIVGAREYIFSEHIGILGDLAAGKEQTFGTL
TARNNAFLGGKLHYGHPDFLNALYMNTRGGVSKAQKGLHLNEDIYAGMNAVGRGG
RIKHSEYYQCGKGRDLGFGTILNFQTKIGTGMGEQILSREYYYLGTQLPIDRFLTFYY
AHPGFQINNMLVILSVQVFIVTMVFLGTLKSSVTICKYTSSGQYIGGQSGCYNLVPVF
QWIERCIISIFLVFMIAFMPLFLQELVERGTWSAIWRLLKQFMSLSPVFEVFSTQIQTH
SVLSNLTFGGARYIATGRGFATSRISFSILFSRFAGPSIYLGMRTLIMLLYVTLTIWTP
WVIYFWVSILSLCIAPFLFNPHQFVFSDFLIDYREYLRWMSRGNSRSHNNSWIGYCR
LSRTMITGYKKKKLGHPSEKLSGDVPRAGWRAVLFSEIIFPACMAILFIIAYMFVKSFP
LDGKQPPSGLVRIAVVSIGPIVWNAAILLTLFLVSLFLGPMLDPVFPLFGSVMAFIAHF
LGTIGMIGFFEFLWFLESWEASHAVLGLIAVISIQRAIHKILIAVFLSREFKHDETNRAW
WTGRWYGRGLGTHAMSQPAREFVVKIIELSLWSSDLILGHILLFMLTPAVLIPYFDRL
HAMMLFWLRPSKQIRAPLYSIKQKRQRRWIIMKYGTVYVTVIAIFVALIALPLVFRHTL
KVECSLCDSL
**SEQ ID NO: 9**
   Gene sequence 1,3-β-D-glucan synthase I of *S. commune* strain Lu15634
   DNA
   *S. commune* 
CCCGTCCCTCAAGGCCGTTCTTTCGCTGGCGACCGACCCGGTGTTCGCGAGAA
CCTGTTGTTTCTGACGATCATCAACCCTTTCTTCTCGTCGCTCTTTAGCTCTCCC
TAGACCGTCTTTTACTCTACTCTTCGACGCACGCCATGTCCGGTCCAGGATATG
GCAGGAATCCATTCGACAATCCCCCGCCCAACAGAGGTCCCTATGGCCAGCAG
CCAGGTTTCCCGGGGCCCGGCCCTCGGCCTTACGACTCGGACGCGGACATGA
GCCAGACCTATGGCAGCACAACCAGGCTCGCCGGCAGTGCCGGTTACAGCGA
CAGAAACGgtgcgaacgtcgctaccgtacttcctcgatcgtcgactcacatatcacgcagGCAGCTTCGA
CGGCGACCGCTCCTACGCGCCCTCAATTGACTCGCGCGCCAGCGTGCCCAGC
ATATCGCCCTTCGCAGACCCGGGTATCGGCTCTAATGAGCCGTATCCCGCTTG
GTCGGTCGAACGCCAGATCCCCATGTCCACGGAGGAGATTGAGGATATCTTCC
TCGACCTCACCCAAAAGTTTGGCTTCCAGCGCGACTCCATGCGGAATACGgtgcgt
gaataagcagcccactcgaccgcgggaacagctcaattgacctgtcacccagTTCGACTTCATGATGCA
CCTCCTTGATTCCCGTGCCTCGCGCATGACGCCCAACCAAGCTCTGCTCACGCT
TCACGCCGACTACATTGGTGGCCAGCACGCCAACTATAGGAAGTGGTATTTCGC
CGCTCAGCTCAACCTCGATGACGCGGTCGGGCAAACCAATAACCCCGGTATCC
AGCGCTTGAAGACCATCAAGGGCGCTACGAAGACCAAGTCGCTCGACAGCGCA
CTCAACCGCTGGCGCAATGCGATGAACAACATGAGCCAGTACGATCGCCTCCG
GCAAATTGCGCTCTATCTCCTCTGCTGGGGAGAAGCAGGCAACATCCGTCTGG
CGCCCGAGTGCTTGTGCTTCATCTTCAAGTGCGCGGACGACTACTACAGAAGTC
CCGAGTGTCAGAACCGGATGGACCCCGTGCCGGAAGGGCTGTACCTCCAGAC
GGTCATCAAGCCGCTCTATCGCTTCCTACGTGATCAGGCGTACGAAGTCGTTGA
TGGGAAGCAAGTGAAGCGCGAGAAGGACCACGACCAGATTATCGGTTATGACG
ACGTCAACCAGTTATTCTGGTATCCGGAAGGTTTGGCTAAGATCGTCATGTCGG
ACAACgtgcgtatgatcttatcggttacaattcgtccgctcacatctttccagACACGACTTGTAGATGTAC
CTCCGGCGCAGCGGTTCATGAAGTTCGCCAAGATCGAGTGGAACCGCGTCTTC
TTCAAGACGTACTTTGAGAAGCGCTCTACTGCCCATCTCCTGGTCAACTTCAAC
CGTATATGGATCCTCCACGTCTCGATGTACTTCTTCTACACGGCATTCAACTCTC
CACGAGTCTACGCGCCGCACGGCAAACTCGACCCCTCCCCTGAGATGACCTGG
TCCGCGACTGCCCTTGGAGGCGCTGTGTCCACCATGATCATGATCCTTGCCACT
ATCGCGGAGTACACCTACATCCCCACGACATGGAACAATGCGTCGCACCTCAC
CACGCGGCTCATTTTCCTCCTGGTCATCCTCGCGCTCACTGCTGGACCAACATT
CTATATCGCCATGATAGACGGACGCACGGACATCGGCCAAGTACCACTCATCGT
GGCCATAGTGCAGTTCTTCATCTCCGTCGTCGCCACCCTCGCTTTCGCTACCAT
CCCTTCTGGTCGCATGTTCGGCGACCGTGTGGCTGGCAAGTCAAGAAAGCACA
TGGCATCGCAGACGTTCACAGCGTCGTACCCGTCCATGAAGCGGTCATCTCGC
GTAGCGAGTATCATGCTGTGGCTTTTGGTCTTTGGCTGCAAATACGTCGAGTCT
TACTTCTTCTTGACGTCCTCCTTCTCCAGCCCGATCGCGGTCATGGCGCGTACG
AAGGTACAGGGCTGCAACGACCGTATCTTCGGCAGCCAGCTGTGCACGAATCA
GGTCCCGTTCGCGCTGGCAATCATGTACGTGATGGACCTGGTACTGTTCTTCCT
GGACACGTACCTGTGGTACATCATCTGGCTGGTGATCTTCTCGATGGTGCGCG
CGTTCAAGCTTGGTATCTCGATCTGGACGCCCTGGAGCGAGATCTTCACCCGCA
TGCCGAAGCGTATCTACGCGAAGCTGCTGGCGACGGCCGAGATGGAGGTCAA
GTATAAGCCCAAGgtatgctgaatgcaatctggtcaggtgaattcaccctcatattgttgtgcagGTGCTCG
TCTCGCAAATCTGGAACGCGGTCATCATCTCCATGTACCGGGAGCATCTCTTGT
CCATCGAGCACGTCCAGCGCCTGCTATACCACCAGGTTGATGGTCCAGACGGT
CGCCGCACCCTCAGGGCACCGCCGTTCTTCACCAGCCAGCGAACTGCGAAGCC
AGGCCTGTTCTTCCCTCCTGGTGGCGAGGCTGAGCGCCGTATCTCGTTCTTTGC
CTCATCGCTGACGACCGCGCTCCCTGAGCCTCTGCCGATCGACGCCATGCCCA
CCTTCACCGTGCTCGTTCCCCATTACTCGGAGAAGATTCTGCTCAGTCTGCGCG
 
AGATTATTCGCGAGGAGGACCAGAACACCCGCGTCACCTTGCTGGAGTACCTC
AAGCAGCTCCACCCTGTCGAATGGGACAACTTCGTCAAGGACACCAAGATCTTG
GCGGAAGAGTCGGGCGACGTCCAGGACGAGAAGCGCGCGCACGGACGACT
TGCCGTTCTACTGCATCGGGTTCAAGACCTCGTCACCAGAGTACACCCTGCGTA
CGCGTATCTGGGCTTCACTGCGCGCACAGACGCTGTACCGCACGGTCTCCGGT
ATGATGAACTACTCCAAGGCGATCAAGCTCCTCTATCGCGTCGAGAACCCGGAT
GTCGTTCATGCCTTCGGTGGAACACGGAACGTCTTGAACGCGAGCTTGAGCG
CATGTCTCGCCGCAAGTTCAAGTTCGTCATCTCGATGCAGCGGTACTCTAAGTT
CAACAAGGAGGAGCAAGAGAACGCCGAATTCCTTCTGCGCGCGTACCCGGATT
TGCAGATCGCGTACCTCGATGAAGAGCCCGGTCCCAGCAAGAGCGACGAGGTT
CGGTTGTTTCGACACTCATCGATGGACACTCCGAGGTGGATGAGAAGACCGG
CCGCCGCAAGCCCAAGTTCCGCATTGAGCTGCCCGGTAACCCCATCCTCGGTG
ACGGGAAGTCGGATAACCAGAACCACGCCATTGTCTTCTACCGCGGCGAGTAC
ATCCAGGTCATCGACGCTAACCAGGACAATTACCTGGAAGAGTGTCTCAAGATC
CGTAACGTCCTGGGCGAGTTTGAGGAATACTCCGTGTCGAGCCAGAGCCCGTA
CGCACAGTGGGGCCACAAGGAGTTCAACAAGTGCCCCGTCGCTATCCTGGGTT
CTCGCGAGTACATCTTCTCGGAGAACATCGGTATCCTCGGTGACATCGCCGCC
GGCAAGGAACAGACGTTCGGTACCATTACGGCGCGTGCGCTTGCGTGGATCGG
CGGCAAGCTGCATTACGGTCACCCGGATTTCCTCAATGCGACGTTCATGACGAC
GCGTGGTGGCGTGTCAAAAGCGCAGAAGGGCTTGCATCTCAACGAGGATATCT
TCGCTGGTATGACCGCCGTGTCCCGCGGAGGGCGCATCAAGCACATGGAGTAC
TACCAGTGCGGCAAAGGTCGTGATCTCGGTTTCGGCACGATCTTGAACTTCCAG
ACGAAGATCGGTACTGGTATGGGCGAGCAGCTCCTCTCGCGCGAGTACTACTA
CCTGGGCACGCAATTGCCTATCGACCGGTTCTTGACGTTCTACTACGCGCACGC
TGGTTTCCACGTCAACAACATCCTGGTCATCTACTCCATCCAGGTCTTCATGGTC
ACCTgtaagtgcaggcgctcatgaccgccgagaacgtagtctgacggatgtgcagTGCTGTACCTGGG
CACATTGAACAAGCAGCTGTTCATCTGCAAGGTCAACTCCAATGGCCAGGTTCT
TAGTGGACAAGCTGGGTGCTACAACCTCATCCCGGTCTTCGAGTGGATTCGCC
GGAGTATCATCTCCATCTTCTTGGTGTTCTTCATCGCCTTCTTGCCTCTATTCTT
GCAAGgtatgttcactttccatgtgtcatccgttagccgctcaccatacgacagAGCTGTGCGAGCGCGG
AACGGAAAGGCGTTGCTGCGTCTCGGAAGCACTTCTTGTCACTGTCGCCCA
TTTTCGAAGTGTTCTCCACCCAGATTTACTCGCAGGCGCTCTTGAACAACATGA
GCTTCGGTGGTGCGCGCTACATCGCCACAGGTCGTGGTTTCGCGACTAGTCGC
ATACCCTTCAACATCCTCTACTCGCGTTTCGCGCCGCCAAGCATCTACATGGGC
ATGCGTAACCTGCTGCTCCTGCTGTACGCGACGATGGCCATTTGGATCCCGCA
CCTGATCTACTTCTGGTTCTCCGTCCTCTCCCTCTGCATCGCGCCATTCATGTTC
AATCCGCATCAATTCTCGTACGCCGACTTCATCATCGACTACCGGGAGTTCTTG
CGCTGGATGTCGCGCGGTAACTCGCGAACGAAGGCGAGCAGCTGGTACGGAT
ACTGCCGTCTGTCGCGTACCGCGATTACTGGGTACAAGAAGAAGCTGGGA
CACCCGTCGGAGAAGCTGTCGGGCGACGTACCGCGTGCGCCGTGGAGGAACG
TTATCTTCTCGGAGATCCTGTGGCCCATCGGCGCGTGCATCATCTTCATCGTCG
CGTACATGTTCGTCAAGTCGTTCCCCGACGAGCAGGGCAACGCGCCGCCGAGC
CCGCTGGTCCGGATTCTGCTCATCGCGGTTGGCCCTACTGTGTGGAACGCGGC
GGTGCTCATAACGCTGTTCTTCCTGTCGCTCTTCCTGGGCCCGATGATGGATGG
CTGGGTCAAGTTCGGCTCGGTCATGGCGGCCCTTGCGCATGGCCTGGCGCTTA
TAGGCATGCTCACGTTCTTTGAGTTCTTCgtacgtccttcgcgttgtgtcgtcaagtgctctgctaacg
ccgtcttcagTGGTTCCTTGAGCTCTGGGATGCCTCGCACGCCGTGCTCGGCGTCAT
CGCTATCATTGCCGTTCAGCGCGGATCCAGAAGATCCTCATTGCCGTCTTCCT
GACGCGTGAGTACAAGCACGACGAGACGAACCGCGCGTGGTGGACAGGTAAAT
GGTATGGACGCGGGCTGGGTACCTCGGCCATGTCCCAGCCGGCGCGCGAGTT
 
CATCGTGAAGATCGTGGAGATGTCGTTGTGGACGTCGGACTTCCTGCTTGCGC
ACCTGTTGCTCATCATCTTGACGGTGCCGCTACTGCTGCCGTTCTTCAACTCAAT
TCATTCGACGATGCTTTgtgagtggtttgtagtcgttggtcatggatgatttctgactcgcgtgcagTCTGG
TTGCGCCCTTCGAAGCAGATTAGGCAACCTCTGTTCTCCACCAAGCAGAAGCGG
CAACGGCGATGGATTgtgagttcctttgattgctctgggtaccgaccttcgctcacctttcttagGTCATGAA
GTATACCGTGGTATATCTCGTGGTGGTGGCTTTCCTCGTCGCGCTCATCGCTCT
GCgtacgttttccctcgcgctcaccctgtattttcactaacgtttcctccagCCGCCCTCTTCCGCGAGAGC
ATCCACTTCAACTGCGAGATCTGCCAGAGTATATAGTCATATAACGACGTCTATC
GTATCGCCGGACGAGAGCCCCGTCGCCTACACACTGACATGGAATCGCTGTGT
ATACAATCGATCTTCTGACCGCGTCGGGGGCGTTGCCGTCTTTCTACTATCAAT
TTGCTTGTGTATCAACATTTCTTCTCTCCAAGCCTACATTGACATAGAGTAATAG
CCCATGTTCATACAACAATCGCATAGCATTGCATATACCAT
**SEQ ID NO: 10**
   translation of SEQ ID NO: 13
   amino acid
   *S. commune* 
MSGPGYGRNPFDNPPPNRGPYGQQPGFPGPGPRPYDSDADMSQTYGSTTRLAG
SAGYSDRNGSFDGDRSYAPSIDSRASVPSISPFADPGIGSNEPYPAWSVERQIPMS
TEEIEDIFLDLTQKFGFQRDSMRNTFDFMMHLLDSRASRMTPNQALLTLHADYIGGQ
HANYRKWYFAAQLNLDDAVGQTNNPGIQRLKTIKGATKTKSLDSALNRWRNAMNN
MSQYDRLRQIALYLLCWGEAGNIRLAPECLCFIFKCADDYYRSPECQNRMDPVPEG
LYLQTVIKPLYRFLRDQAYEVVDGKQVKREKDHDQIIGYDDVNQLFWYPEGLAKIVM
SDNTRLVDVPPAQRFMKFAKIEWNRVFFKTYFEKRSTAHLLVNFNRIWILHVSMYFF
YTAFNSPRVYAPHGKLDPSPEMTWSATALGGAVSTMIMILATIAEYTYIPTTWNNAS
HLTTRLIFLLVILALTAGPTFYIAMIDGRTDIGQVPLIVAIVQFFISVVATLAFATIPSGRM
FGDRVAGKSRKHMASQTFTASYPSMKRSSRVASIMLWLLVFGCKYVESYFFLTSSF
SSPIAVMARTKVQGCNDRIFGSQLCTNQVPFALAIMYVMDLVLFFLDTYLWYIIWLVI
FSMVRAFKLGISIWTPWSEIFTRMPKRIYAKLLATAEMEVKYKPKVLVSQIWNAVIISM
YREHLLSIEHVQRLLYHQVDGPDGRRTLRAPPFFTSQRTAKPGLFFPPGGEAERRIS
FFASSLTTALPEPLPIDAMPTFTVLVPHYSEKILLSLREIIREEDQNTRVTLLEYLKQLH
PVEWDNFVKDTKILAEESGDVQDEKRARTDDLPFYCIGFKTSSPEYTLRTRIWASLR
AQTLYRTVSGMMNYSKAIKLLYRVENPDVVHAFGGNTERLERELERMSRRKFKFVI
SMQRYSKFNKEEQENAEFLLRAYPDLQIAYLDEEPGPSKSDEVRLFSTLIDGHSEVD
EKTGRRKPKFRIELPGNPILGDGKSDNQNHAIVFYRGEYIQVIDANQDNYLEECLKIR
NVLGEFEEYSVSSQSPYAQWGHKEFNKCPVAILGSREYIFSENIGILGDIAAGKEQTF
GTITARALAWIGGKLHYGHPDFLNATFMTTRGGVSKAQKGLHLNEDIFAGMTAVSR
GGRIKHMEYYQCGKGRDLGFGTILNFQTKIGTGMGEQLLSREYYYLGTQLPIDRFLT
FYYAHAGFHVNNILVIYSIQVFMVTLLYLGTLNKQLFICKVNSNGQVLSGQAGCYNLI
PVFEWIRRSIISIFLVFFIAFLPLFLQELCERGTGKALLRLGKHFLSLSPIFEVFSTQIYS
QALLNNMSFGGARYIATGRGFATSRIPFNILYSRFAPPSIYMGMRNLLLLLYATMAIW
IPHLIYFWFSVLSLCIAPFMFNPHQFSYADFIIDYREFLRWMSRGNSRTKASSWYGY
CRLSRTAITGYKKKKLGHPSEKLSGDVPRAPWRNVIFSEILWPIGACIIFIVAYMFVKS
FPDEQGNAPPSPLVRILLIAVGPTVWNAAVLITLFFLSLFLGPMMDGWVKFGSVMAA
LAHGLALIGMLTFFEFFWFLELWDASHAVLGVIAIIAVQRGIQKILIAVFLTRKWYGRG
LGTSAMSQPAREFIVKIVEMSLWTSDFLLAHLLLIILTVPLLLPFFNSIHSTMLFWLRPS
KQIRQPLFSTKQKRQRRWIVMKYTVVYLVVVAFLVALIALPALFRESIHFNCEICQSI
**SEQ ID NO: 11**
   Gene sequence 1,3-β-D-glucan synthase II of *S. commune* strain Lu15634
   DNA
   *S. commune* 
CTGTCCAAGGAGGAGATCGAGGACATCTTCCTCGATTTGACGCAGAAGTTTGGC
TTTCAGCGGGATTCCATGCGGAATATGgtacgtggcgtgtgcccatgtgcggcgttctgaggcctaa
cgttttccgccagTTCGACTTCACCATGCAGCTGCTTGACAGCCGAGCGTCTCGTATG
ACCCCCAACCAGGCGCTCCTCACCCTCCACGCCGACTACATTGGTGGCCAGCA
TGCGAACTACCGGAAGTGGTACTTCGCGGCGCAGCTCGACCTTGACGACGCCG
TGGGACAAACTCAGAATCCGGGTCTCAACCGCCTCAAGTCCACTCGCGGATCG
GGCAAGCGACCACGCCATGAAAAGTCGCTGAACACGGCATTGGAGCGCTGGC
GGCAAGCCATGAACAACATGTCGCAGTATGACCGCTTACGCCAGATCGCGCTC
TACCTGCTCTGCTGGGGCGAAGCGGCGCAAGTGCGATTCATGCCCGAGTGCTT
GTGCTTCATCTTCAAGTGCGCCGACGACTACTATCGTTCGCCGGAGTGCCAGAA
CAGGATGGAGCCGGTACCGGAGGGTCTCTACCTGAGGACGGTCGTAAAGCCG
CTCTACAGATTTGTCCGGGATCAAGGCTATGAGGTGGTGGAGGGAAAATTCGTA
CGGCGGGAACGGGATCACGACCAAATCATTGGTTACGATGACGTGAATCAGCT
GTTCTGGTACCCGGAGGGAATTGCCCGTATCGTCCTGTCGGACAAGgtaagcacctc
tgtgcatcttctgtgacatacagggctaattgtcgagcagAGTCGTCTAGTCGACCTCCCCCAGCA
CAGCGCTTCATGAAGTTCGACCGTATCGAGTGGAATCGCGTCTTCTTCAAGACG
TTTTACGAGACTCGATCCTTCACGCATCTTTTGGTCGACTTCAACCGTATCTGGG
TCGTGCACATCGCTCTCTACTTCTTCTACACTGCATACAACTCCCCCACGATCTA
CGCCATCAACGGCAACACACCGACGTCTCTGGCTTGGAGCGCGACTGCGCTCG
GCGGTGCGGTAGCGACAGGTATCATGATCCTCGCCACGATCGCCGAGTTCTCG
CACATCCCCACGACATGGAACAACACCTCGCATCTGACTCGCCGCCTCGCCTTC
CTCCTCGTCACGCTCGGCCTCACATGTGGTCCGACGTTCTACGTCGCGATTGCA
GAGAGCAACGGGAGCGGCGGCTCTTTGGCCTTGATTCTCGGTATCGTCCAGTT
CTTCATCTCCGTCGTGGCAACTGCGCTCTTCACTATCATGCCTTCTGGTCGTAT
GTTCGGCGACCGTGTCGCAGGCAAGAGTCGCAAGTATCTCGCCAGCCAGACGT
TCACGGCCAGCTACCCGTCGTTGCCCAAGCACCAGCGGTTCGCCTCACTCCTG
ATGTGGTTCCTCATCTTCGGGTGCAAGTTGACGGAGAGTTACTTCTTTCTGACG
CTGTCCTTCCGCGACCCTATCCGCGTCATGGTCGGCATGAAGATCCAGAACTG
CGAGGACAAGATTTTCGGCAGCGGCCTTTGCAGGAATCACGCAGCATTCACCC
TCACGATCATGTACATCATGGACCTCGTCTTGTTCTTCCTCGACACCTTCCTTTG
GTATGTCATCTGGAACTCGGTTTTCAGTATCGCACGCTCTTTCGTACTCGGCCTT
TCGATCTGGACACCGTGGAGAGACATCTTCCAGCGTCTGCCGAAGCGGATCTA
CGCGAAGCTTCTGGCGACTGGCGACATGGAGGTCAAGTACAAGCCCAAGgtatgc
gttgagctcgccgtaaatccacttaaggctaacacgttcgcagGTCTTGGTCTCGCAAATCTGGAAC
GCCATCATCATCTCCATGTACCGCGAGCACTTGCTCTCTATTGAGCACGTCCAG
AAGCTCCTGTACCACCAAGTGGACACTGGCGAAGCCGGCAAGCGGAGTCTTCG
CGCGCCTCCGTTCTTCGTCGCGCAGGGCAGCAGCGGTGGCTCGGGCGAGTTC
TTCCCGCCTGGCAGCGAGGCCGAGCGTCGTATCTCTTTCTTCGCGCAGTCGCT
TTCTACGGAGATTCCTCAGCCCATCCCGGTCGACGCCATGCCGACGTTCACGG
TGCTTACGCCTCACTACAGCGAGAAGgtacatgctccccttgtagccatatgacatcagctgactgtc
gtgcacagATCCTTCTCTCTCTCCGTGAAATTATCCGCGAGGAGGACCAGAACACT
CGCGTTACGTTGCTCGAGTACCTGAAGCAGCTGCATCCGGTCGAGTGGGAGAA
TTTCGTCAAGGACACTAAAATTTTGGCCGAGGAGTCCGCTATGTTTAACGGTCC
GAGTCCTTTCGGCAACGACGAGAAGGGTCAGTCCAAGATGGACGATCTACCGT
TCTACTGCATCGGTTTCAAGAGCGCCGCGCCCGAGTACACCCTCCGCACCCGT
 
ATCTGGGCGTCCCTGCGCGCAGACGCTGTACCGCACGGTCTCCGGCATGAT
GAACTATGCGAAGGCGATCAAGCTGCTCTACCGCGTTGAGAACCCGGAGGTCG
TACAACAGTTCGGCGGCAACACGGACAAGCTCGAGCGCGAGTTGGAGCGGATG
GCGCGACGGAAGTTCAAGTTCCTCGTGTCCATGCAGCGCTACTCGAAGTTCAAC
AAGGAGGAGCACGAGAACGCCGAGTTCTTGCTCCGCGCGTACCCGGACTTGCA
GATCGCGTACCTCGAGGAAGAGCCCCCTCGCAAGGAGGGCGGCGATCCACGC
ATCTTCTCTGCCCTCGTCGACGGCCACAGCGACATCATCCCGGAGACCGGCAA
GCGGCGCCCCAAGTTCCGTATCGAGCTGCCCGGTAACCCCATTCTCGGTGACG
GTAAATCCGACAATCAGAACCACGCTATCGTCTTCTACCGCGGCGAGTACCTCC
AGCTTATCGACGCCAACCAGGACAACTACCTCGAGGAGTGCTTGAAGATCCGTA
ACGTGCTCGCCGAGTTTGAGGAGTACGACGTCTCCAGCCAGAGCCCGTACGCG
CAGTGGAGTGTCAAGGAGTTCAAGCGCTCTCCGGTCGCCATCGTCGGTGCACG
CGAGTACATCTTCTCAGAGCACATCGGTATCCTCGGTGATCTGGCGGCTGGCAA
GGAACAGACGTTCGGTACGCTCACGGCACGCAACAACGCCTTCCTTGGCGGCA
AGCTGCACTACGGTCACCCCGATTTCCTCAACGCCCTCTACATGAACACGCGCG
GTGGTGTCTCCAAGGCGCAGAAGGGTCTCCATCTCAACGAGGATATCTACGCC
GGTATGAACGCGGTCGGTCGCGGTGGACGCATTAAGCACAGCGAGTACTATCA
GTGCGGCAAGGGTCGTGACCTCGGTTTCGGCACCATCTTGAACTTCCAGACCA
AGATCGGTACGGGTATGGGCGAGCAGATCCTCTCGCGCGAGTACTACTATCTC
GGAACACAACTGCCCATCGATCGCTTCCTCACGTTCTACTACGCGCACCCGGGT
TTCCAGATCAACAACATGCTGGTCATCCTCTCCGTGCAGGTCTTCATCGTTACCA
gtacgttcaatgcatattgttagcctgacaacgtctgacgaatttccagTGGTCTTCCTCGGTACCTTGAA
GTCTTCGGTCACGATCTGCAAGTACACGTCCAGCGGTCAGTACATCGGTGGTCA
ATCCGGTTGCTACAACCTCGTCCCGGTCTTCCAGTGGATCGAGCGCTGCATCAT
CAGCATCTTCTTGGTGTTCATGATCGCTTTCATGCCGCTCTTCCTGCAAGgtaaga
gcttgtcaacctgctcaaggggcttgcgctgatcatcatctcagAACTCGTCGAGCGCGGTACCTGGA
GTGCCATCTGGCGTCTGCTCAAGCAGTTTATGTCGCTGTCGCCTGTCTTCGAGG
TGTTCTCCACCCAGATTCAGACGCACTCCGTGTTGAGCAACTTGACGTTCGGTG
GTGCGCGTTACATCGCTACCGGTCGTGGGTTCGCCACCAGTCGTATCAGCTTC
AGCATCTTGTTCTCGCGTTTCGCAGGCCCGAGTATCTACCTCGGCATGCGCACG
CTCATTATGCTGCTCTACGTGACGTTGACGATCTGGACGCCATGGGTCATTTAC
TTCTGGGTTTCCATTCTCTCGCTCTGCATCGCGCCGTTCTTGTTCAACCCGCATC
AATTCGTATTCTCGGACTTCCTCATCGACTACAGgtacgtcggacgagcgctgttccgcgacgt
aagctgaccggttatacagGGAATACCTGCGGTGGATGTCGCGTGGCAACTCGCGCTCG
CACAACAACTCCTGGATTGGGTACTGCCGGTTGTCCCGCACGATGATCACTGG
GTACAAGAAGAAGAAGCTGGGCCACCCGTCGGAGAAGCTTTCCGGCGACGTTC
CTCGTGCAGGCTGGCGCGCCGTCTTGTTCTCGGAGATCATCTTCCCGGCGTGC
ATGGCCATCCTCTTCATCATCGCGTACATGTTCGTCAAGTCGTTCCCTCTCGAC
GGCAAGCAGCCTCCCTCCGGCCTCGTTCGCATCGCCGTCGTGTCTATCGGCCC
CATCGTGTGGAACGCCGCCATCCTGTTGACGCTCTTCCTTGTGTCGTTGTTCCT
CGGCCCCATGCTCGACCCGGTCTTCCCCCTCTTCGGTTCCGTTATGGCCTTCAT
CGCGCATTTCCTTGGCACAATCGGAATGATTGGGTTCTTCGAGTTCCTGgtatgtgc
ccatacctttcattcgacttcaactatctaacagattcatagTGGTTCCTCGAGTCCTGGGAGGCGTC
GCATGCCGTGCTGGGTCTCATCGCCGTCATCTCCATCCAGCGCGCCATTCACA
AGATCCTTATCGCCGTTTTCCTCAGTCGCGAGTTCAAGCACGACGAGACGAACA
GGGCCTGGTGGACTGGTCGCTGGTATGGCCGTGGCCTCGGCACGCACGCCAT
GTCGCAGCCGGCGCGTGAGTTCGTCGTCAAGATCATCGAGTTGTCGCTTTGGA
GCTCGGATCTCATACTCGGCCACATCCTGCTGTTCATGCTTACTCCGGCCGTCC
TCATCCCGTACTTCGACCGTTTGCACGCCATGATGCTCTgtacgtcgtgtctcattgtctgtgtt
ggtcatactcttaccctctcttagTCTGGCTGCGTCCCTCGAAGCAAATCCGCGCGCCTCTG
 
TACTCGATCAAGCAGAAGAGGCAAAGACGCTGGATTgtcagtgttcagtgccttattctatcag
ctcttactaacgtcttcatagATCATGAAGTACGGTACTGTATACGTTACCGTCATCGCGAT
CTTCGTCGCGCTCATCGCGCTTCgtgagtttccttgctatttttcgtacctgagcgtcgctgacccctttccc
agCCCTCGTATTCCGACACACTCTAAAGGTCGAGTGCTCCCTTTGCGACAGCTT
GTAATATCGGACTCGTATATATCTAGACTTCTCCGCACCATGTGTAGCTGACGCT
TGGGTATACTTCGCGGTGCCGAGCTAATTGTCGACGGACATTCTCCATCGTTGA
GTGCAGCGACGTCGGGTGGTTTACGACACGGACACTTTTCATTGTACCCTCTAC
GAATGCAAGAACTCTCTTACGACCAGTACCTATGTGCTAAGCCGTCGCCTGTTC
AGGATCATACATACATACGTTTCTAGATACCTTACAGTTAGGCCTATTCAGGGAG
AGTCTGCATAAAA
**SEQ ID NO: 12**
   translation of SEQ ID NO: 15
   amino acid
   *S. commune* 
MPRPGGTSAEGGYASSPSMETTPSDPFGTANGAPRRYYDNDSEEYGPGRRDTYA
SDSSNQGLTDPGYYDQNGAYDPYPTGDTDSDGDVYGQRYGPSAESLGTHKFGHS
DSSTPTFVDYSASSGGRDSYPAWTAERNIPLSKEEIEDIFLDLTQKFGFQRDSMRN
MFDFTMQLLDSRASRMTPNQALLTLHADYIGGQHANYRKWYFAAQLDLDDAVGQT
QNPGLNRLKSTRGSGKRPRHEKSLNTALERWRQAMNNMSQYDRLRQIALYLLCW
GEAAQVRFMPECLCFIFKCADDYYRSPECQNRMEPVPEGLYLRTVVKPLYRFVRD
QGYEVVEGKFVRRERDHDQIIGYDDVNQLFWYPEGIARIVLSDKSRLVDLPPAQRF
MKFDRIEWNRVFFKTFYETRSFTHLLVDFNRIWVVHIALYFFYTAYNSPTIYAINGNT
PTSLAWSATALGGAVATGIMILATIAEFSHIPTTWNNTSHLTRRLAFLLVTLGLTCGPT
FYVAIAESNGSGGSLALILGIVQFFISVVATALFTIMPSGRMFGDRVAGKSRKYLASQ
TFTASYPSLPKHQRFASLLMWFLIFGCKLTESYFFLTLSFRDPIRVMVGMKIQNCED
KIFGSGLCRNHAAFTLTIMYIMDLVLFFLDTFLWYVIWNSVFSIARSFVLGLSIWTPWR
DIFQRLPKRIYAKLLATGDMEVKYKPKVLVSQIWNAIIISMYREHLLSIEHVQKLLYHQ
VDTGEAGKRSLRAPPFFVAQGSSGGSGEFFPPGSEAERRISFFAQSLSTEIPQPIPV
DAMPTFTVLTPHYSEKILLSLREIIREEDQNTRVTLLEYLKQLHPVEWENFVKDTKILA
EESAMFNGPSPFGNDEKGQSKMDDLPFYCIGFKSAAPEYTLRTRIWASLRAQTLYR
TVSGMMNYAKAIKLLYRVENPEVVQQFGGNTDKLERELERMARRKFKFLVSMQRY
SKFNKEEHENAEFLLRAYPDLQIAYLEEEPPRKEGGDPRIFSALVDGHSDIIPETGKR
RPKFRIELPGNPILGDGKSDNQNHAIVFYRGEYLQLIDANQDNYLEECLKIRNVLAEF
EEYDVSSQSPYAQWSVKEFKRSPVAIVGAREYIFSEHIGILGDLAAGKEQTFGTLTA
RNNAFLGGKLHYGHPDFLNALYMNTRGGVSKAQKGLHLNEDIYAGMNAVGRGGRI
KHSEYYQCGKGRDLGFGTILNFQTKIGTGMGEQILSREYYYLGTQLPIDRFLTFYYA
HPGFQINNMLVILSVQVFIVTMVFLGTLKSSVTICKYTSSGQYIGGQSGCYNLVPVFQ
WIERCIISIFLVFMIAFMPLFLQELVERGTWSAIWRLLKQFMSLSPVFEVFSTQIQTHS
VLSNLTFGGARYIATGRGFATSRISFSILFSRFAGPSIYLGMRTLIMLLYVTLTIWTPW
VIYFWVSILSLCIAPFLFNPHQFVFSDFLIDYREYLRWMSRGNSRSHNNSWIGYCRL
SRTMITGYKKKKLGHPSEKLSGDVPRAGWRAVLFSEIIFPACMAILFIIAYMFVKSFPL
DGKQPPSGLVRIAVVSIGPIVWNAAILLTLFLVSLFLGPMLDPVFPLFGSVMAFIAHFL
GTIGMIGFFEFLWFLESWEASHAVLGLIAVISIQRAIHKILIAVFLSREFKHDETNRAW
WTGRWYGRGLGTHAMSQPAREFVVKIIELSLWSSDLILGHILLFMLTPAVLIPYFDRL
HAMMLFWLRPSKQIRAPLYSIKQKRQRRWIIMKYGTVYVTVIAIFVALIALPLVFRHTL
KVECSLCDSL
**SEQ ID NO: 13**
   cDNA 1,3-β-D-glucan synthase I of *S. commune* strain Lu15634
   DNA
   *S. commune* 
ATGTCCGGTCCAGGATATGGCAGGAATCCATTCGACAATCCCCCGCCCAACAG
AGGTCCCTATGGCCAGCAGCCAGGTTTCCCGGGCCCGGCCCTCGGCCTTAC
GACTCGGACGCGGACATGAGCCAGACCTATGGCAGCACAACCAGGCTCGCCG
GCAGTGCCGGTTACAGCGACAGAAACGGCAGCTTCGACGGCGACCGCTCCTAC
GCGCCCTCAATTGACTCGCGCGCCAGCGTGCCCAGCATATCGCCCTTCGCAGA
CCCGGGTATCGGCTCTAATGAGCCGTATCCCGCTTGGTCGGTCGAACGCCAGA
TCCCCATGTCCACGGAGGAGATTGAGGATATCTTCCTCGACCTCACCCAAAAGT
TTGGCTTCCAGCGCGACTCCATGCGGAATACGTTCGACTTCATGATGCACCTCC
TTGATTCCCGTGCCTCGCGCATGACGCCCAACCAAGCTCTGCTCACGCTTCACG
CCGACTACATTGGTGGCCAGCACGCCAACTATAGGAAGTGGTATTTCGCCGCTC
AGCTCAACCTCGATGACGCGGTCGGCAAACCAATAACCCCGGTATCCAGCGC
TTGAAGACCATCAAGGGCGCTACGAAGACCAAGTCGCTCGACAGCGCACTCAA
CCGCTGGCGCAATGCGATGAACAACATGAGCCAGTACGATCGCCTCCGGCAAA
TTGCGCTCTATCTCCTCTGCTGGGGAGAAGCAGGCAACATCCGTCTGGCGCCC
GAGTGCTTGTGCTTCATCTTCAAGTGCGCGGACGACTACTACAGAAGTCCCGAG
TGTCAGAACCGGATGGACCCCGTGCCGGAAGGGCTGTACCTCCAGACGGTCAT
CAAGCCGCTCTATCGCTTCCTACGTGATCAGGCGTACGAAGTCGTTGATGGGAA
GCAAGTGAAGCGCGAGAAGGACCACGACCAGATTATCGGTTATGACGACGTCA
ACCAGTTATTCTGGTATCCGGAAGGTTTGGCTAAGATCGTCATGTCGGACAACA
CACGACTTGTAGATGTACCTCCGGCGCAGCGGTTCATGAAGTTCGCCAAGATC
GAGTGGAACCGCGTCTTCTTCAAGACGTACTTTGAGAAGCGCTCTACTGCCCAT
CTCCTGGTCAACTTCAACCGTATATGGATCCTCCACGTCTCGATGTACTTCTTCT
ACACGGCATTCAACTCTCCACGAGTCTACGCGCCGCACGGCAAACTCGACCCC
TCCCCTGAGATGACCTGGTCCGCGACTGCCCTTGGAGGCGCTGTGTCCACCAT
GATCATGATCCTTGCCACTATCGCGGAGTACACCTACATCCCCACGACATGGAA
CAATGCGTCGCACCTCACCACGCGGCTCATTTTCCTCCTGGTCATCCTCGCGCT
CACTGCTGGACCAACATTCTATATCGCCATGATAGACGGACGCACGGACATCGG
CCAAGTACCACTCATCGTGGCCATAGTGCAGTTCTTCATCTCCGTCGTCGCCAC
CCTCGCTTTCGCTACCATCCCTTCTGGTCGCATGTTCGGCGACCGTGTGGCTG
GCAAGTCAAGAAAGCACATGGCATCGCAGACGTTCACAGCGTCGTACCCGTCC
ATGAAGCGGTCATCTCGCGTAGCGAGTATCATGCTGTGGCTTTTGGTCTTTGGC
TGCAAATACGTCGAGTCTTACTTCTTCTTGACGTCCTCCTTCTCCAGCCCGATCG
CGGTCATGGCGCGTACGAAGGTACAGGGCTGCAACGACCGTATCTTCGGCAGC
CAGCTGTGCACGAATCAGGTCCCGTTCGCGCTGGCAATCATGTACGTGATGGA
CCTGGTACTGTTCTTCCTGGACACGTACCTGTGGTACATCATCTGGCTGGTGAT
CTTCTCGATGGTGCGCGCGTTCAAGCTTGGTATCTCGATCTGGACGCCCTGGA
GCGAGATCTTCACCCGCATGCCGAAGCGTATCTACGCGAAGCTGCTGGCGACG
GCCGAGATGGAGGTCAAGTATAAGCCCAAGGTGCTCGTCTCGCAAATCTGGAA
CGCGGTCATCATCTCCATGTACCGGGAGCATCTCTTGTCCATCGAGCACGTCCA
GCGCCTGCTATACCACCAGGTTGATGGTCCAGACGGTCGCCGCACCCTCAGGG
CACCGCCGTTCTTCACCAGCCAGCGAACTGCGAAGCCAGGCCTGTTCTTCCCT
CCTGGTGGCGAGGCTGAGCGCCGTATCTCGTTCTTTGCCTCATCGCTGACGAC
CGCGCTCCCTGAGCCTCTGCCGATCGACGCCATGCCCACCTTCACCGTGCTCG
TTCCCCATTACTCGGAGAAGATTCTGCTCAGTCTGCGCGAGATTATTCGCGAGG
AGGACCAGAACACCCGCGTCACCTTGCTGGAGTACCTCAAGCAGCTCCACCCT
GTCGAATGGGACAACTTCGTCAAGGACACCAAGATCTTGGCGGAAGAGTCGGG
 
CGACGTCCAGGACGAGAAGCGCGCGCACGGACGACTTGCCGTTCTACTGC
ATCGGGTTCAAGACCTCGTCACCAGAGTACACCCTGCGTACGCGTATCTGGGC
TTCACTGCGCGCACAGACGCTGTACCGCACGGTCTCCGGTATGATGAACTACTC
CAAGGCGATCAAGCTCCTCTATCGCGTCGAGAACCCGGATGTCGTTCATGCCTT
CGGTGGGAACACGGAACGTCTTGAACGCGAGCTTGAGCGCATGTCTCGCCGCA
AGTTCAAGTTCGTCATCTCGATGCAGCGGTACTCTAAGTTCAACAAGGAGGAGC
AAGAGAACGCCGAATTCCTTCTGCGCGCGTACCCGGATTTGCAGATCGCGTAC
CTCGATGAAGAGCCCGGTCCCAGCAAGAGCGACGAGGTTCGGTTGTTTTCGAC
ACTCATCGATGGACACTCCGAGGTGGATGAGAAGACCGGCCGCCGCAAGCCCA
AGTTCCGCATTGAGCTGCCCGGTAACCCCATCCTCGGTGACGGAAGTCGGAT
AACCAGAACCACGCCATTGTCTTCTACCGCGGCGAGTACATCCAGGTCATCGAC
GCTAACCAGGACAATTACCTGGAAGAGTGTCTCAAGATCCGTAACGTCCTGGGC
GAGTTTGAGGAATACTCCGTGTCGAGCCAGAGCCCGTACGCACAGTGGGGCCA
CAAGGAGTTCAACAAGTGCCCCGTCGCTATCCTGGGTTCTCGCGAGTACATCTT
CTCGGAGAACATCGGTATCCTCGGTGACATCGCCGCCGGCAAGGAACAGACGT
TCGGTACCATTACGGCGCGTGCGCTTGCGTGGATCGGCGGCAAGCTGCATTAC
GGTCACCCGGATTTCCTCAATGCGACGTTCATGACGACGCGTGGTGGCGTGTC
AAAAGCGCAGAAGGGCTTGCATCTCAACGAGGATATCTTCGCTGGTATGACCGC
CGTGTCCCGCGGAGGGCGCATCAAGCACATGGAGTACTACCAGTGCGGCAAAG
GTCGTGATCTCGGTTTCGGCACGATCTTGAACTTCCAGACGAAGATCGGTACTG
GTATGGGCGAGCAGCTCCTCTCGCGCGAGTACTACTACCTGGGCACGCAATTG
CCTATCGACCGGTTCTTGACGTTCTACTACGCGCACGCTGGTTTCCACGTCAAC
AACATCCTGGTCATCTACTCCATCCAGGTCTTCATGGTCACCTTGCTGTACCTG
GGCACATTGAACAAGCAGCTGTTCATCTGCAAGGTCAACTCCAATGGCCAGGTT
CTTAGTGGACAAGCTGGGTGCTACAACCTCATCCCGGTCTTCGAGTGGATTCGC
CGGAGTATCATCTCCATCTTCTTGGTGTTCTTCATCGCCTTCTTGCCTCTATTCTT
GCAAGAGCTGTGCGAGCGCGGAACGGGAAAGGCGTTGCTGCGTCTCGGGAAG
CACTTCTTGTCACTGTCGCCCATTTTCGAAGTGTTCTCCACCCAGATTTACTCGC
AGGCGCTCTTGAACAACATGAGCTTCGGTGGTGCGCGCTACATCGCCACAGGT
CGTGGTTTCGCGACTAGTCGCATACCCTTCAACATCCTCTACTCGCGTTTCGCG
CCGCCAAGCATCTACATGGGCATGCGTAACCTGCTGCTCCTGCTGTACGCGAC
GATGGCCATTTGGATCCCGCACCTGATCTACTTCTGGTTCTCCGTCCTCTCCCT
CTGCATCGCGCCATTCATGTTCAATCCGCATCAATTCTCGTACGCCGACTTCATC
ATCGACTACCGGGAGTTCTTGCGCTGGATGTCGCGCGGTAACTCGCGAACGAA
GGCGAGCAGCTGGTACGGATACTGCCGTCTGTCGCGTACCGCGATTACTGGGT
ACAAGAAGAAGAAGCTGGGACACCCGTCGGAGAAGCTGTCGGGCGACGTACC
GCGTGCGCCGTGGAGGAACGTTATCTTCTCGGAGATCCTGTGGCCCATCGGCG
CGTGCATCATCTTCATCGTCGCGTACATGTTCGTCAAGTCGTTCCCCGACGAGC
AGGGCAACGCGCCGCCGAGCCCGCTGGTCCGGATTCTGCTCATCGCGGTTGG
CCCTACTGTGTGGAACGCGGCGGTGCTCATAACGCTGTTCTTCCTGTCGCTCTT
CCTGGGCCCGATGATGGATGGCTGGGTCAAGTTCGGCTCGGTCATGGCGGCC
CTTGCGCATGGCCTGGCGCTTATAGGCATGCTCACGTTCTTTGAGTTCTTCTGG
TTCCTTGAGCTCTGGGATGCCTCGCACGCCGTGCTCGGCGTCATCGCTATCATT
GCCGTTCAGCGCGGGATCCAGAAGATCCTCATTGCCGTCTTCCTGACGCGTGA
GTACAAGCACGACGAGACGAACCGCGCGTGGTGGACAGGTAAATGGTATGGAC
GCGGGCTGGGTACCTCGGCCATGTCCCAGCCGGCGCGCGAGTTCATCGTGAA
GATCGTGGAGATGTCGTTGTGGACGTCGGACTTCCTGCTTGCGCACCTGTTGCT
CATCATCTTGACGGTGCCGCTACTGCTGCCGTTCTTCAACTCAATTCATTCGAC
GATGCTTTTCTGGTTGCGCCCTTCGAAGCAGATTAGGCAACCTCTGTTCTCCAC
CAAGCAGAAGCGGCAACGGCGATGGATTGTCATGAAGTATACCGTGGTATATCT
 
CGTGGTGGTGGCTTTCCTCGTCGCGCTCATCGCTCTGCCCGCCCTCTTCCGCG
AGAGCATCCACTTCAACTGCGAGATCTGCCAGAGTATATAG
**SEQ ID NO:** 14
   polypeptide sequence 1,3-β-D-glucan synthase I of *S. commune* strain Lu15634
   amino acid
   *S. commune* 
MSGPGYGRNPFDNPPPNRGPYGQQPGFPGPGPRPYDSDADMSQTYGSTTRLAG
SAGYSDRNGSFDGDRSYAPSIDSRASVPSISPFADPGIGSNEPYPAWSVERQIPMS
TEEIEDIFLDLTQKFGFQRDSMRNTFDFMMHLLDSRASRMTPNQALLTLHADYIGGQ
HANYRKWYFAAQLNLDDAVGQTNNPGIQRLKTIKGATKTKSLDSALNRWRNAMNN
MSQYDRLRQIALYLLCWGEAGNIRLAPECLCFIFKCADDYYRSPECQNRMDPVPEG
LYLQTVIKPLYRFLRDQAYEVVDGKQVKREKDHDQIIGYDDVNQLFWYPEGLAKIVM
SDNTRLVDVPPAQRFMKFAKIEWNRVFFKTYFEKRSTAHLLVNFNRIWILHVSMYFF
YTAFNSPRVYAPHGKLDPSPEMTWSATALGGAVSTMIMILATIAEYTYIPTTWNNAS
HLTTRLIFLLVILALTAGPTFYIAMIDGRTDIGQVPLIVAIVQFFISVVATLAFATIPSGRM
FGDRVAGKSRKHMASQTFTASYPSMKRSSRVASIMLWLLVFGCKYVESYFFLTSSF
SSPIAVMARTKVQGCNDRIFGSQLCTNQVPFALAIMYVMDLVLFFLDTYLWYIIWLVI
FSMVRAFKLGISIWTPWSEIFTRMPKRIYAKLLATAEMEVKYKPKVLVSQIWNAVIISM
YREHLLSIEHVQRLLYHQVDGPDGRRTLRAPPFFTSQRTAKPGLFFPPGGEAERRIS
FFASSLTTALPEPLPIDAMPTFTVLVPHYSEKILLSLREIIREEDQNTRVTLLEYLKQLH
PVEWDNFVKDTKILAEESGDVQDEKRARTDDLPFYCIGFKTSSPEYTLRTRIWASLR
AQTLYRTVSGMMNYSKAIKLLYRVENPDVVHAFGGNTERLERELERMSRRKFKFVI
SMQRYSKFNKEEQENAEFLLRAYPDLQIAYLDEEPGPSKSDEVRLFSTLIDGHSEVD
EKTGRRKPKFRIELPGNPILGDGKSDNQNHAIVFYRGEYIQVIDANQDNYLEECLKIR
NVLGEFEEYSVSSQSPYAQWGHKEFNKCPVAILGSREYIFSENIGILGDIAAGKEQTF
GTITARALAWIGGKLHYGHPDFLNATFMTTRGGVSKAQKGLHLNEDIFAGMTAVSR
GGRIKHMEYYQCGKGRDLGFGTILNFQTKIGTGMGEQLLSREYYYLGTQLPIDRFLT
FYYAHAGFHVNNILVIYSIQVFMVTLLYLGTLNKQLFICKVNSNGQVLSGQAGCYNLI
PVFEWIRRSIISIFLVFFIAFLPLFLQELCERGTGKALLRLGKHFLSLSPIFEVFSTQIYS
QALLNNMSFGGARYIATGRGFATSRIPFNILYSRFAPPSIYMGMRNLLLLLYATMAIW
IPHLIYFWFSVLSLCIAPFMFNPHQFSYADFIIDYREFLRWMSRGNSRTKASSWYGY
CRLSRTAITGYKKKKLGHPSEKLSGDVPRAPWRNVIFSEILWPIGACIIFIVAYMFVKS
FPDEQGNAPPSPLVRILLIAVGPTVWNAAVLITLFFLSLFLGPMMDGWVKFGSVMAA
LAHGLALIGMLTFFEFFWFLELWDASHAVLGVIAIIAVQRGIQKILIAVFLTREYKHDET
NRAWWTGKWYGRGLGTSAMSQPAREFIVKIVEMSLWTSDFLLAHLLLIILTVPLLLP
FFNSIHSTMLFWLRPSKQIRQPLFSTKQKRQRRWIVMKYTVVYLVVVAFLVALIALPA
LFRESIHFNCEICQSI
**SEQ ID NO: 15**
   cDNA 1,3-β-D-glucan synthase II of *S. commune* strain Lu15634
   DNA
   *S*. *commune* 
ATGCCGAGGCCGGGCGGCACCAGCGCAGAAGGCGGCTACGCATCATCGCCGT
CGATGGAGACGACCCCCAGCGATCCCTTCGGAACCGCGAACGGCGCGCCCCG
CCGCTACTACGACAATGATTCTGAGGAGTACGGACCTGGCCGTAGAGACACCT
ACGCGTCCGACAGCAGTAATCAGGGCCTCACGGACCCGGGCTACTACGACCAG
AATGGCGCCTATGATCCCTATCCGACCGGGGACACCGATTCCGACGGCGACGT
 
CTACGGCCAGCGATATGGACCCTCAGCAGAGTCGCTTGGCACCCACAAGTTCG
GCCATTCCGATTCATCCACGCCGACTTTTGTCGACTACAGCGCATCCTCCGGCG
GGAGGGATTCGTACCCTGCATGGACTGCCGAACGCAACATCCCGCTGTCCAAG
GAGGAGATCGAGGACATCTTCCTCGATTTGACGCAGAAGTTTGGCTTTCAGCGG
GATTCCATGCGGAATATGTTCGACTTCACCATGCAGCTGCTTGACAGCCGAGCG
TCTCGTATGACCCCCAACCAGGCGCTCCTCACCCTCCACGCCGACTACATTGGT
GGCCAGCATGCGAACTACCGGAAGTGGTACTTCGCGGCGCAGCTCGACCTTGA
CGACGCCGTGGGACAAACTCAGAATCCGGGTCTCAACCGCCTCAAGTCCACTC
GCGGATCGGGCAAGCGACCACGCCATGAAAAGTCGCTGAACACGGCATTGGAG
CGCTGGCGGCAAGCCATGAACAACATGTCGCAGTATGACCGCTTACGCCAGAT
CGCGCTCTACCTGCTCTGCTGGGGCGAAGCGGCGCAAGTGCGATTCATGCCCG
AGTGCTTGTGCTTCATCTTCAAGTGCGCCGACGACTACTATCGTTCGCCGGAGT
GCCAGAACAGGATGGAGCCGGTACCGGAGGGTCTCTACCTGAGGACGGTCGT
AAAGCCGCTCTACAGATTTGTCCGGGATCAAGGCTATGAGGTGGTGGAGGGAA
AATTCGTACGGCGGGAACGGGATCACGACCAAATCATTGGTTACGATGACGTGA
ATCAGCTGTTCTGGTACCCGGAGGGAATTGCCCGTATCGTCCTGTCGGACAAG
AGTCGTCTAGTCGACCTCCCCCCAGCACAGCGCTTCATGAAGTTCGACCGTATC
GAGTGGAATCGCGTCTTCTTCAAGACGTTTTACGAGACTCGATCCTTCACGCAT
CTTTTGGTCGACTTCAACCGTATCTGGGTCGTGCACATCGCTCTCTACTTCTTCT
ACACTGCATACAACTCCCCCACGATCTACGCCATCAACGGCAACACACCGACGT
CTCTGGCTTGGAGCGCGACTGCGCTCGGCGGTGCGGTAGCGACAGGTATCAT
GATCCTCGCCACGATCGCCGAGTTCTCGCACATCCCCACGACATGGAACAACA
CCTCGCATCTGACTCGCCGCCTCGCCTTCCTCCTCGTCACGCTCGGCCTCACAT
GTGGTCCGACGTTCTACGTCGCGATTGCAGAGAGCAACGGGAGCGGCGGCTCT
TTGGCCTTGATTCTCGGTATCGTCCAGTTCTTCATCTCCGTCGTGGCAACTGCG
CTCTTCACTATCATGCCTTCTGGTCGTATGTTCGGCGACCGTGTCGCAGGCAAG
AGTCGCAAGTATCTCGCCAGCCAGACGTTCACGGCCAGCTACCCGTCGTTGCC
CAAGCACCAGCGGTTCGCCTCACTCCTGATGTGGTTCCTCATCTTCGGGTGCAA
GTTGACGGAGAGTTACTTCTTTCTGACGCTGTCCTTCCGCGACCCTATCCGCGT
CATGGTCGGCATGAAGATCCAGAACTGCGAGGACAAGATTTTCGGCAGCGGCC
TTTGCAGGAATCACGCAGCATTCACCCTCACGATCATGTACATCATGGACCTCG
TCTTGTTCTTCCTCGACACCTTCCTTTGGTATGTCATCTGGAACTCGGTTTTCAG
TATCGCACGCTCTTTCGTACTCGGCCTTTCGATCTGGACACCGTGGAGAGACAT
CTTCCAGCGTCTGCCGAAGCGGATCTACGCGAAGCTTCTGGCGACTGGCGACA
TGGAGGTCAAGTACAAGCCCAAGGTCTTGGTCTCGCAAATCTGGAACGCCATCA
TCATCTCCATGTACCGCGAGCACTTGCTCTCTATTGAGCACGTCCAGAAGCTCC
TGTACCACCAAGTGGACACTGGCGAAGCCGGCAAGCGGAGTCTTCGCGCGCCT
CCGTTCTTCGTCGCGCAGGGCAGCAGCGGTGGCTCGGGCGAGTTCTTCCCGC
CTGGCAGCGAGGCCGAGCGTCGTATCTCTTTCTTCGCGCAGTCGCTTTCTACG
GAGATTCCTCAGCCCATCCCGGTCGACGCCATGCCGACGTTCACGGTGCTTAC
GCCTCACTACAGCGAGAAGATCCTTCTCTCTCTCCGTGAAATTATCCGCGAGGA
GGACCAGAACACTCGCGTTACGTTGCTCGAGTACCTGAAGCAGCTGCATCCGG
TCGAGTGGGAGAATTTCGTCAAGGACACTAAAATTTTGGCCGAGGAGTCCGCTA
TGTTTAACGGTCCGAGTCCTTTCGGCAACGACGAGAAGGGTCAGTCCAAGATG
GACGATCTACCGTTCTACTGCATCGGTTTCAAGAGCGCCGCGCCCGAGTACAC
CCTCCGCACCCGTATCTGGGCGTCCCTGCGCGCGCAGACGCTGTACCGCACG
GTCTCCGGCATGATGAACTATGCGAAGGCGATCAAGCTGCTCTACCGCGTTGA
GAACCCGGAGGTCGTACAACAGTTCGGCGGCAACACGGACAAGCTCGAGCGC
GAGTTGGAGCGGATGGCGCGACGGAAGTTCAAGTTCCTCGTGTCCATGCAGCG
CTACTCGAAGTTCAACAAGGAGGAGCACGAGAACGCCGAGTTCTTGCTCCGCG
 
CGTACCCGGACTTGCAGATCGCGTACCTCGAGGAAGAGCCCCCTCGCAAGGAG
GGCGGCGATCCACGCATCTTCTCTGCCCTCGTCGACGGCCACAGCGACATCAT
CCCGGAGACCGGCAAGCGGCGCCCCAAGTTCCGTATCGAGCTGCCCGGTAAC
CCCATTCTCGGTGACGGTAAATCCGACAATCAGAACCACGCTATCGTCTTCTAC
CGCGGCGAGTACCTCCAGCTTATCGACGCCAACCAGGACAACTACCTCGAGGA
GTGCTTGAAGATCCGTAACGTGCTCGCCGAGTTTGAGGAGTACGACGTCTCCA
GCCAGAGCCCGTACGCGCAGTGGAGTGTCAAGGAGTTCAAGCGCTCTCCGGTC
GCCATCGTCGGTGCACGCGAGTACATCTTCTCAGAGCACATCGGTATCCTCGGT
GATCTGGCGGCTGGCAAGGAACAGACGTTCGGTACGCTCACGGCACGCAACAA
CGCCTTCCTTGGCGGCAAGCTGCACTACGGTCACCCCGATTTCCTCAACGCCC
TCTACATGAACACGCGCGGTGGTGTCTCCAAGGCGCAGAAGGGTCTCCATCTC
AACGAGGATATCTACGCCGGTATGAACGCGGTCGGTCGCGGTGGACGCATTAA
GCACAGCGAGTACTATCAGTGCGGCAAGGGTCGTGACCTCGGTTTCGGCACCA
TCTTGAACTTCCAGACCAAGATCGGTACGGGTATGGGCGAGCAGATCCTCTCG
CGCGAGTACTACTATCTCGGAACACAACTGCCCATCGATCGCTTCCTCACGTTC
TACTACGCGCACCCGGGTTTCCAGATCAACAACATGCTGGTCATCCTCTCCGTG
CAGGTCTTCATCGTTACCATGGTCTTCCTCGGTACCTTGAAGTCTTCGGTCACG
ATCTGCAAGTACACGTCCAGCGGTCAGTACATCGGTGGTCAATCCGGTTGCTAC
AACCTCGTCCCGGTCTTCCAGTGGATCGAGCGCTGCATCATCAGCATCTTCTTG
GTGTTCATGATCGCTTTCATGCCGCTCTTCCTGCAAGAACTCGTCGAGCGCGGT
ACCTGGAGTGCCATCTGGCGTCTGCTCAAGCAGTTTATGTCGCTGTCGCCTGTC
TTCGAGGTGTTCTCCACCCAGATTCAGACGCACTCCGTGTTGAGCAACTTGACG
TTCGGTGGTGCGCGTTACATCGCTACCGGTCGTGGGTTCGCCACCAGTCGTAT
CAGCTTCAGCATCTTGTTCTCGCGTTTCGCAGGCCCGAGTATCTACCTCGGCAT
GCGCACGCTCATTATGCTGCTCTACGTGACGTTGACGATCTGGACGCCATGGG
TCATTTACTTCTGGGTTTCCATTCTCTCGCTCTGCATCGCGCCGTTCTTGTTCAA
CCCGCATCAATTCGTATTCTCGGACTTCCTCATCGACTACAGGGAATACCTGCG
GTGGATGTCGCGTGGCAACTCGCGCTCGCACAACAACTCCTGGATTGGGTACT
GCCGGTTGTCCCGCACGATGATCACTGGGTACAAGAAGAAGAAGCTGGGCCAC
CCGTCGGAGAAGCTTTCCGGCGACGTTCCTCGTGCAGGCTGGCGCGCCGTCTT
GTTCTCGGAGATCATCTTCCCGGCGTGCATGGCCATCCTCTTCATCATCGCGTA
CATGTTCGTCAAGTCGTTCCCTCTCGACGGCAAGCAGCCTCCCTCCGGCCTCG
TTCGCATCGCCGTCGTGTCTATCGGCCCCATCGTGTGGAACGCCGCCATCCTG
TTGACGCTCTTCCTTGTGTCGTTGTTCCTCGGCCCCATGCTCGACCCGGTCTTC
CCCCTCTTCGGTTCCGTTATGGCCTTCATCGCGCATTTCCTTGGCACAATCGGA
ATGATTGGGTTCTTCGAGTTCCTGTGGTTCCTCGAGTCCTGGAGGCGTCGCAT
GCCGTGCTGGGTCTCATCGCCGTCATCTCCATCCAGCGCGCCATTCACAAGAT
CCTTATCGCCGTTTTCCTCAGTCGCGAGTTCAAGCACGACGAGACGAACAGGG
CCTGGTGGACTGGTCGCTGGTATGGCCGTGGCCTCGGCACGCACGCCATGTC
GCAGCCGGCGCGTGAGTTCGTCGTCAAGATCATCGAGTTGTCGCTTTGGAGCT
CGGATCTCATACTCGGCCACATCCTGCTGTTCATGCTTACTCCGGCCGTCCTCA
TCCCGTACTTCGACCGTTTGCACGCCATGATGCTCTTCTGGCTGCGTCCCTCGA
AGCAAATCCGCGCGCCTCTGTACTCGATCAAGCAGAAGAGGCAAAGACGCTGG
ATTATCATGAAGTACGGTACTGTATACGTTACCGTCATCGCGATCTTCGTCGCG
CTCATCGCGCTTCCCCTCGTATTCCGACACACTCTAAAGGTCGAGTGCTCCCTT
TGCGACAGCTTGTAA
**SEQ ID NO: 16**
   polypeptide sequence 1,3-β-D-glucan synthase II of *S. commune* strain Lu15634
   amino acid
   *S*. *commune* 
MPRPGGTSAEGGYASSPSMETTPSDPFGTANGAPRRYYDNDSEEYGPGRRDTYA
SDSSNQGLTDPGYYDQNGAYDPYPTGDTDSDGDVYGQRYGPSAESLGTHKFGHS
DSSTPTFVDYSASSGGRDSYPAWTAERNIPLSKEEIEDIFLDLTQKFGFQRDSMRN
MFDFTMQLLDSRASRMTPNQALLTLHADYIGGQHANYRKWYFAAQLDLDDAVGQT
QNPGLNRLKSTRGSGKRPRHEKSLNTALERWRQAMNNMSQYDRLRQIALYLLCW
GEAAQVRFMPECLCFIFKCADDYYRSPECQNRMEPVPEGLYLRTVVKPLYRFVRD
QGYEVVEGKFVRRERDHDQIIGYDDVNQLFWYPEGIARIVLSDKSRLVDLPPAQRF
MKFDRIEWNRVFFKTFYETRSFTHLLVDFNRIWVVHIALYFFYTAYNSPTIYAINGNT
PTSLAWSATALGGAVATGIMILATIAEFSHIPTTWNNTSHLTRRLAFLLVTLGLTCGPT
FYVAIAESNGSGGSLALILGIVQFFISVVATALFTIMPSGRMFGDRVAGKSRKYLASQ
TFTASYPSLPKHQRFASLLMWFLIFGCKLTESYFFLTLSFRDPIRVMVGMKIQNCED
KIFGSGLCRNHAAFTLTIMYIMDLVLFFLDTFLWYVIWNSVFSIARSFVLGLSIWTPWR
DIFQRLPKRIYAKLLATGDMEVKYKPKVLVSQIWNAIIISMYREHLLSIEHVQKLLYHQ
VDTGEAGKRSLRAPPFFVAQGSSGGSGEFFPPGSEAERRISFFAQSLSTEIPQPIPV
DAMPTFTVLTPHYSEKILLSLREIIREEDQNTRVTLLEYLKQLHPVEWENFVKDTKILA
EESAMFNGPSPFGNDEKGQSKMDDLPFYCIGFKSAAPEYTLRTRIWASLRAQTLYR
TVSGMMNYAKAIKLLYRVENPEVVQQFGGNTDKLERELERMARRKFKFLVSMQRY
SKFNKEEHENAEFLLRAYPDLQIAYLEEEPPRKEGGDPRIFSALVDGHSDIIPETGKR
RPKFRIELPGNPILGDGKSDNQNHAIVFYRGEYLQLIDANQDNYLEECLKIRNVLAEF
EEYDVSSQSPYAQWSVKEFKRSPVAIVGAREYIFSEHIGILGDLAAGKEQTFGTLTA
RNNAFLGGKLHYGHPDFLNALYMNTRGGVSKAQKGLHLNEDIYAGMNAVGRGGRI
KHSEYYQCGKGRDLGFGTILNFQTKIGTGMGEQILSREYYYLGTQLPIDRFLTFYYA
HPGFQINNMLVILSVQVFIVTMVFLGTLKSSVTICKYTSSGQYIGGQSGCYNLVPVFQ
WIERCIISIFLVFMIAFMPLFLQELVERGTWSAIWRLLKQFMSLSPVFEVFSTQIQTHS
VLSNLTFGGARYIATGRGFATSRISFSILFSRFAGPSIYLGMRTLIMLLYVTLTIWTPW
VIYFWVSILSLCIAPFLFNPHQFVFSDFLIDYREYLRWMSRGNSRSHNNSWIGYCRL
SRTMITGYKKKKLGHPSEKLSGDVPRAGWRAVLFSEIIFPACMAILFIIAYMFVKSFPL
DGKQPPSGLVRIAVVSIGPIVWNAAILLTLFLVSLFLGPMLDPVFPLFGSVMAFIAHFL
GTIGMIGFFEFLWFLESWEASHAVLGLIAVISIQRAIHKILIAVFLSREFKHDETNRAW
WTGRWYGRGLGTHAMSQPAREFVVKIIELSLWSSDLILGHILLFMLTPAVLIPYFDRL
HAMMLFWLRPSKQIRAPLYSIKQKRQRRWIIMKYGTVYVTVIAIFVALIALPLVFRHTL
KVECSLCDSL
**SEQ ID NO: 17**
   *tef1* promoter
   DNA
   *S. commune* 
ATCGCCATTGTAAGCCGCAGACGGGCACGCTTCCAACCCCCATCGATGGGCGC
TCGATGTCCATCTCATCGGCGACTCATCATTGTATCTCGCGCAGTCCCATCCCT
CGCCGCTCGCCTGTAGTTTATGCTATTTATCTTTGCACCAGTCGTTGTATTACTC
CCTCGTCGTGTAGAAAGTACCAGATAAAATGCATGTAATCCTAATGAAATTTGCA
CGACACGAAGATCCGGCAGGGTTGTGGGCAAGGGGCAGCGGGAACGAATGGA
TGGCGGGGTACAGCGAGTACCCGGCAGTGCCACAGTCAGTGTCACACACGTGA
CTGATTGTCCATTAGCGTGACCGATAACATCGATCAAAAATTTTATTTCAGAGGA
CGATAAATAAGGGCCGACGGTGCGCGTCCGTCTTTCTCTCAACCCTCATCTTCC
TCTCGTCTCTCACTCTTCCCCCCTCCACCACTACCAAGTAAGTTCAAACTTCCTC
TCATCGCCTTTGCACACATCGCCTACGCCCCATCTCTCTCCATCTGCCTCGCGA
 
ACGGCGCCCCCATCGTCGCTTTCCCGCGCGAGATCTTGTGCGATCTAGTTTACT
GACAATCTCACCTAGAAAACATCAAA
**SEQ ID NO: 18**
   *tef1* terminator
   DNA
   *S*. *commune* 
ATCCAAGTCCGGTGGCAAGGTCACCAAGTCCGCCGAGAAGGCCGCCAAGAAGA
AGTAAATGTAGATGTACATATGTATTTCTCATTCCGTTTCCTTCCTCTTGTTGTT
GTTTCACTGGTCCTCTCGTGCTCGCTCGCATCGCATACAGCCATTGTTGTCACC
ACTATAACTTCACGCATTCTGTATTTCATGCCAGGCGACGGGGTGTTCCTGCCA
GGCCTGTCGCTTGTTGTAACGCTAATGAAAAGTCACGAGTAGTGGACGAACGAC
GATGTATTTCTATGTGCTGTAGCGATTATCCATTTCGAGTTCGCCATCGAGCTCT
CTTCAAACCTAGGTGCGACGTTGTGAATGCAGTAGCAAGTGCAGAGTATTGCAG
ACTCGTCCATTGATGATAACTTCAAGCTACGTCAGAGCCAGATGCTACTGAACC
CGGGCC
**SEQ ID NO: 19**
   Ura_forw (NotI) primer
   DNA
   artificial
   ATAAGAATGCGGCCGCTCCAGCTCGACCTTGCGCCG
**SEQ ID NO: 20**
   Ura_rev (Xbal) primer
   DNA
   artificial
   CTAGTCTAGAGGATCCGACGTGGAGGAGCC
**SEQ ID NO. 21**
   TefP_forw (Xbal) primer
   DNA
   artificial
   CTAGTCTAGAATCGCCATTGTAAGCCGCAG
**SEQ ID NO: 22**
   TefP_rev (Spel) primer
   DNA
   artificial
   CTAGACTAGTTTTGATGTTTTCTAGGTGAG
**SEQ ID NO: 23**
   TefT_forw (Sall) primer
   DNA
   artificial
   ACGCGTCGACCAAGTCCGGTGGCAAGGTCA
**SEQ ID NO: 24**
   TefT_rev (Sall) primer
   DNA
   artificial
   CCGACGTCGACGGGTTCAGTAGCATCTGGCT
**SEQ ID NO: 25**
   TefT_forw (EcoRV) primer
   DNA
   artificial
   CATGGTGATATCCAAGTCCGGTGGCAAGGTCA
**SEQ ID NO: 26**
   TefT_rev (Apal) primer
   DNA
   artificial
   CCGTATGGGCCCGGGTTCAGTAGCATCTGGCT
**SEQ ID NO: 27**
   GS1_forw (Spel) primer
   DNA
   artificial
   CTAGACTAGTCCCGTCCCTCAAGGCCGTTC
**SEQ ID NO: 28**
   GS1_rev (Sall) primer
   DNA
   artificial
   AATGGCCGACGTCGACATGGTATATGCAATGCTATG
**SEQ ID NO: 29**
   Fusion TefP_GS1_forw (Xbal) primer
   DNA
   artificial
   CTAGTCTAGAATCGCCATTGTAAGCCGCAG
**SEQ ID NO: 30**
   Fusion TefP_GS1_rev (Sall) primer
   DNA
   artificial
   AATGGCCGACGTCGACATGGTATATGCAATGCTATG
**SEQ ID NO: 31**
   GS2_forw (Spel) primer
   DNA
   artificial
   CTAGACTAGTCTGTCCAAAGAAGAGATCGA
**SEQ ID NO: 32**
   GS2_rev (EcoRV) primer
   DNA
   artificial
   TACATGCGATATCTTTTATGCAGACTCTCCCTG
**SEQ ID NO: 33**
   *ura* gene
   DNA
   *S*. *commune* 
TCCAGCTCGACCTTGCGCCGCTTGGAGTAACGTTCAGCGTCTTCGTCGTCCTCG
TCGCGCTCGTGTACGATGATGGGCTCAGCCATGGCAGGTATACAAGCTCAGAG
TCAATGGGGGACGAGGTCTCAAGCCGTGAAAGTCGTCGTCGAACAACGTCAAG
TTCGAGACGGACCAGAGTTGGATTTCGTGATTAGATCTACGCTCGATCACAGAA
TGATCAAAGAACAAAGCTTGCCAAAAGGGGATCTCCCATCAACTTCAACTTGCC
CCAAACCATCATGACCGCCGCTCATAAGCTCACATACGGTCAGCGCGCTGCAA
GGTTCACCAATCCCGCGGCGAAAGCCCTGCTGGAAACCATGGAGCGCAAGAAG
AGCAATCTATCCGTCAGCGTCGACGTCGTAAAATCCGCCGATCTGCTCGCTATT
GTCGATACCGTCGGGCCCTATATCTGTCTGATAAAGGCATTGCACTGTCGCTTG
CGGTCTTGGGATGCTGCTTATACTCTATGAAGACCCATGTGGATGTTGTCGAAG
ACTTCGACTCGTCGCTCGTCACCAAGCTTCAGGCTCTGGCCGAGAAGCATGATT
TCCTCATCTTTGAGGACAGAAAATTCGCCGACATAGGTCTGTCCGTCGAATCTC
TATCGATGTCAACTCTGATGACTTGCACAGGCAACACCGTCGCTCTGCAGTACT
CTAGTGGCGTGCACAAAATTGCCAGCTGGTCGCACATCACGAACGCACACCCT
GTTCCAGGACCGTCAATCATCAGTGGCCTCGCATCGGTAGGACAACCCCTCGG
TCGCGGACTCCTCCTGCTCGCAGAGATGAGCACGAAGGGCTCACTTGCGACAG
GCGCGTACACTGAAGCCGCCGTCCAGATGGCAAGGGAGAACCGCGGCTTCGT
CATCGGGTTCATCGCCCAACGGCGGATGGATGGTATTGGCGCGCCTCCAGGG
GTGAATGTCGAGGACGAGGATTTTCTTGTCTTGACACCAGGTGTCGGACTCGAT
GTGAAGGGCGATGGGATGGGGCAGCAATACAGGACGCCGAAGCAAGTGGTAC
AGGAAGATGGGTGCGATGTAATCATCGTGGGTCGCGGATTTATGGCAAGGAC
CCATCGAAGGTGGAAGAGATACGGAGGCAGGCAGAGCGTTACCAGGCTGCAG
GATGGGCGGCGTACATTGAGAGGGTCAACGCCTTGGTATAGCTAATCTGATCG
GTGTTGTCTTGTTAAGCGTCAGGCTCAATGGAACGCTTTGGACGAGCGGAGAGT
AACTTGAATTAGCAGTGTATACTTCGGGCAAATCAATCGTGATAAATACAAGAGC
ACGCTCACGCACGTCCAATCTCCCTCAAAATCTCCATCTTTCTCGCCTCATTCAC
CTTCCTGAACCCAGCCGGCGACATCTCGAACAGACCATGCCCACCCGACAGCG
CACGCAGCCTATTCGAGTAGTCCAGCATCCGGCTGAGCGGCGCCACCGCCTGC
 
ACCGCGCGCTTCATCTTCACGCCCGCCGCCTCCCTCGCCGCAGTGCCGCCAGA
GGGCGACACCCACTCCGGGGGCACGTACACGCCGTCCGCAGGGTACGGCTCC
TCCACGTCGGATCC
SEQ ID NO: 34
   Ura protein
   amino acid
   *S. commune*

MTAAHKLTYGQRAARFTNPAAKALLETMERKKSNLSVSVDVVKSADLLAIVDTVGPY
ICLIKTHVDVVEDFDSSLVTKLQALAEKHDFLIFEDRKFADIGNTVALQYSSGVHKIAS
WSHITNAHPVPGPSIISGLASVGQPLGRGLLLLAEMSTKGSLATGAYTEAAVQMARE
NRGFVIGFIAQRRMDGIGAPPGVNVEDEDFLVLTPGVGLDVKGDGMGQQYRTPKQ
VVQEDGCDVIIVGRGIYGKDPSKVEEIRRQAERYQAAGWAAYIERVNALV

## Claims

1. A genetically modified microorganism capable of producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0.3, **characterized in that** said genetically modified microorganism overexpresses (i) a polynucleotide encoding a polypeptide having 1,3-β-D-glucan synthase-activity, and/or (ii) a polypeptide having 1,3-β-D-glucan synthase-activity, compared to a corresponding non-modified control microorganism of the same strain, wherein said polymer is selected from the group consisting of schizophyllan and scleroglucan, and wherein said microorganism is *Schizophyllum commune.*

2. A use of the genetically modified microorganism according to claim 1 for producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0.3, wherein said polymer is selected from the group consisting of schizophyllan and scleroglucan, and
wherein said microorganism is *Schizophyllum commune.*

3. A method of producing a polymer consisting of a linear main chain of β-D-(1-3)-glucopyranosyl units having a single β-D-glucopyranosyl unit (1-6) linked to a β-D-glucopyranosyl unit of the linear main chain with an average branching degree of about 0.3, said method comprising the steps of:
(a) introducing into a microorganism being able to synthesize said polymer
(i) a strong promoter upstream of a polynucleotide encoding a polypeptide having 1,3-β-D-glucan synthase-activity thereby increasing the expression of said polynucleotide, or
(ii) a polynucleotide encoding a polypeptide having 1,3-β-D-glucan synthase-activity;
(b) culturing said microorganism of (a) in a medium, thereby allowing said microorganism to produce said polymer; and
(c) optionally recovering said polymer from the medium,
wherein said polymer is selected from the group consisting of schizophyllan and scleroglucan, and
wherein said microorganism is *Schizophyllum commune.*

4. The genetically modified microorganism according to claim 1, the use according to claim 2, or the method according to claim 3, wherein said polynucleotide is a 1,3-β-D-glucan synthase gene.

5. The genetically modified microorganism according to claim 1 or 4, the use according to claim 2 or 4, or the method according to claim 3 or 4, wherein said polynucleotide comprises a nucleotide sequence being at least 70% identical to the nucleotide sequence of SEQ ID NO: 1, SEQ ID NO: 3, SEQ ID NO: 5, SEQ ID NO: 7, SEQ ID NO: 9, SEQ ID NO: 11, SEQ ID NO: 13, or SEQ ID NO: 15.

6. The genetically modified microorganism according to any one of claims 1 or 4 to 5, the use according to any one of claims 2 or 4 to 5, or the method according to any one of claims 3 to 5, wherein said polypeptide is a 1,3-β-D-glucan synthase.

7. The genetically modified microorganism according to any one of claims 1 or 4 to 6, the use according to any one of claims 2 or 4 to 6, or the method according to any one of claims 3 to 6, wherein said polypeptide comprises an amino acid sequence which is at least 70% identical to the amino acid sequence of SEQ ID NO: 6, SEQ ID NO: 8, SEQ ID NO: 14, or SEQ ID NO: 16.

8. The genetically modified microorganism according to any one of claims 1 or 4 to 7, wherein said modified microorganism is able to produce at least 1.5 times more of said polymer compared to said non-modified control microorganism.

## Patentansprüche

1. Gentechnisch veränderter Mikroorganismus mit der Fähigkeit zur Herstellung eines Polymers, das aus einer geradlinigen Hauptkette von β-D-(1-3)-Glucopyranosyl-Einheiten mit einer einzelnen β-D-Glucopyranosyl-Einheit, die mit einer β-D-Glucopyranosyl-Einheit der geradlinigen Hauptkette (1-6)-verknüpft ist, bei einem durchschnittlichen Verzweigungsgrad von etwa 0,3, besteht, **dadurch gekennzeichnet, dass** der gentechnisch veränderte Mikroorganismus (i) ein Polynukleotid, das ein Polypeptid mit 1,3-β-D-Glucansynthase-Aktivität codiert, und/oder (ii) ein Polypeptid mit 1,3-β-D-Glucansynthase-Aktivität verglichen mit einem entsprechenden nicht veränderten Kontrollmikroorganismus des gleichen Stamms überexprimiert,
wobei das Polymer aus der Gruppe bestehend aus Schizophyllan und Skleroglucan ausgewählt ist und wobei es sich bei dem Mikroorganismus um *Schizophyllum commune* handelt.

2. Verwendung des gentechnisch veränderten Mikroorganismus nach Anspruch 1 zur Herstellung eines Polymers, das aus einer geradlinigen Hauptkette von β-D-(1-3)-Glucopyranosyl-Einheiten mit einer einzelnen β-D-Glucopyranosyl-Einheit, die mit einer β-D-Glucopyranosyl-Einheit der geradlinigen Hauptkette (1-6)-verknüpft ist, bei einem durchschnittlichen Verzweigungsgrad von etwa 0,3, besteht,
wobei das Polymer aus der Gruppe bestehend aus Schizophyllan und Skleroglucan ausgewählt ist und
wobei es sich bei dem Mikroorganismus um *Schizophyllum commune* handelt.

3. Verfahren zur Herstellung eines Polymers, das aus einer geradlinigen Hauptkette von β-D-(1-3)-Glucopyranosyl-Einheiten mit einer einzelnen β-D-Glucopyranosyl-Einheit, die mit einer β-D-Glucopyranosyl-Einheit der geradlinigen Hauptkette (1-6)-verknüpft ist, bei einem durchschnittlichen Verzweigungsgrad von etwa 0,3, besteht, wobei das Verfahren die folgenden Schritte umfasst:
(a) Einführen
(i) eines starken Promotors stromaufwärts von einem Polynukleotid, das ein Polypeptid mit 1,3-β-D-Glucansynthase-Aktivität codiert, wodurch die Expression des Polynukleotids erhöht wird, oder
(ii) eines Polynukleotids, das ein Polypeptid mit 1,3-β-D-Glucansynthase-Aktivität codiert, in einen Mikroorganismus, der das Polymer synthetisieren kann;
(b) Kultivieren des Mikroorganismus aus (a) in einem Medium, wodurch dem Mikroorganismus die Herstellung des Polymers ermöglicht wird; und
(c) gegebenenfalls Gewinnen des Polymers aus dem Medium,
wobei das Polymer aus der Gruppe bestehend aus Schizophyllan und Skleroglucan ausgewählt ist und
wobei es sich bei dem Mikroorganismus um *Schizophyllum commune* handelt.

4. Gentechnisch veränderter Mikroorganismus nach Anspruch 1, Verwendung nach Anspruch 2 oder Verfahren nach Anspruch 3, wobei es sich bei dem Polynukleotid um ein 1,3-β-D-Glucansynthase-Gen handelt.

5. Gentechnisch veränderter Mikroorganismus nach Anspruch 1 oder 4, Verwendung nach Anspruch 2 oder 4 oder Verfahren nach Anspruch 3 oder 4, wobei das Polynukleotid eine Nukleotidsequenz umfasst, die zu wenigstens 70% identisch mit der Nukleotidsequenz unter SEQ ID NO; 1 , SEQ ID NO: 3, SEQ ID NO: 5, SEQ ID NO: 7, SEQ ID NO: 9, SEQ ID NO: 11, SEQ ID NO: 13 oder SEQ ID NO: 15 ist.

6. Gentechnisch veränderter Mikroorganismus nach einem der Ansprüche 1 oder 4 bis 5, Verwendung nach einem der Ansprüche 2 oder 4 bis 5 oder Verfahren nach einem der Ansprüche 3 bis 5, wobei es sich bei dem Polypeptid um eine 1,3-β-D-Glucansynthase handelt.

7. Gentechnisch veränderter Mikroorganismus nach einem der Ansprüche 1 oder 4 bis 6, Verwendung nach einem der Ansprüche 2 oder 4 bis 6 oder Verfahren nach einem der Ansprüche 3 bis 6, wobei das Polypeptid eine Aminosäuresequenz umfasst, die zu wenigstens 70% identisch mit der Aminosäuresequenz unter SEQ ID NO: 6, SEQ ID NO: 8, SEQ ID NO: 14 oder SEQ ID NO: 16 ist.

8. Gentechnisch veränderter Mikroorganismus nach einem der Ansprüche 1 oder 4 bis 7, wobei der veränderte Mikroorganismus in der Lage ist, verglichen mit dem nicht veränderten Kontrollmikroorganismus wenigstens 1,5 Mal mehr Polymer zu produzieren.

## Revendications

1. Microorganisme génétiquement modifié capable de produire un polymère étant constitué par une chaîne principale linéaire d'unités de β-D-(1-3)-glucopyranosyle, ayant une seule unité de β-D-glucopyranosyle liée en (1-6) à une unité de β-D-glucopyranosyle de la chaîne principale linéaire avec un degré moyen de ramification de 0,3 environ, **caractérisé en ce que** ledit microorganisme génétiquement modifié surexprime (i) un polynucléotide codant pour un polypeptide qui a une activité de 1,3-β-D-glucane synthase et/ou (ii) un polypeptide ayant une activité de 1,3-β-D-glucane synthase, en comparaison avec un microorganisme témoin non modifié correspondant de la même souche,
dans lequel ledit polymère est choisi dans le groupe constitué par le schizophyllane et le scléroglucane, et dans lequel ledit microorganisme est *Schizophyllum commune.*

2. Utilisation du microorganisme génétiquement modifié, selon la revendication 1, pour produire un polymère constitué par une chaîne principale linéaire d'unités de β-D-(1-3)-glucopyranosyle ayant une seule unité de β-D-glucopyranosyle liée en (1-6) à une unité de β-D-glucopyranosyle de la chaîne principale linéaire, avec un degré moyen de ramification de 0,3 environ,
dans lequel ledit polymère est choisi dans le groupe constitué par le schizophyllane et le scléroglucane, et dans lequel ledit microorganisme est *Schizophyllum commune.*

3. Procédé de production d'un polymère constitué par une chaîne principale linéaire d'unités de β-D-(1-3)-glucopyranosyle ayant une seule unité de β-D-glucopyranosyle liée en (1-6) à une unité de β-D-glucopyranosyle de la chaîne principale linéaire, avec un degré moyen de ramification de 0,3 environ, ledit procédé comprenant les étapes consistant à :
(a) introduire dans un microorganisme étant capable de synthétiser ledit polymère
(i) un promoteur fort en amont d'un polynucléotide qui code pour un polypeptide ayant une activité de 1,3-β-D-glucane synthase, en augmentant de ce fait l'expression dudit polynucléotide, ou
(ii) un polynucléotide codant pour un polypeptide qui a une activité de 1,3-β-D-glucane synthase ;
(b) cultiver ledit microorganisme du paragraphe (a) dans un milieu, en permettant de ce fait au dit microorganisme de produire ledit polymère ; et
(c) récupérer, en option, ledit polymère à partir du milieu,
dans lequel ledit polymère est choisi dans le groupe constitué par le schizophyllane et le scléroglucane, et dans lequel ledit microorganisme est *Schizophyllum commune.*

4. Microorganisme génétiquement modifié selon la revendication 1, utilisation selon la revendication 2, ou procédé selon la revendication 3, dans lequel/laquelle ledit polynucléotide est un gène de 1,3-β-D-glucane synthase.

5. Microorganisme génétiquement modifié selon les revendications 1 ou 4, utilisation selon les revendications 2 ou 4, ou bien procédé selon les revendications 3 ou 4, dans lequel/laquelle ledit polynucléotide comprend une séquence nucléotidique étant identique au moins à 70% à la séquence nucléotidique de SEQ ID n° : 1, SEQ ID n° : 3, SEQ ID n° : 5, SEQ ID n° : 7, SEQ ID n° : 9, SEQ ID n° : 11, SEQ ID n° : 13 ou SEQ ID n° : 15.

6. Microorganisme génétiquement modifié selon l'une quelconque des revendications 1 ou 4 à 5, utilisation selon l'une quelconque des revendications 2 ou 4 à 5, ou bien procédé selon l'une quelconque des revendications 3 à 5, dans lequel/laquelle ledit polypeptide est une 1,3-β-D-glucane synthase.

7. Microorganisme génétiquement modifié selon l'une quelconque des revendications 1 ou 4 à 6, utilisation selon l'une quelconque des revendications 2 ou 4 à 6, ou bien procédé selon l'une quelconque des revendications 3 à 6, dans lequel/laquelle ledit polypeptide comprend une séquence d'acides aminés qui est identique au moins à 70% à la séquence d'acides aminés de SEQ ID n° : 6, SEQ ID n° : 8, SEQ ID n° : 14 ou SEQ ID n° : 16.

8. Microorganisme génétiquement modifié selon l'une quelconque des revendications 1 ou 4 à 7, ledit microorganisme modifié étant capable de produire au moins 1,5 fois plus dudit polymère que ledit microorganisme témoin non modifié.
